# EUROPEAN PATENT APPLICATION

(11) **EP 3 604 289 A1**
(43) Date of publication of application: **05.02.2020**
(21) Application number: 18777563.0
(22) Date of filing: 19.03.2018
(51) Int. Cl.: C07D 277/82, C07C 69/75, C08F 20/38, G02B 5/30

(54) **POLYMERIZABLE COMPOUND PRODUCTION METHOD AND POLYMERIZABLE COMPOUND SOLUTION**

(30) Priority: 27.03.2017 JP 2017061659
(71) Applicant: Zeon Corporation, Tokyo 100-8246 (JP)
(72) Inventor: OKUYAMA Kumi, Tokyo 100-8246 (JP); SAKAMOTO Kei, Tokyo 100-8246 (JP); MIMA Takanori, Tokyo 100-8246 (JP)
(74) Representative: Adam, Holger
(86) International application number: PCT/JP2018/010845
(87) International publication number: WO 2018/180716

(57) **Abstract**

Disclosed is a method of producing a polymerizable compound that enables production of, in high yield, a polymerizable compound used for producing an optical film or the like. The disclosed method of producing a polymerizable compound comprises reacting a compound represented by formula (I) with a compound represented by formula (II) in an organic solvent in which a base having a pKa from 6.1 to 9.5 is present, so as to obtain a reaction solution containing a polymerizable compound represented by formula (III).

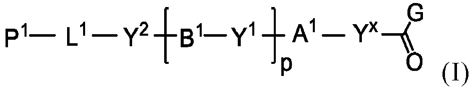

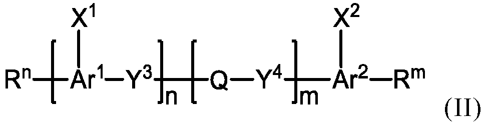

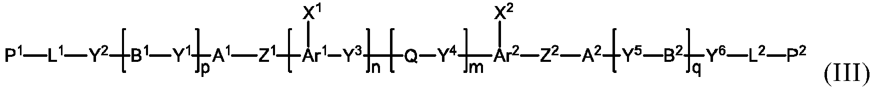

## Description

### TECHNICAL FIELD

The present disclosure relates to a method of producing a polymerizable compound and a solution of a polymerizable compound, and specifically, relates to a method of producing a polymerizable compound that enables production of, in high yield, a polymerizable compound used for producing an optical film or the like, and a solution containing the polymerizable compound produced by the production method.

### BACKGROUND

Quarter-wave plates that convert linearly polarized light to circularly polarized light and half-wave plates that perform 90° conversion of the plane of vibration of linearly polarized light are known as retardation plates that are used for a flat panel display device (FPD). These retardation plates can be converted to a retardation of 1/4λ or 1/2λ of the wavelength of light with respect to specific monochromatic light. Recently, various retardation plates which are wideband retardation plates that can achieve uniform retardation with respect to light over a wide wavelength region having so-called reverse wavelength dispersion have been considered.

On the one hand, it has been desired to reduce the thickness of the flat panel display device as much as possible along with an improvement in functionality and widespread use of information terminals such as mobile personal computers and mobile phones. Therefore, a reduction in the thickness of the retardation plates which are components has also been desired.

In terms of methods of achieving thickness-reduction, the method of creating retardation plates by applying a polymerizable composition comprising a low-molecular weight polymerizable compound on a film substrate has been considered to be promising in recent years. Moreover, there has been much development of low-molecular weight polymerizable compositions having a wavelength dispersion property or a polymerizable compound in which these compounds are used (for example, refer to PTL 1 to 3).

Specifically, a compound having a practical low melting point, having an excellent solubility in a general-purpose solvent, and which can produce an optical film that can achieve uniform conversion of polarized light over a wide wavelength band has been provided (for example, refer to PTL 4).

Further, for example, PTL 5 proposes the technique for producing the compound described in PTL 4 in high yield by reacting the following compound (A) with 2,5-dihydroxybenzaldehyde in the presence of a base such as triethylamine.

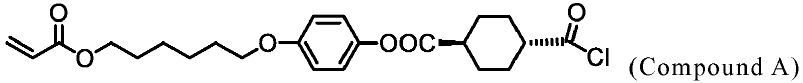

### CITATION LIST

### Patent Literature

PTL 1: WO2012/147904
PTL 2: WO2012/141245
PTL 3: WO2014/126113
PTL 4: WO2014/010325
PTL 5: WO2015/141784

### SUMMARY

### (Technical Problem)

However, the conventional production method using triethylamine as a base as described in PTL 5 has room for improvement in terms of producing the compound in a higher yield.

The present disclosure was conceived in view of the above-described circumstances, and an object of the present disclosure is to provide a method of producing a polymerizable compound that enables production of, in high yield, a polymerizable compound used for producing an optical film or the like.

Another object of the present disclosure is to provide a solution containing the polymerizable compound produced by the aforementioned production method.

### (Solution to Problem)

The inventors made keen research for solving the aforementioned problems, and as a result, discovered that when a compound represented by the following formula (I) is reacted with a compound represented by the following formula (II) in an organic solvent in which a base having a pKa from 6.1 to 9.5 is present, the polymerizable compound represented by the following formula (III) used to produce an optical film or the like can be produced in high yield, and completed the present disclosure.

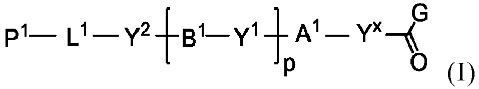

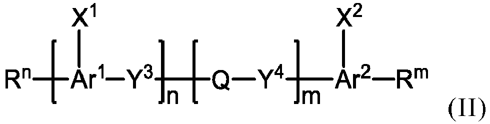

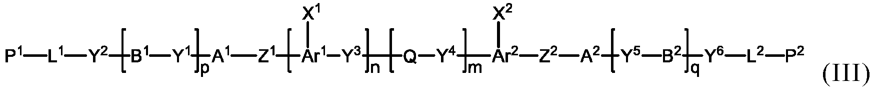

Accordingly, the present disclosure provides a method of producing a polymerizable compound and a solution of a polymerizable comound given below.
[1] A method of producing a polymerizable compound, comprising: reacting a compound represented by formula (I) with a compound represented by formula (II) in an organic solvent in which a base having a pKa from 6.1 to 9.5 is present, so as to obtain a reaction solution containing a polymerizable compound represented by formula (III): 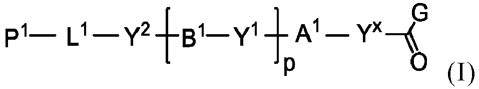 where in the formula (I),
   Y^{x} represents a single bond, -CH₂-, -CH₂-CH₂-, or -CH=CH-,
   A¹ and B¹ each independently represent a cyclic aliphatic group which may have a substituent, or an aromatic group which may have a substituent,
   Y¹ and Y² each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, -NR²¹-C(=O)-NR²²-, -O-CH₂-, -O-CH₂-CH₂-, -CH₂-O-, -CH₂-CH₂-O-, -CH₂-O-CH₂-, -CH₂-CH₂-O-CH₂-, -CH₂-O-CH₂-CH₂-, -CH₂-CH₂-O-CH₂-CH₂-, -C(=O)-CH₂-, -C(=O)-CH₂-CH₂-, -CH₂-C(=O)-, -CH₂-CH₂-C(=O)-, -O-C(=O)-CH₂-, -CH₂-C(=O)-O-, -O-C(=O)-CH₂-CH₂-, -CH₂-CH₂-C(=O)-O-, -NR²¹-C(=O)-CH₂-, -NR²¹-C(=O)-CH₂-CH₂-, -CH₂-C(=O)-NR²¹-, -CH₂-CH₂-C(=O)-NR²¹-, -O-C(=O)-O-CH₂-, -O-C(=O)-O-CH₂-CH₂-, -CH₂-O-C(=O)-O-, -CH₂-CH₂-O-C(=O)-O-, -CH₂-O-C(=O)-O-CH₂-, -CH₂-CH₂-O-C(=O)-O-CH₂-, -CH₂-O-C(=O)-O-CH₂-CH₂-, -CH₂-CH₂-O-C(=O)-O-CH₂-CH₂-, -NR²¹-C(=O)-NR²²-CH₂-, -NR²¹-C(=O)-NR²²-O-CH₂-CH₂-, -CH₂-CH₂-O-NR²¹-C(=O)-NR²²-, -CH₂-NR²¹-C(=O)-NR²²-, -CH₂-CH₂-NR²¹-C(=O)-NR²²-, -NR²¹-C(=O)-NR²²-CH₂-CH₂-, -CH₂-NR²¹-C(=O)-NR²²-CH₂-, -CH₂-CH₂-NR²¹-C(=O)-NR²²-CH₂-, -CH₂-NR²¹-C(=O)-NR²²-CH₂-CH₂-, -CH₂-NR²¹-C(=O)-NR²-O-CH₂-CH₂-, -CH₂-CH₂-O-NR²¹-C(=O)-NR²²-CH₂-, -CH₂-CH₂-NR²¹-C(=O)-NR²²-O-CH₂-CH₂-, or -CH₂-CH-O-NR²¹-C(=O)-NR²²-CH₂-CH₂-, where R²¹ and R²² each independently represent a hydrogen atom or an alkyl group having 1 to 6 carbon atoms,
   L¹ is an organic group which is either an alkylene group having 1 to 20 carbon atoms, or an alkylene group having 3 to 20 carbon atoms in which at least one methylene group (-CH₂-) contained in the alkylene group is substituted by -O- or -C(=O)-, and the hydrogen atom included in the organic group of L¹ may be substituted by an alkyl group having 1 to 5 carbon atoms, an alkoxy group having 1 to 5 carbon atoms, or a halogen atom, with the proviso that the methylene groups (-CH₂-) on both ends of L¹ are not substituted with -O- or -C(=O)-,
   P¹ represents a hydrogen atom or a polymerizable group,
   p is an integer from 0 to 3, and
   G represents a leaving group, 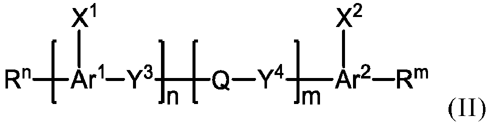
   where in the formula (II),
   Ar¹ and Ar² each independently represent an aromatic hydrocarbon ring group which may have a substituent, or an aromatic heterocyclic ring group which may have a substituent,
   X¹ and X² each independently represent -CHO, or -C(=O)-R^{a}, where R^{a} represents an organic group having 1 to 20 carbon atoms which may have a substituent,
   Y³ and Y⁴ each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, or -NR²¹-C(=O)-NR²²-, where R²¹ and R²² each independently represent a hydrogen atom or an alkyl group having 1 to 6 carbon atoms,
   Q represents an organic group having 1 to 20 carbon atoms which may have a substituent,
   n and m each independently represent an integer from 0 to 3, and
   Rⁿ and R^{m} each independently represent -CH₂-CH₂-OR^{b}, -CH₂-OR^{b}, -CH₂-CH₂-OH, -CH₂-OH, -OR^{b}, -COOR^{b}, -NHR²⁰, -SH, a hydroxyl group, or a carboxyl group, where R²⁰ represents a hydrogen atom or an alkyl group having 1 to 6 carbon atoms, R^{b} represents a protecting group, and when Rⁿ or R^{m} is -CH₂-CH₂-OR^{b}, -CH₂-OR^{b}, -OR^{b}, or COOR^{b}, at least one of Rn and Rm is -CH₂-CH₂-OH, -CH₂-OH, -NHR²⁰, -SH, a hydroxyl group, or a carboxyl group, 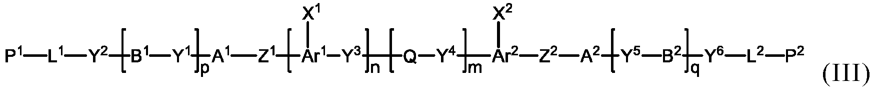
   wherein the formula (III),
   A¹, A², B¹ and B² each independently represent a cyclic aliphatic group which may have a substituent, or an aromatic group which may have a substituent,
   Ar¹ and Ar² each independently represent an aromatic hydrocarbon ring group which may have a substituent, or an aromatic heterocyclic ring group which may have a substituent,
   X¹ and X² each independently represent -CHO, or -C(=O)-R^{a}, where R^{a} represents an organic group having 1 to 20 carbon atoms which may have a substituent,
   Z¹ and Z² each independently represent -C(=O)-O-, -O-C(=O)-, -C(=O)-S-, -S-C(=O)-, -NR²⁰-C(=O)-, -C(=O)-NR²¹-, -CH=CH-C(=O)-O-, -O-C(=O)-CH=CH-, -CH₂-C(=O)-O-, -O-C(=O)-CH₂-, -CH₂-O-C(=O)-, -C(=O)-O-CH₂-, -CH₂-CH₂-C(=O)-O-, -O-C(=O)-CH₂-CH₂-, -CH₂-CH₂-O-C(=O)-, -C(=O)-O-CH₂-CH₂-, or -C(=O)-O-C(=O)-, where R²⁰ represents a hydrogen atom or an alkyl group having 1 to 6 carbon atoms,
   Y¹ to Y⁶ each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, -NR²¹-C(=O)-NR²²-, -O-CH₂-, -O-CH₂-CH₂-, -CH₂-O-, -CH₂-CH₂-O-, -CH₂-O-CH₂-, -CH₂-CH₂-O-CH₂-, -CH₂-O-CH₂-CH₂-, -CH₂-CH₂-O-CH₂-CH₂-, -C(=O)-CH₂-, -C(=O)-CH₂-CH₂-, -CH₂-C(=O)-, -CH₂-CH₂-C(=O)-, -O-C(=O)-CH₂-, -CH₂-C(=O)-O-, -O-C(=O)-CH₂-CH₂-, -CH₂-CH₂-C(=O)-O-, -NR²¹-C(=O)-CH₂-, -NR²¹-C(=O)-CH₂-CH₂-, -CH₂-C(=O)-NR²¹-, -CH₂-CH₂-C(=O)-NR²¹-, -O-C(=O)-O-CH₂-, -O-C(=O)-O-CH₂-CH₂-, -CH₂-O-C(=O)-O-, -CH₂-CH₂-O-C(=O)-O-, -CH₂-O-C(=O)-O-CH₂-, -CH₂-CH₂-O-C(=O)-O-CH₂-, -CH₂-O-C(=O)-O-CH₂-CH₂-, -CH₂-CH₂-O-C(=O)-O-CH₂-CH₂-, -NR²¹-C(=O)-NR²²-CH₂-, -NR²¹-C(=O)-NR²²-O-CH₂-CH₂-, -CH₂-CH₂-O-NR²¹-C(=O)-NR²²-, -CH₂-NR²¹-C(=O)-NR²²-, -CH₂-CH₂-NR²¹-C(=O)-NR²²-, -NR²¹-C(=O)-NR²²-CH₂-CH₂-, -CH₂-NR²¹-C(=O)-NR²²-CH₂-, -CH₂-CH₂-NR²¹-C(=O)-NR²²-CH₂-, -CH₂-NR²¹-C(=O)-NR²²-CH₂-CH₂-, -CH₂-NR²¹-C(=O)-NR²²-O-CH₂-CH₂-, -CH₂-CH₂-O-NR²¹-C(=O)-NR²²-CH₂-, -CH₂-CH₂-NR²¹-C(=O)-NR²²-O-CH₂-CH₂-, or -CH₂-CH₂-O-NR²¹-C(=O)-NR²²-CH₂-CH₂-, where R²¹ and R²² each independently represent a hydrogen atom or an alkyl group having 1 to 6 carbon atoms,
   L¹ and L² each represent an organic group which is either an alkylene group having 1 to 20 carbon atoms, or an alkylene group having 3 to 20 carbon atoms in which at least one methylene group (-CH₂-) contained in the alkylene group is substituted by -O- or -C(=O)-, and the hydrogen atom included in the organic group of L¹ and L² may be substituted by an alkyl group having 1 to 5 carbon atoms, an alkoxy group having 1 to 5 carbon atoms, or a halogen atom, with the proviso that the methylene groups (-CH₂-) on both ends of L¹ are not substituted with -O- or -C(=O)-,
   Q represents an organic group having 1 to 20 carbon atoms which may have a substituent,
   P¹ and P² each independently represent a hydrogen atom or a polymerizable group, and at least one of P¹ and P² represents a polymerizable group, and
   p, q, n and m each independently represent an integer from 0 to 3.
[2] The method of producing a polymerizable compound according to [1], wherein the base is a tertiary amine.
[3] The method of producing a polymerizable compound according to [1] or [2], wherein the base has a pKa from 6.5 to 7.5.
[4] The method of producing a polymerizable compound according to any one of [1] to [3], wherein at least one pyridine having at least two alkyl groups having 1 to 6 carbon atoms is used as the base.
[5] The method of producing a polymerizable compound according to any one of [1] to [4], wherein at least one pyridine where at least two hydrogen atoms among hydrogen atoms at the 2-position, 4-position and 6-position in the pyridine are substituted with an alkyl group having 1 to 6 carbon atoms is used as the base..
[6] The method of producing a polymerizable compound according to any of [1] to [3], wherein at least one compound selected from the group consisting of 2,4-lutidine, 2,6-lutidine, and 2,4,6-collidine is used as the base.
[7] The method of producing a polymerizable compound according to any of [1] to [6], wherein the Ar¹-X¹ and Ar²-X² each independently are represented by any of the following formulas (VIII-1) to (VIII-7): 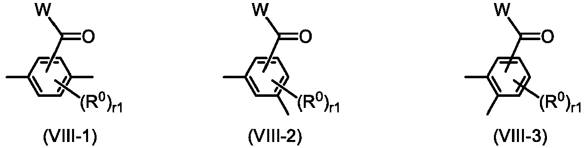 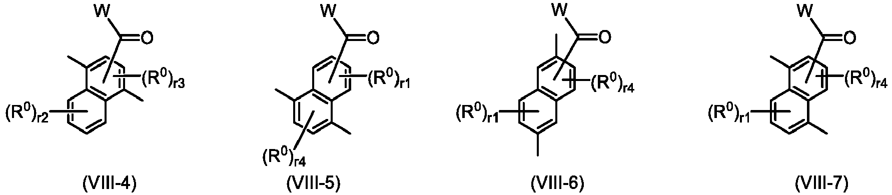 where in the formulas (VIII-1) to (VIII-7),
   W represents a hydrogen atom or an alkyl group having 1 to 6 carbon atoms, and
   R⁰ represents a halogen atom; a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N,N-dialkylamino group having 2 to 12 carbon atoms, an alkoxy group having 1 to 6 carbon atoms, a nitro group, -C(=O)-R^{a1}, -O-C(=O)-R^{a1}, -C(=O)-O-R^{a1}, or -SO₂R^{a1}, where R^{a1} represents an alkyl group having 1 to 6 carbon atoms, or an aromatic hydrocarbon ring group having 6 to 20 carbon atoms which may have an alkyl group having 1 to 6 carbon atoms or an alkoxy group having 1 to 6 carbon atoms as a substituent, r1 is an integer from 0 to 3, r2 is an integer from 0 to 4, r3 is 0 or 1, and r4 is an integer from 0 to 2, with the proviso that when there is a plurality of R⁰, each R⁰ may be the same or may be different.
[8] The method of producing a polymerizable compound according to any one of [1] to [7], wherein the polymerizable compound represented by formula (III) is represented by any of the following formulas (III-1) to (III-6): 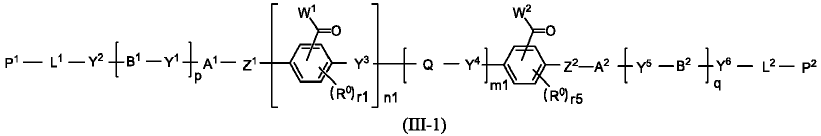 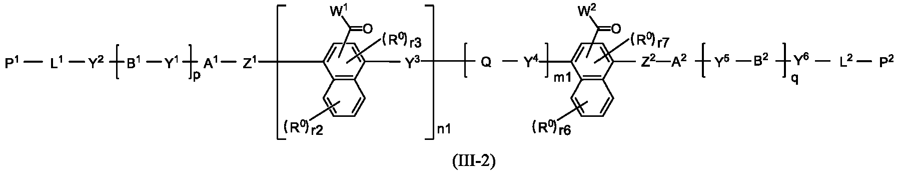 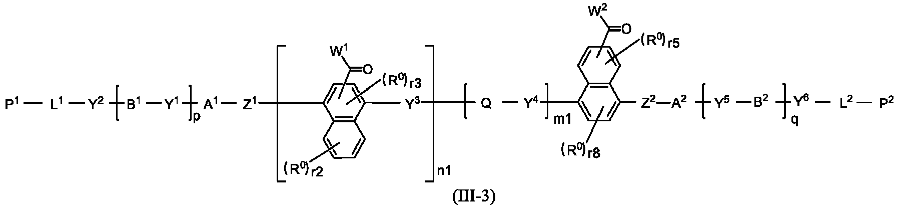 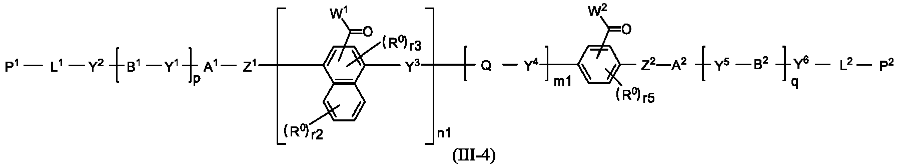 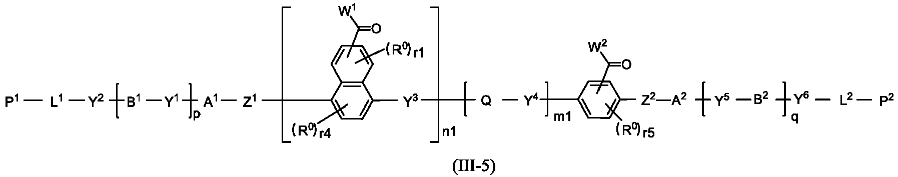 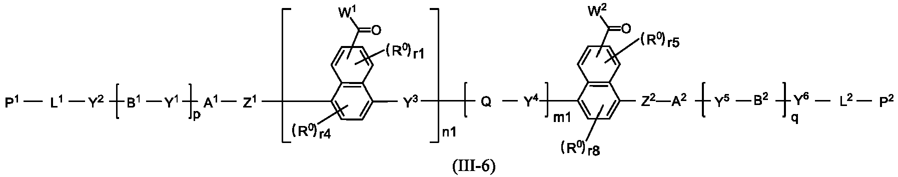 where in the formulas (III-1) to (III-6),
   W¹ and W² each independently represent a hydrogen atom or an organic group having 1 to 20 carbon atoms which may have a substituent,
   n1 is an integer of 0 or 1,
   m1 is an integer of 0 or 1,
   R⁰ represents a halogen atom; a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N,N-dialkylamino group having 2 to 12 carbon atoms, an alkoxy group having 1 to 6 carbon atoms, a nitro group, -C(=O)-R^{a1}, -O-C(=O)-R^{a1}, -C(=O)-O-R^{a1}, or -SO₂R^{a1}, where R^{a1} represents an alkyl group having 1 to 6 carbon atoms, or an aromatic hydrocarbon ring group having 6 to 20 carbon atoms which may have an alkyl group having 1 to 6 carbon atoms or an alkoxy group having 1 to 6 carbon atoms as a substituent, r1 and r5 each independently represent an integer from 0 to 3, r2 and r6 each independently represent an integer from 0 to 4, r3 and r7 each independently are 0 or 1, and r4 and r8 each independently represent an integer from 0 to 2, wherein, when there is a plurality of R⁰, each R⁰ may be the same or may be different, and
   A¹, A2, B¹, B², Y¹ to Y⁶, L¹, L², P¹, P², Z¹, Z², Q, p, and q are the same as defined above.
[9] The method of producing a polymerizable compound according to any one of [1] to [8], wherein the P¹ and P² each independently are represented by the following formula (IV): 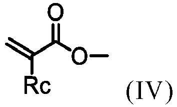 where in the formula (IV), Rc represents a hydrogen atom, a methyl group or a chlorine atom.
[10] The method of producing a polymerizable compound according to any one of [1] to [9], wherein Q is represented by any of the following formulas (VII-1) to (VII-29): 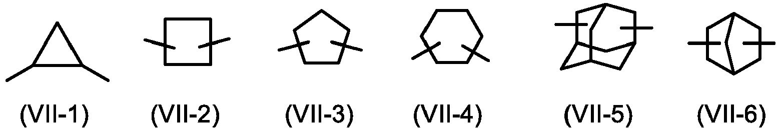 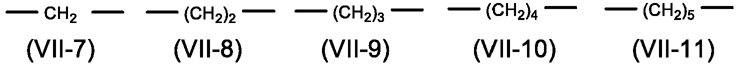 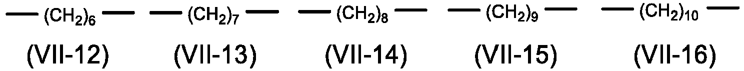 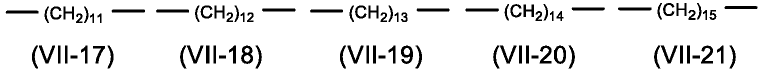 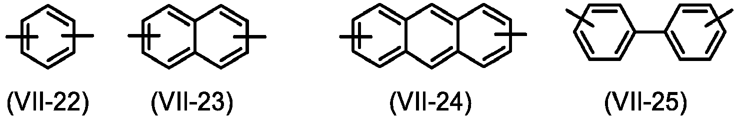 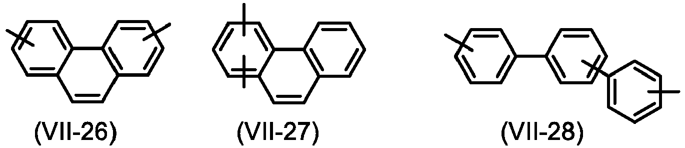 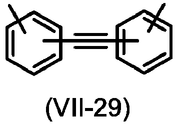
[11] The method of producing a polymerizable compound according to any one of [1] to [10], wherein the compound represented by formula (I) is reacted with a compound represented by formula (II) in the presence of a polymerizable compound represented by the following formula (XII): 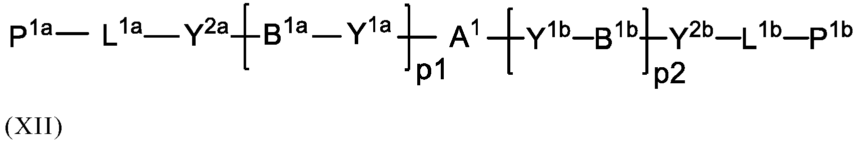 where in the formula (XII),
   A¹, B^{1a} and B^{1b} each independently represent a cyclic aliphatic group which may have a substituent, or an aromatic group which may have a substituent,
   Y^{1a}, Y^{1b}, Y^{2a} and Y^{2b} each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, -NR²¹-C(=O)-NR²²-, -O-CH₂-, -O-CH₂-CH₂-, -CH₂-O-, -CH₂-CH₂-O-, -CH₂-O-CH₂-, -CH₂-CH₂-O-CH₂-, -CH₂-O-CH₂-CH₂-, -CH₂-CH₂-O-CH₂-CH₂-, -C(=O)-CH₂-, -C(=O)-CH₂-CH₂-, -CH₂-C(=O)-, -CH₂-CH₂-C(=O)-, -O-C(=O)-CH₂-, -CH₂-C(=O)-O-, -O-C(=O)-CH₂-CH₂-, -CH₂-CH₂-C(=O)-O-, -NR²¹-C(=O)-CH₂-, -NR²¹-C(=O)-CH₂-CH₂-, -CH₂-C(=O)-NR²¹-, -CH₂-CH₂-C(=O)-NR²¹-, -O-C(=O)-O-CH₂-, -O-C(=O)-O-CH₂-CH₂-, -CH₂-O-C(=O)-O-, -CH₂-CH₂-O-C(=O)-O-, -CH₂-O-C(=O)-O-CH₂-, -CH₂-CH₂-O-C(=O)-O-CH₂-, -CH₂-O-C(=O)-O-CH₂-CH₂-, -CH₂-CH₂-O-C(=O)-O-CH₂-CH₂-, -NR²¹-C(=O)-NR²²-CH₂-, -NR²¹-C(=O)-NR²²-O-CH₂-CH₂-, -CH₂-CH₂-O-NR²¹-C(=O)-NR²²-, -CH₂-NR²¹-C(=O)-NR²²-, -CH₂-CH₂-NR²¹-C(=O)-NR²²-, -NR²¹-C(=O)-NR²²-CH₂-CH₂-, -CH₂-NR²¹-C(=O)-NR²²-CH₂-, -CH₂-CH₂-NR²¹-C(=O)-NR²²-CH₂-, -CH₂-NR²¹-C(=O)-NR²²-CH₂-CH₂-, -CH₂-NR²¹-C(=O)-NR²²-O-CH₂-CH₂-, -CH₂-CH₂-O-NR²¹-C(=O)-NR²²-CH₂-, -CH₂-CH₂-NR²¹-C(=O)-NR²²-O-CH₂-CH₂-, or -CH₂-CH₂-O-NR²¹-C(=O)-NR²²-CH₂-CH₂-, where R²¹ and R²² each independently represent a hydrogen atom or an alkyl group having 1 to 6 carbon atoms,
   L^{1a} and L^{1b} each independently represent an organic group which is either an alkylene group having 1 to 20 carbon atoms, or an alkylene group having 3 to 20 carbon atoms in which at least one methylene group (-CH₂-) contained in the alkylene group is substituted by -O- or -C(=O)-, and the hydrogen atom included in the organic groups of L^{1a} and L^{1b} may be substituted by an alkyl group having 1 to 5 carbon atoms, an alkoxy group having 1 to 5 carbon atoms, or a halogen atom, with the proviso that the methylene groups (-CH₂-) on both ends of L^{1a} and L^{1b} are not substituted with -O- or -C(=O)-,
   P^{1a} and P^{1b} each independently represent a polymerizable group, and
   p1 and p2 each independently represent an integer from 0 to 3.
[12] A method of producing a polymerizable compound, comprising: a Step 1 which uses the method of producing a polymerizable compound according to any one of [1] to [11] to obtain the polymerizable compound represented by the formula (III); and a Step 2 which reacts the polymerizable compound represented by the formula (III) obtained in the Step 1 with a compound represented by the following formula (V) to obtain a polymerizable compound represented by the following formula (VI):

   **D-NH₂** (V)

   where in the formula (V), D is represented by the following formula (V-I) or (V-II): 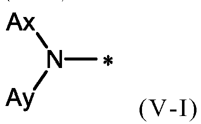 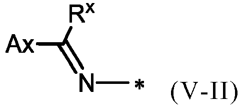 where * represents an amino group,
   Ax represents an organic group having at least one aromatic ring selected from the group consisting of an aromatic hydrocarbon ring having 6 to 30 carbon atoms and an aromatic heterocyclic ring having 2 to 30 carbon atoms, and the aromatic ring included in Ax may have a substituent,
   Ay represents a hydrogen atom or an organic group having 1 to 30 carbon atoms which may have a substituent, and
   R^{x} represents a hydrogen atom or an organic group having 1 to 30 carbon atoms which may have a substituent, 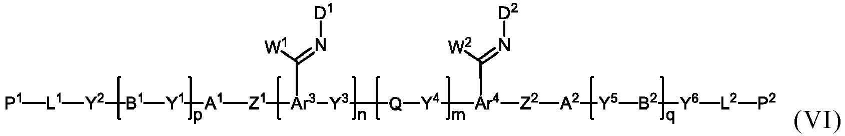
   where in the formula (VI),
   W¹ and W² each independently represent a hydrogen atom or an organic group having 1 to 20 carbon atoms which may have a substituent,
   Ar³ and Ar⁴ each independently represent an aromatic hydrocarbon ring group which may have a substituent, or an aromatic heterocyclic ring group which may have a substituent,
   D¹ and D² each independently represent the following formula (V-I) or (V-II), 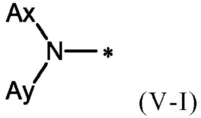 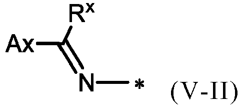
      where * represents an amino group,
      Ax represents an organic group having at least one aromatic ring selected from the group consisting of an aromatic hydrocarbon ring having 6 to 30 carbon atoms and an aromatic heterocyclic ring having 2 to 30 carbon atoms, and the aromatic ring included in Ax may have a substituent,
      Ay represents a hydrogen atom or an organic group having 1 to 30 carbon atoms which may have a substituent,
      R^{x} represents a hydrogen atom or an organic group having 1 to 30 carbon atoms which may have a substituent, and
      A¹, A², B¹, B², Y¹ to Y⁶, L¹, L², P¹, P², Z¹, Z², Q, p, q, n and m are the same as defined above.
[13] The method of producing a polymerizable compound according to [12], wherein the Ar³-W¹C=N-D¹ and Ar⁴-W²C=N-D² each independently are represented by any of the following formulas (IX-1) to (IX-14): 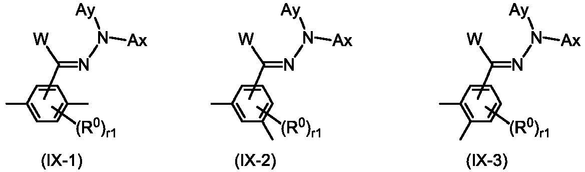 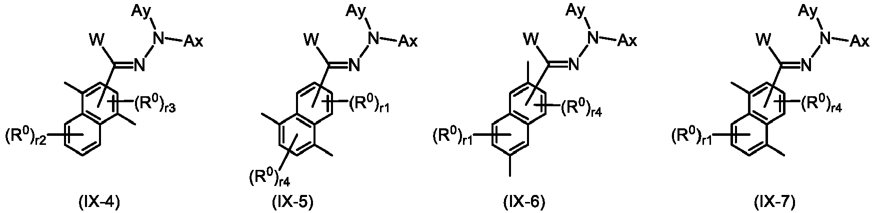 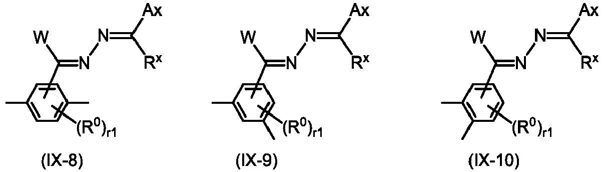 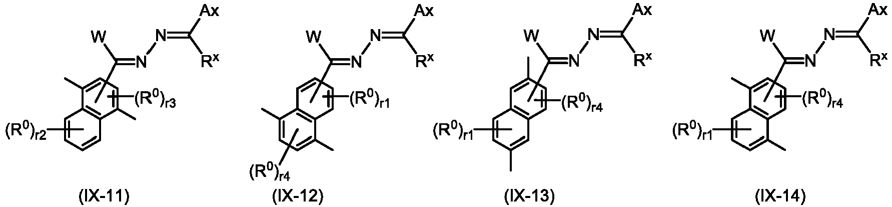 where in the formulas (IX-1) to (IX-14),
   Ax represents an organic group having at least one aromatic ring selected from the group consisting of an aromatic hydrocarbon ring having 6 to 30 carbon atoms and an aromatic heterocyclic ring having 2 to 30 carbon atoms, and the aromatic ring included in Ax may have a substituent,
   Ay represents a hydrogen atom or an organic group having 1 to 30 carbon atoms which may have a substituent,
   R^{x} represents a hydrogen atom or an organic group having 1 to 30 carbon atoms which may have a substituent,
   W represents a hydrogen atom or an alkyl group having 1 to 6 carbon atoms, and
   R⁰ represents a halogen atom; a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N,N-dialkylamino group having 2 to 12 carbon atoms, an alkoxy group having 1 to 6 carbon atoms, a nitro group, -C(=O)-R^{a1}, -O-C(=O)-R^{a1}, -C(=O)-O-R^{a1}, or -SO₂R^{a1}, where R^{a1} represents an alkyl group having 1 to 6 carbon atoms, or an aromatic hydrocarbon ring group having 6 to 20 carbon atoms which may have an alkyl group having 1 to 6 carbon atoms or an alkoxy group having 1 to 6 carbon atoms as a substituent, r1 is an integer from 0 to 3, r2 is an integer from 0 to 4, r3 is 0 or 1, and r4 is an integer from 0 to 2, with the proviso that when there is a plurality of R⁰, each R⁰ may be the same or may be different.
[14] The method of producing a polymerizable compound according to [12] or [13], wherein the Ax each independently represents the following formula (XI): 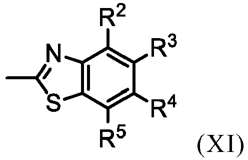 where in the formula (XI),
   R² to R⁵ each independently represent a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, -OCF₃; -O-C(=O)-R^{b1}, or -C(=O)-O-R^{b1},
   R^{b1} represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring group having 5 to 18 carbon atoms which may have a substituent, and
   each of R² to R⁵ may be the same or different, one or more ring constituent C-R² to C-R⁵ may be replaced by a nitrogen atom.
[15] The method of producing a polymerizable compound according to any one of [12] to [14], wherein the polymerizable compound represented by formula (VI) is represented by any of the following formulas (VI-1) to (VI-12): 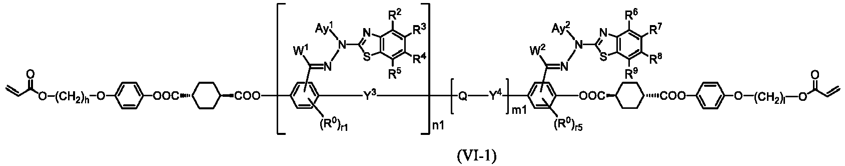 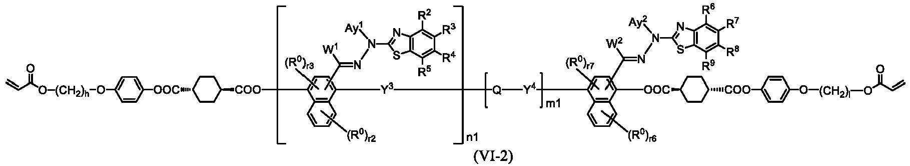 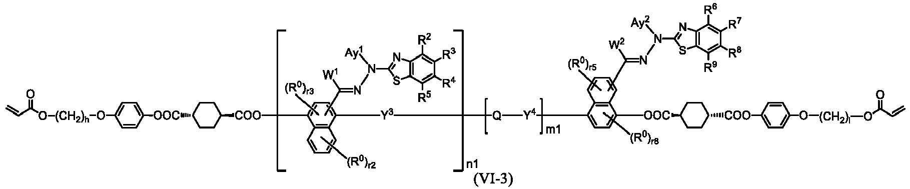 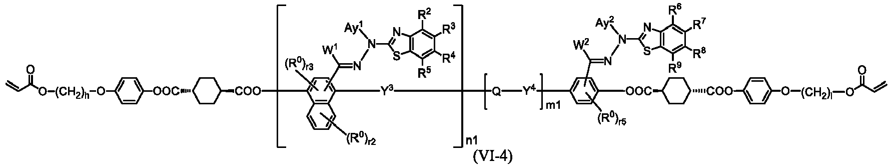 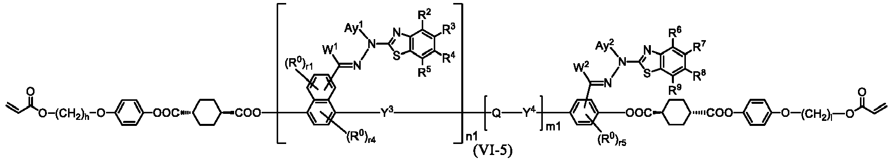 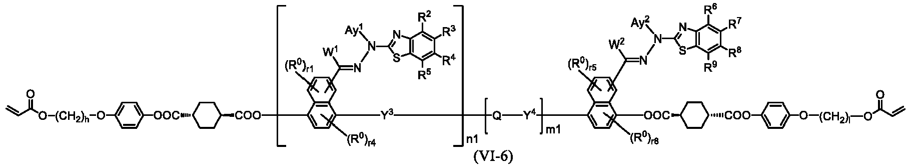 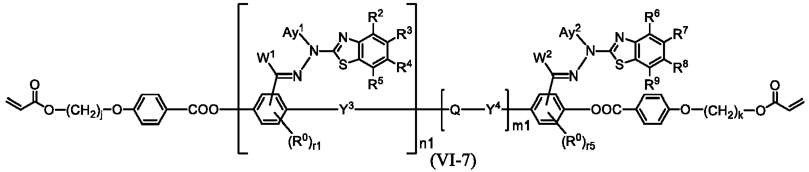 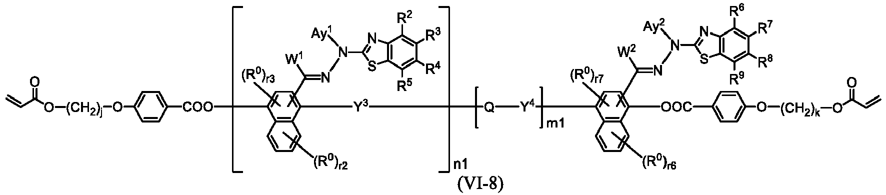 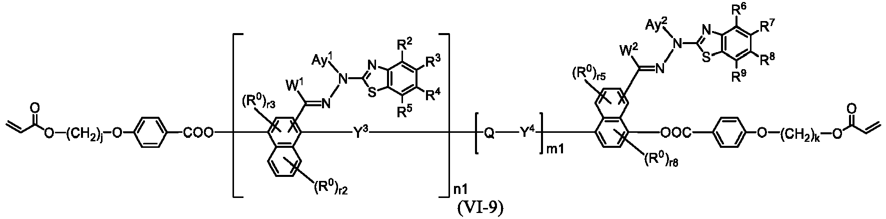 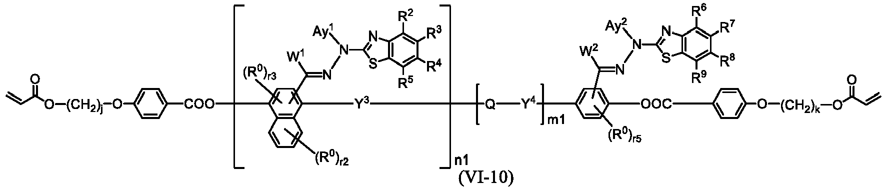 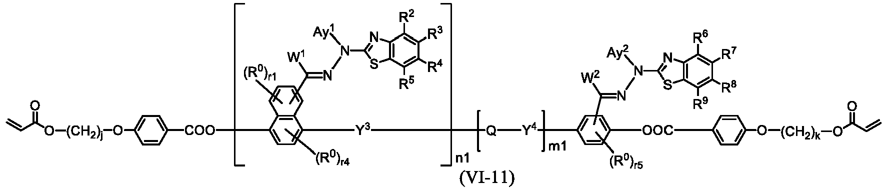 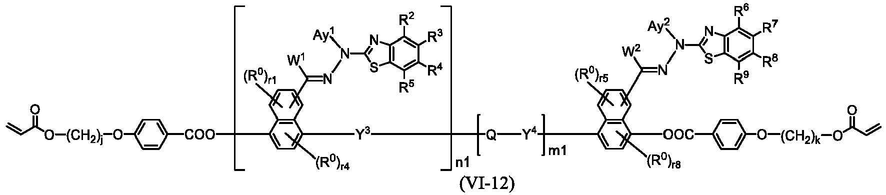 where in the formulas (VI-1) to (VI-12),
   W¹ and W² each independently represent a hydrogen atom or an organic group having 1 to 20 carbon atoms which may have a substituent,
   Ay¹ and Ay² each independently represent a hydrogen atom or an organic group having 1 to 30 carbon atoms which may have a substituent,
   n1 is an integer of 0 or 1,
   m1 is an integer of 0 or 1,
   R² to R⁹ each independently represent a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, -OCF₃, -O-C(=O)-R^{b1}, or -C(=O)-O-R^{b1},
   R^{b1} represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring group having 5 to 18 carbon atoms which may have a substituent,
   the plurality of R² to R⁹ may be the same or different, and one or more ring constituent C-R² to C-R⁹ may be replaced by a nitrogen atom,
   R⁰ represents a halogen atom; a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N,N-dialkylamino group having 2 to 12 carbon atoms, an alkoxy group having 1 to 6 carbon atoms, a nitro group, -C(=O)-R^{a1}, -O-C(=O)-R^{a1}, -C(=O)-O-R^{a1}, or -SO₂R^{a1}, where R^{a1} represents an alkyl group having 1 to 6 carbon atoms, or an aromatic hydrocarbon ring group having 6 to 20 carbon atoms which may have an alkyl group having 1 to 6 carbon atoms or an alkoxy group having 1 to 6 carbon atoms as a substituent, r1 and r5 each independently represent an integer from 0 to 3, r2 and r6 each independently represent an integer from 0 to 4, r3 and r7 each independently are 0 or 1, and r4 and r8 each independently represent an integer from 0 to 2, wherein, when there is a plurality of R⁰, each R⁰ may be the same or may be different,
   h, 1, j, and k each independently represent an integer from 1 to 18, and
   Y³, Y⁴, and Q are the same as defined above.
[16] The method of producing a polymerizable compound according to any one of [12] to [15], wherein the compound represented by formula (V) and an acid are added to a reaction solution obtained in the Step 1 to perform a reaction in the Step 2.
[17] The method of producing a polymerizable compound according to [16], wherein the acid is an inorganic acid or an organic acid having 1 to 20 carbon atoms.
[18] The method of producing a polymerizable compound according to [16] or [17], wherein the acid is an acidic aqueous solution, and the organic solvent is a water-immiscible organic solvent.
[19] The method of producing a polymerizable compound according to any one of [16] to [18], wherein the acid is at least one compound selected from the group consisting of hydrochloric acid, sulfuric acid, phosphoric acid, boric acid, sulfonic acid, sulfinic acid, formic acid, acetic acid and oxalic acid.
[20] A solution comprising the polymerizable compound represented by the formula (III) obtained using the method according to any one of [1] to [11], and a polymerizable compound represented by the following formula (XII): 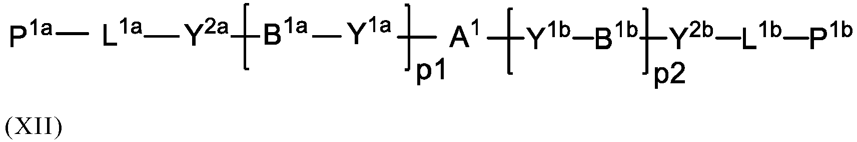 where in the formula (XII),
   A¹, B^{1a} and B^{1b} each independently represent a cyclic aliphatic group which may have a substituent, or an aromatic group which may have a substituent,
   Y^{1a}, Y^{1b}, Y^{2a} and Y^{2b} each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, -NR²¹-C(=O)-NR²²-, -O-CH₂-, -O-CH₂-CH₂-, -CH₂-O-, -CH₂-CH₂-O-, -CH₂-O-CH₂-, -CH₂-CH₂-O-CH₂-, -CH₂-O-CH₂-CH₂-, -CH₂-CH₂-O-CH₂-CH₂-, -C(=O)-CH₂-, -C(=O)-CH₂-CH₂-, -CH₂-C(=O)-, -CH₂-CH₂-C(=O)-, -O-C(=O)-CH₂-, -CH₂-C(=O)-O-, -O-C(=O)-CH₂-CH₂-, -CH₂-CH₂-C(=O)-O-, -NR²¹-C(=O)-CH₂-, -NR²¹-C(=O)-CH₂-CH₂-, -CH₂-C(=O)-NR²¹-, -CH₂-CH₂-C(=O)-NR²¹-, -O-C(=O)-O-CH₂-, -O-C(=O)-O-CH₂-CH₂-, -CH₂-O-C(=O)-O-, -CH₂-CH₂-O-C(=O)-O-, -CH₂-O-C(=O)-O-CH₂-, -CH₂-CH₂-O-C(=O)-O-CH₂-, -CH₂-O-C(=O)-O-CH₂-CH₂-, -CH₂-CH₂-O-C(=O)-O-CH₂-CH₂-, -NR²¹-C(=O)-NR²²-CH₂-, -NR²¹-C(=O)-NR²²-O-CH₂-CH₂-, -CH₂-CH₂-O-NR²¹-C(=O)-NR²²-, -CH₂-NR²¹-C(=O)-NR²²-, -CH₂-CH₂-NR²¹-C(=O)-NR²²-, -NR²¹-C(=O)-NR²²-CH₂-CH₂-, -CH₂-NR²¹-C(=O)-NR²²-CH₂-, -CH₂-CH₂-NR²¹-C(=O)-NR²²-CH₂-, -CH₂-NR²¹-C(=O)-NR²²-CH₂-CH₂-, -CH₂-NR²¹-C(=O)-NR²²-O-CH₂-CH₂-, -CH₂-CH₂-O-NR²¹-C(=O)-NR²²-CH₂-, -CH₂-CH₂-NR²¹-C(=O)-NR²²-O-CH₂-CH₂-, or -CH₂-CH₂-O-NR²¹-C(=O)-NR²²-CH₂-CH₂-, where R²¹ and R²² each independently represent a hydrogen atom or an alkyl group having 1 to 6 carbon atoms,
   L^{1a} and L^{1b} each independently represent an organic group which is either an alkylene group having 1 to 20 carbon atoms, or an alkylene group having 3 to 20 carbon atoms in which at least one methylene group (-CH₂-) contained in the alkylene group is substituted by -O- or -C(=O)-, and the hydrogen atom included in the organic group of L^{1a} and L^{1b} may be substituted by an alkyl group having 1 to 5 carbon atoms, an alkoxy group having 1 to 5 carbon atoms, or a halogen atom, with the proviso that the methylene groups (-CH₂-) on both ends of L^{1a} and L^{1b} are not substituted with -O- or -C(=O)-,
   P^{1a} and P^{1b} each independently represent a polymerizable group, and
   p1 and p2 each independently represent an integer from 0 to 3.
[21] A solution comprising the polymerizable compound represented by formula (VI) obtained using the method according to any one of [12] to [19] and a polymerizable compound represented by the following formula (XII): 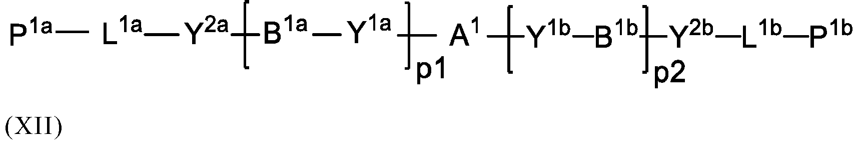 where in the formula (XII),
   A¹, B^{1a} and B^{1b} each independently represent a cyclic aliphatic group which may have a substituent, or an aromatic group which may have a substituent,
   Y^{1a}, Y^{1b}, Y^{2a} and Y^{2b} each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, -NR²¹-C(=O)-NR²²-, -O-CH₂-, -O-CH₂-CH₂-, -CH₂-O-, -CH₂-CH₂-O-, -CH₂-O-CH₂-, -CH₂-CH₂-O-CH₂-, -CH₂-O-CH₂-CH₂-, -CH₂-CH₂-O-CH₂-CH₂-, -C(=O)-CH₂-, -C(=O)-CH₂-CH₂-, -CH₂-C(=O)-, -CH₂-CH₂-C(=O)-, -O-C(=O)-CH₂-, -CH₂-C(=O)-O-, -O-C(=O)-CH₂-CH₂-, -CH₂-CH₂-C(=O)-O-, -NR²¹-C(=O)-CH₂-, -NR²¹-C(=O)-CH₂-CH₂-, -CH₂-C(=O)-NR²¹-, -CH₂-CH₂-C(=O)-NR²¹-, -O-C(=O)-O-CH₂-, -O-C(=O)-O-CH₂-CH₂-, -CH₂-O-C(=O)-O-, -CH₂-CH₂-O-C(=O)-O-, -CH₂-O-C(=O)-O-CH₂-, -CH₂-CH₂-O-C(=O)-O-CH₂-, -CH₂-O-C(=O)-O-CH₂-CH₂-, -CH₂-CH₂-O-C(=O)-O-CH₂-CH₂-, -NR²¹-C(=O)-NR²²-CH₂-, -NR²¹-C(=O)-NR²²-O-CH₂-CH₂-, -CH₂-CH₂-O-NR²¹-C(=O)-NR²²-, -CH₂-NR²¹-C(=O)-NR²²-, -CH₂-CH₂-NR²¹-C(=O)-NR²²-, -NR²¹-C(=O)-NR²²-CH₂-CH₂-, -CH₂-NR²¹-C(=O)-NR²²-CH₂-, -CH₂-CH₂-NR²¹-C(=O)-NR²²-CH₂-, -CH₂-NR²¹-C(=O)-NR²²-CH₂-CH₂-, -CH₂-NR²¹-C(=O)-NR²²-O-CH₂-CH₂-, -CH₂-CH₂-O-NR²¹-C(=O)-NR²²-CH₂-, -CH₂-CH₂-NR²¹-C(=O)-NR²²-O-CH₂-CH₂-, or -CH₂-CH₂-O-NR²¹-C(=O)-NR²²-CH₂-CH₂-, where R²¹ and R²² each independently represent a hydrogen atom or an alkyl group having 1 to 6 carbon atoms,
   L^{1a} and L^{1b} each independently represent an organic group which is either an alkylene group having 1 to 20 carbon atoms, or an alkylene group having 3 to 20 carbon atoms in which at least one methylene group (-CH₂-) contained in the alkylene group is substituted by -O- or -C(=O)-, and the hydrogen atom included in the organic group of L^{1a} and L^{1b} may be substituted by an alkyl group having 1 to 5 carbon atoms, an alkoxy group having 1 to 5 carbon atoms, or a halogen atom, with the proviso that the methylene groups (-CH₂-) on both ends of L^{1a} and L^{1b} are not substituted with -O- or -C(=O)-,
   P^{1a} and P^{1b} each independently represent a polymerizable group, and
   p1 and p2 each independently represent an integer from 0 to 3.

### (Advantageous Effect)

The present disclosure provides a method of producing a polymerizable compound that enables production of, in high yield, a polymerizable compound used for producing an optical film or the like.

The present disclosure also provides a solution comprising a polymerizable compound produced by the aforementioned production method.

### DETAILED DESCRIPTION

The present disclosure will be described in detail below. Note that, in the present disclosure, "may have a substituent" means "unsubstituted, or having a substituent". Further, when an organic group such as an alkyl group or an aromatic hydrocarbon ring group contained in the general formula has a substituent, the number of carbon atoms of the organic group having the substituent does not include the number of carbon atoms of the substituent. For example, when an aromatic hydrocarbon ring group having 6 to 20 carbon atoms is the substituent, the number of carbon atoms of the aromatic hydrocarbon ring group having 6 to 20 does not include the number of carbon atoms of such a substituent. Furthermore, in the present disclosure, the phrase "alkyl group" means a chain (linear or branched) saturated hydrocarbon group, and the "alkyl group" does not include a "cyclic alkyl group" which is a cyclic saturated hydrocarbon group.

### ((1-1) Method of producing the polymerizable compound (Method of producing a first compound))

The method of producing the compound of the present disclosure (method of producing the first compound) contains the Step 1 for reacting a compound represented by the following formula (I) (hereinafter, referred to as "compound (I)") with a compound represented by the following formula (II) (hereinafter, referred to as "compound (II)") in an organic solvent in which base having a pKa from 6.1 to 9.5 is present to obtain a reaction solution containing the polymerizable compound represented by the following formula (III) (hereinafter, referred to as "compound (III)").

### «Compound represented by formula (I)»

The compound represented by formula (I) will be described below.

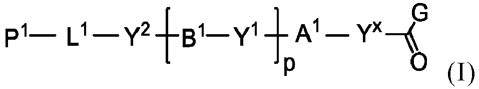

In the aforementioned formula (I), Y^{x} is a single bond, -CH₂-, -CH₂-CH₂-, or -CH=CH-.

In the aforementioned formula (I), A¹ and B¹ each independently represent a cyclic aliphatic group which may have a substituent, or an aromatic group which may have a substituent, and are preferably a cyclic aliphatic group having 5 to 20 carbon atoms which may have a substituent, or an aromatic group having 2 to 20 carbon atoms which may have a substituent. Furthermore, A¹ is preferably a cyclic aliphatic group having 5 to 20 carbon atoms which may have a substituent, and B¹ is preferably an aromatic group having 2 to 20 carbon atoms which may have a substituent. When a plurality of B¹ are present, these may be the same or different.

Specific examples of the cyclic aliphatic group include a cycloalkanediyl group having 5 to 20 carbon atoms such as a cyclopentane-1,3-diyl group, a cyclohexane-1,4-diyl group, a 1,4-cycloheptane-1,4-diyl group, and a cyclooctane-1,5-diyl group; a bicycloalkanediyl group having 5 to 20 carbon atoms such as a decahydronaphthalene-1,5-diol group, a decahydronaphthalene-2,6-diol group and the like. Thereamong, a cycloalkanediyl group having 5 to 20 carbon atoms which may have a substituent is preferable as the cyclic aliphatic group, a cyclohexanediol group is more preferable, and specifically, a cyclohexane-1,4-diol group represented by the following formula (a) is preferable. The cyclic aliphatic group may be a trans-isomer represented by formula (a1), a cis-isomer represented by formula (a2), or may be a mixture of cis- and trans-isomers, but a trans-isomer represented by formula (a1) is more preferable.

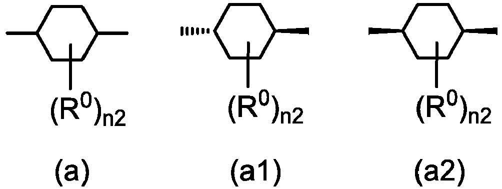

In the aforementioned formulas (a), (a1) and (a2), R⁰ represents a halogen atom such as a fluorine atom, a chlorine atom, and a bromine atom; a cyano group; an alkyl group having 1 to 6 carbon atoms such as a methyl group, an ethyl group, a propyl group, an isopropyl group, a butyl group, a sec-butyl group, and a tertiary butyl group; an alkenyl group having 2 to 6 carbon atoms; an alkyl group having 1 to 6 carbon atoms in which at least one hydrogen atom is substituted with a halogen atom, an N,N-dialkylamino group having 2 to 12 carbon atoms; an alkoxy group having 1 to 6 carbon atoms such as a methoxy group and an ispropoxy group; a nitro group; -C(=O)-R^{a1}, -O-C(=O)-R^{a1}, -C(=O)-O-R^{a1}; or -SO₂R^{a1}, where R^{a1} represents an alkyl group having 1 to 6 carbon atoms such as a methyl group and an ethyl group, or an aromatic hydrocarbon ring group having 6 to 20 carbon atoms which may have an alkoxy group having 1 to 6 carbon atoms or an alkyl group having 1 to 6 carbon atoms as a substituent, such as a phenyl group, a 4-methylphenyl group, or a 4-methoxyphenyl group. When there is a plurality of substituents, the plurality of substituents may be the same or different from each other. From the viewpoint of solubility improvement, R⁰ is preferably a halogen atom; a cyano group, an alkyl group having 1 to 6 carbon atoms, an alkyl group having 1 to 6 carbon atoms in which at least one hydrogen atom is substituted with a halogen atom, an alkoxy group having 1 to 6 carbon atoms, a nitro group. When there is a plurality of R⁰, the plurality of substituents may be the same or different from each other.

Furthermore, in the aforementioned formulas (a), (a1) and (a2), n2 is an integer from 0 to 4. Moreover, it is preferable that n2 = 0.

Examples of the aromatic group include an aromatic hydrocarbon ring group having 6 to 20 carbon atoms such as a 1,2-phenylene group, a 1,3-phenylene group, a 1,4-phenylene group, a 1,4-naphthalene group, a 1,5-naphthalene group, a 2,6-naphthalene group, and a 4,4'-biphenylene group; an aromatic heterocyclic ring group having 2 to 20 carbon atoms such as a furan-2,5-diol group, a thiophene-2,5-diol group, a pyridine-2,5-diyl group and a pyrazine-2,5-diyl group; and the like. Thereamong, an aromatic hydrocarbon ring group having 6 to 20 carbon atoms is preferable as the aromatic group, a phenylene group is more preferable, and specifically, a 1,4-phenylene group represented by the following formula (b) is preferable. 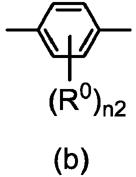 where R⁰ and n2 are the same as defined above, and the preferred examples thereof are the same.

Further, regarding the combination of A¹ and B¹, A¹ is preferably the aforementioned formula (a), (a1) or (a2), and B¹ is preferably the aforementioned formula (b), and furthermore, A¹ is particularly preferably the aforementioned formula (a1), and B¹ is particularly preferably the aforementioned formula (b).

In the aforementioned formula (I), Y¹ and Y² each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, -NR²¹-C(=O)-NR²²-, -O-CH₂-, -O-CH₂-CH₂-, -CH₂-O-, -CH₂-CH₂-O-, -CH₂-O-CH₂-, -CH₂-CH₂-O-CH₂-, -CH₂-O-CH₂-CH₂-, -CH₂-CH₂-O-CH₂-CH₂-, -C(=O)-CH₂-, -C(=O)-CH₂-CH₂-, -CH₂-C(=O)-, -CH₂-CH₂-C(=O)-, -O-C(=O)-CH₂-, -CH₂-C(=O)-O-, -O-C(=O)-CH₂-CH₂-, -CH₂-CH₂-C(=O)-O-, -NR²¹-C(=O)-CH₂-, -NR²¹-C(=O)-CH₂-CH₂-, -CH₂-C(=O)-NR²¹-, -CH₂-CH₂-C(=O)-NR²¹-, -O-C(=O)-O-CH₂-, -O-C(=O)-O-CH₂-CH₂-, -CH₂-O-C(=O)-O-, -CH₂-CH₂-O-C(=O)-O-, -CH₂-O-C(=O)-O-CH₂-, -CH₂-CH₂-O-C(=O)-O-CH₂-, -CH₂-O-C(=O)-O-CH₂-CH₂-, -CH₂-CH₂-O-C(=O)-O-CH₂-CH₂-, -NR²¹-C(=O)-NR²²-CH₂-, -NR²¹-C(=O)-NR²²-O-CH₂-CH₂-, -CH₂-CH₂-O-NR²¹-C(=O)-NR²²-, -CH₂-NR²¹-C(=O)-NR²²-, -CH₂-CH₂-NR²¹-C(=O)-NR²²-, -NR²¹-C(=O)-NR²²-CH₂-CH₂-, -CH₂-NR²¹-C(=O)-NR²²-CH₂-, -CH₂-CH₂-NR²¹-C(=O)-NR²²-CH₂-, -CH₂-NR²¹-C(=O)-NR²²-CH₂-CH₂-, -CH₂-NR²¹-C(=O)-NR²²-O-CH₂-CH₂-, -CH₂-CH₂-O-NR²¹-C(=O)-NR²²-CH₂-, -CH₂-CH₂-NR²¹-C(=O)-NR²²-O-CH₂-CH₂-, or -CH₂-CH₂-O-NR²¹-C(=O)-NR²²-CH₂-CH₂-, where R²¹ and R²² each independently represent a hydrogen atom or an alkyl group having 1 to 6 carbon atoms.

Thereamong, Y¹ and Y² each independently preferably represent -O-, -C(=O)-, -C(=O)-O-, -O-C(=O)-O-, or -O-C(=O)-.

When a plurality of Y¹ are present, these may be the same or different.

In the aforementioned Formula (I), L¹ is an organic group which is either an alkylene group having 1 to 20 carbon atoms, or an alkylene group having 3 to 20 carbon atoms in which at least one methylene group (-CH₂-) contained in the alkylene group is substituted by -O- or -C(=O)-, and the hydrogen atom included in the organic group of L¹ may be substituted by an alkyl group having 1 to 5 carbon atoms, an alkoxy group having 1 to 5 carbon atoms, or a halogen atom. Note that, in "an alkylene group having 3 to 20 carbon atoms group in which at least one methylene group (-CH₂-) contained in the alkylene group is substituted with -O- or -C(=O)-", -O-preferably does not replace the consecutive methylene groups in the alkylene group (i.e., the -O-O- configuration is not formed), and -C(=O)-preferably does not replace the consecutive methylene groups in the alkylene group (i.e., the -C(=O)-C(=O)- configuration is not formed).

Here, a group represented by an alkylene group having 1 to 20 carbon atoms which may be substituted with a fluorine atom, or -(CH₂)ₓ-C(=O)-O-(CH₂)_{y}- which may be substituted with a fluorine atom (in the formula, x and y each represent an integer from 2 to 12, and preferably represent an integer from 2 to 8) is preferable as the organic group of L¹, an alkylene group having 2 to 12 carbon atoms which may be substituted with a fluorine atom is more preferable, an unsubstituted alkylene group having 2 to 12 carbon atoms is even more preferable, and the group represented by -(CH₂)z- (in the formula, z represents an integer from 2 to 12, and preferably represents an integer from 2 to 8) is particularly preferable.

In the aforementioned formula (I), P¹ represents a hydrogen atom or a polymerizable group. Here, P¹ preferably represents a polymerizable group.

Here, examples of the polymerizable group of P¹ include the group represented by CH₂=CR¹-C(=O)-O- (R¹ represents a hydrogen atom, a methyl group or a chlorine atom) such as an acryloyloxy group and a methacryloyloxy group, a vinyl group, a vinyl ether group, a p-stilbene group, an acryloyl group, a methacryloyl group, a carboxyl group, a methyl carbonyl group, a hydroxyl group, an amide group, an alkylamino group having 1 to 4 carbon atoms, an amino group, an epoxy group, an oxetanyl group, an aldehyde group, an isocyanate group or a thioisocyanate group and the like. Thereamong, as in the following formula (IV), the group represented by CH₂=CRc-C(=O)-O- is preferable, CH₂=CH-C(=O)-O-(acryloyloxy group) and CH₂-C(CH₃)-C(=O)-O- (methacryloyloxy group) are more preferable, and an acryloyloxy group is even more preferable. 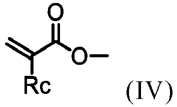 where Rc represents a hydrogen atom, a methyl group or a chlorine atom.

In the aforementioned formula (I), p is an integer from 0 to 3, and preferably an integer from 0 to 2, and more preferably 0 or 1.

In the aforementioned formula (I), G represents a leaving group.

Here, examples of the leaving group of G include a halogen atom such as a chlorine atom, a bromine atom, and an iodine atom; an organic sulfonyloxy group such as a methanesulfonyloxy group, a p-toluenesulfonyloxy group, a trifluoromethylsulfonyloxy group, and a camphorsulfonyloxy group; and the like. From the viewpoint of obtaining the target product at a low cost and a high yield, a halogen atom is preferable, and a chlorine atom is more preferable.

The compound represented by the aforementioned formula (I) may be in a mixture comprising the polymerizable compound represented by the following formula (XII):

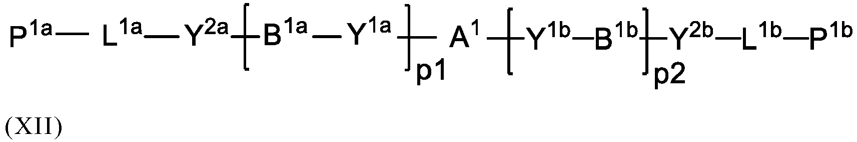

In the aforementioned formula (XII), A¹ is the same as defined above, and the preferred examples are the same, B^{1a} and B^{1b} are the same as defined above for B¹, and the preferred examples are the same, Y^{1a}, Y^{1b}, Y^{2a} and Y^{2b} are the same as defined above for Y¹ and Y², and the preferred examples are the same, L^{1a} and L^{1b} are the same as defined above for L¹, and the preferred examples are the same, P^{1a} and P^{1b} are the same as defined above for P¹, and the preferred examples are the same, and p1 and p2 are the same as defined above for p, and the preferred examples are the same.

### «Compound represented by formula (II)»

The compound represented by formula (II) will be described below.

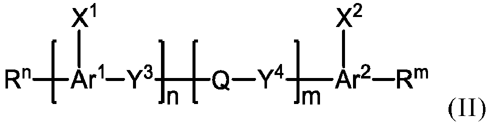

In the aforementioned formula (II), Ar¹ and Ar² each independently represent an aromatic hydrocarbon ring group which may have a substituent, or an aromatic heterocyclic ring group which may have a substituent.

Moreover, examples of the aromatic hydrocarbon ring group of Ar¹ and Ar² include a 1,4-phenylene group, a 1,3-phenylene group, a 1,2-phenylene group, a 1,4-naphthalene group, a 2,6-naphthalene group, a 1,5-naphthalene group, an anthracenyl-9,10-diol group, an anthracenyl-1,4-diol group, an andanthracenyl-2,6-diol group and the like.

Thereamong, a 1,4-phenylene group, a 1,4-naphthalene group or a 2,6-naphthalene group is preferable as the aromatic hydrocarbon ring group, and a 1,4-phenylene group is particularly preferable.

Further, examples of the aromatic heterocyclic ring group of Ar¹ and Ar² include a benzothiazole-4,7-diyl group, a 1,2-benzoisothiazole-4,7-diyl group, a benzoxazole-4,7-diyl group, indole-4,7-diyl group, a benzimidazole-4,7-diyl group, a benzopyrazole-4,7-diyl group, a 1-benzofuran-4,7-diyl group, a 2-benzofuran-4,7-diyl group, a benzo[1,2-d:4,5-d']dithiazolyl-4,8-diol group, a benzo[1,2-d:5,4-d']dithiazolyl-4,8-diol group, a benzothiophenyl-4,7-diyl group, a 1H-isoindole-1,3 (2H)-diol-4,7-diyl group, a benzo[1,2-b:5,4-b']dithiophenyl-4,8-diol group, a benzo[1,2-b:4,5-b']dithiophenyl-4,8-diol group, a benzo[1,2-b:5,4-b']difuranyl-4,8-diol group, a benzo[1,2-b:4,5-b']difuranyl-4,8-diol group, a benzo[2,1-b:4,5-b']dipyrrole-4,8-diol group, a benzo[1,2-b:5,4-b']dipyrrole-4,8-diol group, and a benzo[1,2-d:4,5-d']diimidazole-4,8-diol group and the like.

Thereamong, the aromatic heterocyclic ring group is preferably a benzothiazole-4,7-diyl group, a benzoxazole-4,7-diyl group, 1-benzofuran-4,7-diyl group, a 2-benzofuran-4,7-diyl group, a benzo[1,2-d:4,5-d']dithiazolyl-4,8-diol group, a benzo[1,2-d:5,4-d']dithiazolyl-4,8-diol group, a benzothiophenyl-4,7-diyl group, 1H-isoindole-1,3 (2H)-diol-4,7-diyl group, benzo[1,2-b:5,4-b']dithiophenyl-4,8-diol group, a benzo[1,2-b:4,5-b']dithiophenyl-4,8-diol group, a benzo[1,2-b:5,4-b']difuranyl-4,8-diol group or a benzo[1,2-b:4,5-b']difuranyl-4,8-diol group.

The substituent of the aromatic hydrocarbon ring group and the aromatic heterocyclic ring group represented a halogen atom such as a fluorine atom, a chlorine atom, and a bromine atom; a cyano group; an alkyl group having 1 to 6 carbon atoms such as a methyl group, an ethyl group, a propyl group, an isopropyl group, a butyl group, a sec-butyl group, and a tertiary butyl group; an alkenyl group having 2 to 6 carbon atoms; an alkyl group having 1 to 6 carbon atoms in which at least one hydrogen atom is substituted with a halogen atom, an N,N-dialkylamino group having 2 to 12 carbon atoms; an alkoxy group having 1 to 6 carbon atoms such as a methoxy group and an isopropoxy group; a nitro group; -C(=O)-R^{a1}, -O-C(=O)-R^{a1}, -C(=O)-O-R^{a1}; or -SO₂R^{a1}, where R^{a1} represents an alkyl group having 1 to 6 carbon atoms (e.g., methyl group and ethyl group) or an aromatic hydrocarbon ring group having 6 to 20 carbon atoms which may have an alkyl group having 1 to 6 carbon atoms or an alkoxy group having 1 to 6 carbon atoms as a substituent (e.g., phenyl group, 4-methylphenyl group, and 4-methoxyphenyl group). Note that when there is a plurality of substituents, each substituent may be the same or different.

In the aforementioned formula (II), X¹ and X² each independently represent -CHO, or -C(=O)-R^{a}, where R^{a} represents an organic group having 1 to 20 carbon atoms which may have a substituent.

Examples of the organic group having 1 to 20 carbon atoms of R^{a} include an alkyl group having 1 to 6 carbon atoms such as a methyl group and an ethyl group and the like. Thereamong, a methyl group is preferable.

In the aforementioned formula (II), Y³ and Y⁴ are the same as defined above for Y¹ and Y², and the preferred examples are the same.

In the aforementioned formula (II), Q represents an organic group having 1 to 20 carbon atoms which may have a substituent.

Examples of the organic groups having 1 to 20 carbon atoms of Q include an alkylene group having 1 to 18 carbon atoms which may have a substituent, a cyclic aliphatic group having 3 to 18 carbon atoms which may have a substituent, and an aromatic hydrocarbon ring group having 6 to 18 carbon atoms which may have a substituent.

Examples of the substituents of Q include a halogen atom such as a fluorine atom, and a chlorine atom; a cyano group; an alkyl group having 1 to 6 carbon atoms such as a methyl group, an ethyl group, a propyl group, an isopropyl group, a butyl group, a sec-butyl group, and a tertiary butyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group; an N,N-dialkylamino group having 2 to 12 carbon atoms such as a dimethylamino group; an alkoxy group having 1 to 6 carbon atoms such as a methoxy group, an ethoxy group and an isopropoxy group; a nitro group; an aromatic hydrocarbon ring group having 6 to 20 carbon atoms such as a phenyl group and a naphthyl group; -OCF₃; -C(=O)-R^{b1}; -O-C(=O)-R^{b1}; -C(=O)-O-R^{b1}; -O-C(=O)-R^{b1} or -SO₂R^{a1}.

R^{a1} is the same as defined above, and the preferred examples are the same.

R^{b1} represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring group having 5 to 18 carbon atoms which may have a substituent.

When there is a plurality of substituents, the plurality of substituents may be the same or different from each other.

From the viewpoint of solubility improvement, the substituents of Q are preferably a halogen atom, a cyano group, an alkyl group having 1 to 6 carbon atoms, an alkyl group having 1 to 6 carbon atoms in which at least one hydrogen atom is substituted with a halogen atom, an alkoxy group having 1 to 6 carbon atoms, or a nitro group.

When Q has a plurality of the aforementioned substituents, the substituents may be the same or different.

Examples of the alkyl group of R^{b1} having 1 to 20 carbon atoms in the case when there is an alkyl group having 1 to 20 carbon atoms which may have a substituent include a methyl group, an ethyl group, an n-propyl group, an isopropyl group, a n-butyl group, an isobutyl group, a 1-methylpentyl group, a 1-ethylpentyl group, a sec-butyl group, a t-butyl group, a n-pentyl group, an isopentyl group, a neopentyl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, a n-nonadecyl group, and an n-icosyl group and the like. Note that, the number of carbon atoms of the alkyl group having 1 to 20 carbon atoms which may have a substituent is preferably 1 to 12, and even more preferably 4 to 10.

Examples of the alkenyl group of R^{b1} in the case when the number of carbon atoms of an alkenyl group having 2 to 20 carbon atoms which may have a substituent is from 2 to 20 includes a vinyl group, a propenyl group, an isopropenyl group, a butenyl group, an isobutenyl group, a pentenyl group, a hexenyl group, a heptenyl group, an octenyl group, a decenyl group, an undecenyl group, a dodecenyl group, a tridecenyl group, a tetradecenyl group, a pentadecenyl group, a hexadecenyl group, a heptadecenyl group, an octadecenyl group, a nonadenyl group, and an icosenyl group and the like.

The number of carbons of the alkenyl group having 2 to 20 carbon atoms which may have a substituent is preferably 2 to 12.

Examples of the substituents of the alkyl group having 1 to 20 carbon atoms and the alkenyl group having 2 to 20 carbon atoms of R^{b1} include a halogen atom such as a fluorine atom, and a chlorine atom, a cyano group; an N,N-dialkylamino group having 2 to 12 carbon atoms such as a dimethylamino group; an alkoxy group having 1 to 20 carbon atoms such as a methoxy group, an ethoxy group, an isopropoxy group; an alkoxy group having 1 to 12 carbon atoms substituted with an alkoxy group having 1 to 12 carbon atoms such as a methoxymethoxy group and a methoxyethoxy group; a nitro group; an aromatic hydrocarbon ring group having 6 to 20 carbon atoms such as a phenyl group and a naphthyl group; an aromatic heterocyclic ring group having 2 to 20 carbon atoms such as a triazolyl group, a pyrrolyl group, a furanyl group, a thiophenyl group and a benzothiazole-2-yl group; a cycloalkyl group having 3 to 8 carbon atoms such as a cyclopropyl group, a cyclopentyl group and a cyclohexyl group; a cycloalkyloxy group having 3 to 8 carbon atoms such as a cyclopentyloxy group and a cyclohexyloxy group; a cyclic ether group having 2 to 12 carbon atoms such as a tetrahydrofuranyl group, a tetrahydropyranyl group, a dioxolanyl group and a dioxanyl group; an aryloxy group having 6 to 14 carbon atoms such as a phenoxy group and a naphthoxy group; a fluoroalkyl group having 1 to 12 carbon atoms in which at least one hydrogen atom is substituted with a fluorine atom such as a trifluoromethyl group, a pentafluoroethyl group, and -CH₂CF₃; a benzofuryl group; a benzopyranyl group; a benzodioxolyl group; a benzodioxanyl group and the like. Thereamong, examples of the substituents of the alkyl group having 1 to 20 carbon atoms and the alkenyl group having 2 to 20 carbon atoms of R^{b1} are preferably a halogen atom such as a fluorine atom, and a chlorine atom, a cyano group; an alkoxy group having 1 to 20 carbon atoms such as a methoxy group, an ethoxy group, an isopropoxy group; a nitro group; an aromatic hydrocarbon ring group having 6 to 20 carbon atoms such as a phenyl group and a naphthyl group; an aromatic heterocyclic ring group having 2 to 20 carbon atoms such as a furanyl group and a thiophenyl group; a cycloalkyl group having 3 to 8 carbon atoms such as a cyclopropyl group, a cyclopentyl group and a cyclohexyl group; a fluoroalkyl group having 1 to 12 carbon atoms in which at least one hydrogen atom is substituted with a fluorine atom such as a trifluoromethyl group, a pentafluoroethyl group, and -CH₂CF₃.

Note that, the alkyl group having 1 to 20 carbon atoms and the alkenyl group having 2 to 20 carbon atoms of R^{b1} may have a plurality of substituents selected from the aforementioned substituents. When the alkyl group having 1 to 20 carbon atoms and the alkenyl group having 2 to 20 carbon atoms of R^{b1} have a plurality of substituents, the plurality of substituents may be the same or different.

Examples of the cycloalkyl group having 3 to 12 carbon atoms of R^{b1} in the case of the cycloalkyl group having 3 to 12 carbon atoms which may have a substituent include a cyclopropyl group, a cyclobutyl group, a cyclopentyl group, a cyclohexyl group, a cyclooctyl group and the like. Thereamong, a cyclopentyl group and a cyclohexyl group are preferable.

Examples of the substituents of the cycloalkyl group having 3 to 12 carbon atoms of R^{b1} inlcude a halogen atom such as a fluorine atom, and a chlorine atom, a cyano group; an N,N-dialkylamino group having 2 to 12 carbon atoms such as a dimethylamino group;an alkyl group having 1 to 6 carbon atoms such as a methyl group, an ethyl group and a propyl group; an alkoxy group having 1 to 6 carbon atoms such as a methoxy group, an ethoxy group and an isopropoxy group; a nitro group; and, an aromatic hydrocarbon ring group having 6 to 20 carbon atoms such as a phenyl group and a naphthyl group and the like. Thereamong, the substituents of the cycloalkyl group having 3 to 12 carbon atoms of R^{b1} are preferably a halogen atom such as a fluorine atom, and a chlorine atom; a cyano group; an alkyl group having 1 to 6 carbon atoms such as a methyl group, an ethyl group, and a propyl group; an alkoxy group having 1 to 6 carbon atoms such as a methoxy group, an ethoxy group and an isopropoxy group; a nitro group; and, an aromatic hydrocarbon ring group having 6 to 20 carbon atoms such as a phenyl group and a naphthyl group.

Note that, the cycloalkyl group having 3 to 12 carbon atoms of R^{b1} may have a plurality of substituents. When the cycloalkyl group having 3 to 12 carbon atoms of R^{b1} has a plurality of substituents, the plurality of substituents may be the same or different.

Examples of the aromatic hydrocarbon ring group having 5 to 18 carbon of R^{b1} in the case of the aromatic hydrocarbon ring group having 5 to 18 carbon atoms which may have a substituent include a phenyl group, a 1-naphthyl group, a 2-naphthyl group and the like. Thereamong, a phenyl group is preferable.

Examples of the substituents of the aromatic hydrocarbon ring group having 5 to 18 carbon atoms which may have a substituent include a halogen atom such as a fluorine atom, and a chlorine atom, a cyano group; an N,N-dialkylamino group having 2 to 12 carbon atoms such as a dimethylamino group; an alkoxy group having 1 to 20 carbon atoms such as a methoxy group, an ethoxy group, an isopropoxy group; an alkoxy group having 1 to 12 carbon atoms substituted with an alkoxy group having 1 to 12 carbon atoms such as a methoxymethoxy group and a methoxyethoxy group; a nitro group; an aromatic hydrocarbon ring group having 6 to 20 carbon atoms such as a phenyl group and a naphthyl group; an aromatic heterocyclic ring group having 2 to 20 carbon atoms such as a triazolyl group, a pyrrolyl group, a furanyl group and a thiophenyl group; a cycloalkyl group having 3 to 8 carbon atoms such as a cyclopropyl group, a cyclopentyl group and a cyclohexyl group; a cycloalkyloxy group having 3 to 8 carbon atoms such as a cyclopentyloxy group and a cyclohexyloxy group; a cyclic ether group having 2 to 12 carbon atoms such as a tetrahydrofuranyl group, a tetrahydropyranyl group, a dioxolanyl group and a dioxanyl group; an aryloxy group having 6 to 14 carbon atoms such as a phenoxy group and a naphthoxy group; a fluoroalkyl group having 1 to 12 carbon atoms in which at least one hydrogen atom is substituted with a fluorine atom such as a trifluoromethyl group, a pentafluoroethyl group, and -CH₂CF₃; -OCF₃; a benzofuryl group; a benzopyranyl group; a benzodioxolyl group; a benzodioxanyl group and the like. Thereamong, the substituent of the aromatic hydrocarbon ring group having 5 to 18 carbon atoms is preferably one substituent selected from a halogen atom such as a fluorine atom, and a chlorine atom, a cyano group; an alkoxy group having 1 to 20 carbon atoms such as a methoxy group, an ethoxy group, an isopropoxy group ;nitro group; an aromatic hydrocarbon ring group having 6 to 20 carbon atoms such as a phenyl group and a naphthyl group; an aromatic heterocyclic ring group having 2 to 20 carbon atoms such as a furanyl group and a thiophenyl group; a cycloalkyl group having 3 to 8 carbon atoms such as a cyclopropyl group, a cyclopentyl group and a cyclohexyl groupa; a fluoroalkyl group having 1 to 12 carbon atoms in which at least one hydrogen atom is substituted with a fluorine atom such as a trifluoromethyl group, a pentafluoroethyl group, and -CH₂CF₃; and -OCF₃.

Note that, the aromatic hydrocarbon ring group having 5 to 18 carbon atoms may have a plurality of substituents. When the aromatic hydrocarbon ring group having 5 to 18 carbon atoms has a plurality of substituents, the substituents may be the same or different.

Q is preferably a group represented by any of the following formulas (VII-1) to (VII-29), and the groups represented by the following formulas may have the aforementioned substituents.

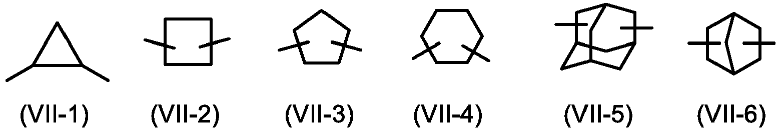

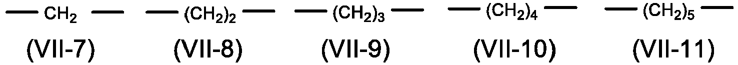

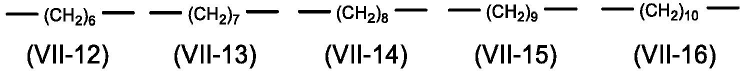

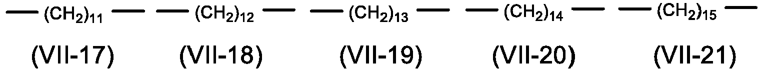

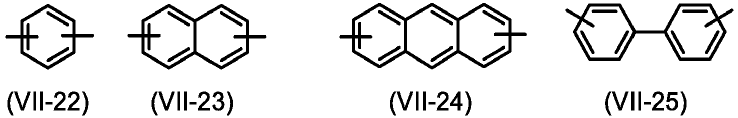

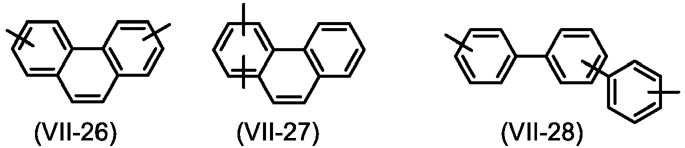

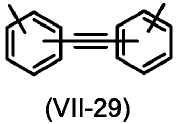

In the aforementioned formula (II), n and m each independently represent an integer from 0 to 3, preferably an integer from 0 to 2, and more preferably 0 or 1. Further, the cases when n and m = 0, n = 0 and m = 1, or n = 1 and m = 1 are particularly preferable.

In the aforementioned formula (II), Rⁿ and R^{m} each independently represent -CH₂-CH₂-OR^{b}, -CH₂-OR^{b}, -CH₂-CH₂-OH, -CH₂-OH, -OR^{b}, -COOR^{b}, -NHR²⁰, -SH, a hydroxyl group, or a carboxyl group.

Here, R²⁰ represents a hydrogen atom or an alkyl group having 1 to 6 carbon atoms. Thereamong, a hydrogen atom is preferable.

Further, R^{b} represents a protecting group.

Here, the protecting group of R^{b} is not specifically limited, and examples of the protecting group of a hydroxyl group or a carboxyl group include a tetrahydropyranyl group, a methoxymethyl group a 2-methoxyethoxymethyl group, a tert-butyldimethylsilyl group, a trimethylsilyl group, a benzyl group and the like. Among these, a tetrahydropyranyl group, a 2-methoxyethoxymethyl group, and a tert-butyldimethylsilyl group are preferable.

Rⁿ and R^{m} each independently are preferably -CH₂-CH₂-OR^{b}, -CH₂-OR^{b}, -CH₂-CH₂-OH, -CH₂-OH, -OR^{b}, -COOR^{b}, a hydroxyl group, or a carboxyl group. Thereamong, CH₂-CH₂-OR^{b}, -CH₂-OR^{b}, -CH₂-CH₂-OH, -CH₂-OH, -OR^{b} or a hydroxyl group are more preferable, and -OR^{b} or a hydroxyl group are particularly preferable.

Furthermore, when Rⁿ or R^{m} are -CH₂-CH₂-OR^{b}, -CH₂-OR^{b}, -OR^{b}, or COOR^{b}, at least one of Rⁿ and R^{m} is -CH₂-CH₂-OH, -CH₂-OH, -NHR²⁰, -SH, a hydroxyl group, or a carboxyl group.

### «Polymerizable compound represented by formula (III)»

The polymerizable compound represented by formula (III) will be described below. Here, the polymerizable compound (III) is formed by reacting the compound (II) with one type of the compound (I) or two types of the compound (I) having different structures.

In the polymerizable compound represented by formula (III), the "P¹-L¹-Y²... ·A¹-(*)" and the "(*)-A²-...Y⁶-L²-P²" may be symmetric structures with (*) as the center of symmetry, or may not be symmetric structures.

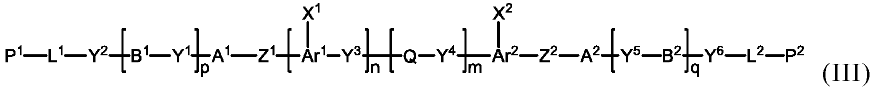

In the aforementioned formula (III), A¹ and B¹ each independently are the same as defined above, and the preferred examples are the same, A² and B² each independently are the same as defined above for A¹ and B¹, and the preferred examples are the same, Ar¹ and Ar² each independently are the same as defined above, and the preferred examples are the same, and X¹ and X² each independently are the same as defined above the, and the preferred examples are the same.

In the aforementioned formula (III), Z¹ and Z² are groups which are formed by reacting the -Yx-C(=O)-G of the compound (I) with Rn or Rm of the compound (II), and each independently represent -C(=O)-O-, -O-C(=O)-, -C(=O)-S-, -S-C(=O)-, -NR²⁰-C(=O)-, -C(=O)-NR²⁰-, -CH=CH-C(=O)-O-, -O-C(=O)-CH=CH-, -CH₂-C(=O)-O-, -O-C(=O)-CH₂-, -CH₂-O-C(=O)-, -C(=O)-O-CH₂-, -CH₂-CH₂-C(=O)-O-, -O-C(=O)-CH₂-CH₂-, -CH₂-CH₂-O-C(=O)-, -C(=O)-O-CH₂-CH₂-, or -C(=O)-O-C(=O)-, where R²⁰ represents a hydrogen atom or an alkyl group having 1 to 6 carbon atoms.

Thereamong, Z¹ and Z² each independently are preferably -C(=O)-O-, -O-C(=O)-, -CH₂-C(=O)-O-, -O-C(=O)-CH₂-, -CH₂-O-C(=O)-, -C(=O)-O-CH₂-, -CH₂-CH₂-C(=O)-O-, -O-C(=O)-CH₂-CH₂-, -CH₂-CH₂-O-C(=O)-, or -C(=O)-O-CH₂-CH₂-, and particularly preferably, -C(=O)-O- or -O-C(=O)-.

In the aforementioned formula (III), Y¹ to Y⁴ each independently are the same as defined above, and the preferred examples are the same, and Y⁵ and Y⁶ each independently are the same as defined above for Y¹ and Y², and the preferred examples are the same.

In the aforementioned formula (III), L¹ is the same as defined above, and the preferred examples are the same, and L² is the same as defined above for L¹, and the preferred examples are the same.

In the aforementioned formula (III), Q is the same as defined above, and the preferred examples are the same.

In the aforementioned formula (III), P¹ is the same as defined above, and the preferred examples are the same, and P² is the same as defined above for P¹, and the preferred examples are the same. At least one of P¹ and P² represents a polymerizable group.

In the aforementioned formula (III), p, n and m each independently are the same as defined above, and the preferred examples are the same, and q is the same as defined above for p, and the preferred examples are the same.

In the aforementioned formula (III), the Ar¹-X¹ and Ar²-X² each independently represent any of the following formulas (VIII-1) to (VIII-7):

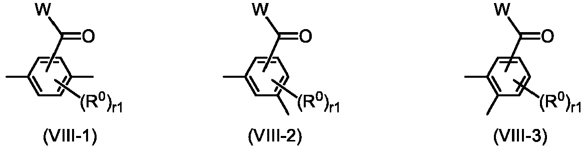

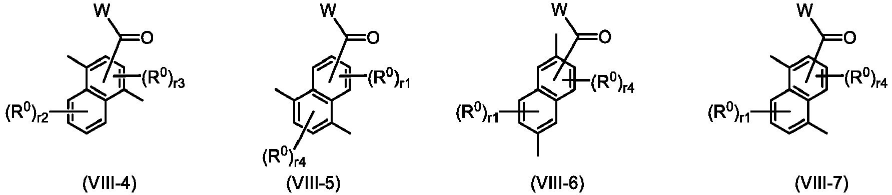

In the aforementioned formulas (VIII-1) to (VIII-7), W represents a hydrogen atom or an alkyl group having 1 to 6 carbon atoms. Here, examples of the alkyl group having 1 to 6 carbon atoms of W include a methyl group, an ethyl group, an n-propyl group, an isopropyl group, a butyl group, a sec-butyl group, a tert-butyl group and the like.

In the aforementioned formulas (VIII-1) to (VIII-7), R⁰ is the same as defined above, and the preferred examples are the same.

When there is a plurality of R⁰, each R⁰ may be the same or may be different.

In the aforementioned formulas (VIII-1) to (VIII-7), r1 is an integer from 0 to 3, preferably an integer from 0 to 2, more preferably 0 or 1, and particularly preferably 0.

In the aforementioned formulas (VIII-1) to (VIII-7), r2 is an integer from 0 to 4, preferably an integer from 0 to 3, more preferably an integer from 0 to 2, particularly preferably 0 or 1, and most preferably 0.

In the aforementioned formulas (VIII-1) to (VIII-7), r3 is 0 or 1, and is preferably 0.

In the aforementioned formulas (VIII-1) to (VIII-7), r4 is an integer from 0 to 2, preferably 0 or 1, and more preferably 0.

The polymerizable compound represented by the aforementioned formula (III) is preferably represented by any of the following formulas (III-1) to (III-6):

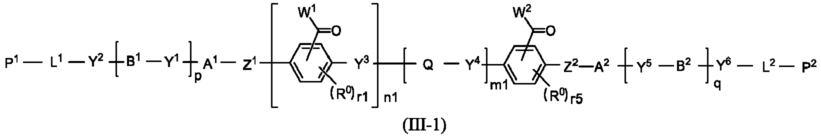

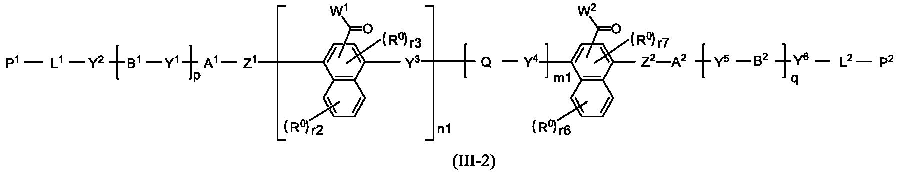

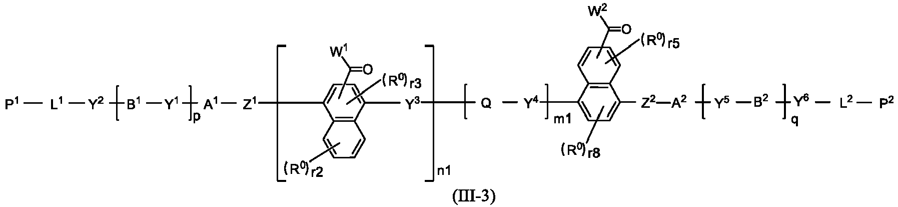

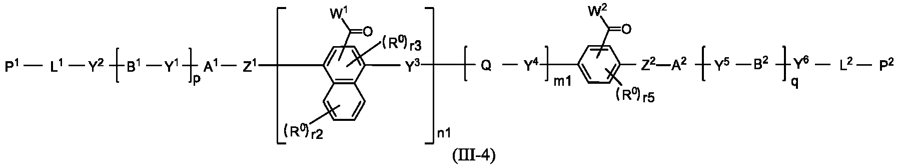

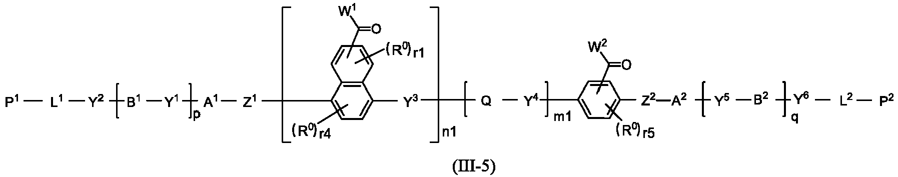

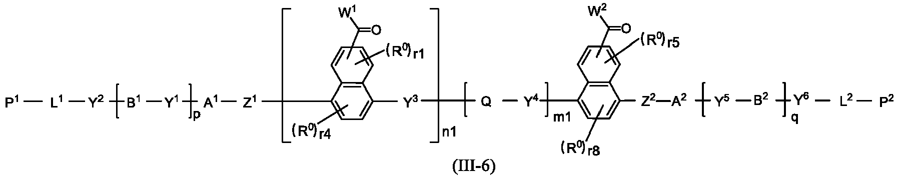

In the aforementioned formulas (III-1) to (III-6), W¹ and W² each independently represent a hydrogen atom or an organic group having 1 to 20 carbon atoms which may have a substituent. Thereamong, W¹ and W² are preferably a hydrogen atom or an alkyl group having 1 to 6 carbon atoms, and a hydrogen atom is more preferable.

In the aforementioned formulas (III-1) to (III-6), n1 and m1 each independently are an integer from 0 to 3, preferably an integer from 0 to 2, and more preferably 0 or 1. Further, any of the cases of n = m = 0, n = 0 and m = 1, or n = 1 and m = 1 are particularly preferable.

In the aforementioned formulas (III-1) to (III-6), R⁰ is the same as defined above, and the preferred examples are the same.

In the aforementioned formulas (III-1) to (III-6), r1 is the same as defined above, and the preferred examples are the same, and r5 is the same as defined above for r1, and the preferred examples are the same.

In the aforementioned formulas (III-1) to (III-6), r2 is the same as defined above, and the preferred examples are the same, and r6 is the same as defined above for r2, and the preferred examples are the same.

In the aforementioned formulas (III-1) to (III-6), r3 is the same as defined above, and the preferred examples are the same, and r7 is the same as defined above for r3, and the preferred examples are the same.

In the aforementioned formulas (III-1) to (III-6), r4 is the same as defined above, and the preferred examples are the same, and r8 is the same as defined above for r4, and the preferred examples are the same.

In the aforementioned formulas (III-1) to (III-6), A¹, A², B¹, B², Y¹ to Y⁶, L¹, L², P¹, P², Z¹, Z², Q, p and q are the same as defined above, and the preferred examples are the same.

### «Base»

The pKa of the base is from 6.1 to 9.5, preferably 6.15 or more, more preferably 6.5 or more, particularly preferably 6.65 or more, and preferably 7.5 or less, and more preferably 6.99 or less.

By the pKa of the base being 6.1 or more, preferably 6.5 or more, the polymerizable compound represented by the aforementioned formula (III) can be produced in high yield, and by the pKa being 9.5 or less, preferably 7.5 or less, the polymerizable compound represented by the aforementioned formula (III) can be produced in high yield.

Here, pKa is the value in water at 25°C, and is the value described in the CRC Handbook of Chemistry and Physics 87th Edition (CRC Press), or if there is no description in the literature, is the value of a simulation described in SciFinder (Chemical Abstracts Service, American Chemical Society).

Further, as the base, a pyridine having at least two alkyl groups having 1 to 6 carbon atoms is preferable, more preferably a pyridine in which at least two hydrogen atoms in the pyridine are substituted with an alkyl group having 1 to 6 carbon atoms (for example, 2,4-lutidine, 2,6-lutidine, 2,4,6-collidine, 3,5-lutidine, 3,4-lutidine, and the like), even more preferably a pyridine in which at least two hydrogen atoms among hydrogen atoms at the 2-position, 4-position and 6-position in the pyridine are substituted with an alkyl group having 1 to 6 carbon atoms, and 2,4-lutidine, 2,6-lutidine, 2,4,6-collidine are particularly preferable.

The base is normally used in an amount of 1 to 3 mol per 1 mol of the compound (I).

### «Organic solvent»

The organic solvent is not specifically limited as long as the solvent is inert to the reactions. Examples of the organic solvent include a chlorine-based solvent such as chloroform and methylene chloride; an amide-based solvent such as N-methylpyrrolidone, N,N-dimethylformamide, N,N-dimethylacetamide, and hexamethylphosphoric triamide; an ether-based solvent such as 1,4-dioxane, cyclopentyl methyl ether, tetrahydrofuran, tetrahydropyran, and 1,3-dioxolane; a sulfur-containing solvent such as dimethyl sulfoxide and sulfolane; a nitrile-based solvent such as acetonitrile; an ester-based solvent such as ethyl acetate and propyl acetate; an aromatic hydrocarbon-based solvent such as benzene, toluene, and xylene; an aliphatic hydrocarbon-based solvent such as n-pentane, n-hexane, and n-octane; an alicyclic hydrocarbon-based solvent such as cyclopentane and cyclohexane; a mixed solvent including two or more solvents among these solvents; and the like.

Thereamong, a polar solvent such as an amide-based solvent and an ether-based solvent is preferable from the viewpoint that the target product can be obtained in high yield.

The amount of organic solvent used is not specifically limited, and may be used in an appropriate amount taking into account the type of compounds to be used, the reaction scale, and the like, but is normally used in an amount of 1 to 50 g per gram of the compound (I).

### <Step 1>

In the aforementioned Step 1, the compound (I) is reacted with the compound (II) in an organic solvent in the presence of a base to obtain a reaction solution including the compound (III).

The amount of the compound (II) and the compound (I) used, in a molar ratio (compound (II):compound (I)), is preferably 1:2 to 1:4, more preferably 1:2 to 1:3, and 1:2 to 1:2.5 is particularly preferable.

Note that, a compound that includes different groups on the right side and the left side can be obtained by effecting a stepwise reaction using two different types of compound (I). For example, 1 mol of a compound (I) is reacted with 1 mol of the compound (II), and then reacted with 1 mol of another compound (I) to obtain a compound that includes different groups on the right side and the left side.

Examples of the reaction method include, (α) a method that adds the compound (I) or an organic solvent solution including the compound (I) to an organic solvent solution including the compound (II) and the base, (β) a method that adds the compound (II) or an organic solvent solution including the compound (II) to an organic solvent solution including the compound (I) and the base, (γ) a method that adds the base to an organic solvent solution including the compound (II) or the compound (I); and the like. Method (α) is preferable since the target product can be obtained in high yield.

The reaction temperature is a temperature within a range from -20°C to the boiling point of the solvent to be used and preferably -15°C to +30°C, and more preferably 0°C to 10°C.

The reaction time is determined taking account of the reaction scale, but is normally set to several minutes to several hours.

The obtained reaction solution is subjected directly to step (2) without washing, extraction, and the like while being maintained at the above temperature.

Note that many of the compound (II) and the compound (I) are known compounds, and may be produced and obtained using a known method (e.g., the methods disclosed in WO2014/010325 and WO2012/147904). The compound (II) may be a commercially-available product and used as is or after purification.

The compound of the compound (I) wherein G is a halogen atom (hal) can be produced by the method described below.

First, the sulfonyl chloride is reacted with trans-1,4-cyclohexanedicarboxylic acid in the presence of a base such as triethylamine and 4-(dimethylamino)pyridine.

Next, the following compound M is obtained by adding the following compound (XII-b) with a base such as triethylamine and 4-(dimethylamino)pyridine in the obtainable reaction mixture and performing the reaction.

The amount of sulfonyl chloride used is normally 0.5 to 1.0 equivalent based on 1 equivalent of trans-1,4-cyclohexanedicarboxylic acid, preferably 0.5 to 0.6 equivalent. Here, equivalent means "molar equivalent".

The amount of compound (XII-b) used is normally 0.5 to 1.0 equivalent based on 1 equivalent of trans-1,4-cyclohexanedicarboxylic acid.

The amount of base used is normally 1.0 to 2.5 equivalents based on 1 equivalent of trans-1,4-cyclohexanedicarboxylic acid 1, preferably 1.0 to 1.4 equivalents.

The reaction temperature is set to 20°C to 30°C, and the reaction time is determined taking account of the reaction scale and the like, but is several minutes to several hours.

A halogenating agent such as thionyl chloride, thionyl bromide, or sulfuryl chloride is then acted on the obtainable compound M to obtain compound (XII-b-x) (example of a compound represented by formula (I)).

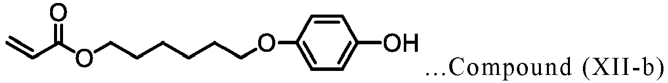

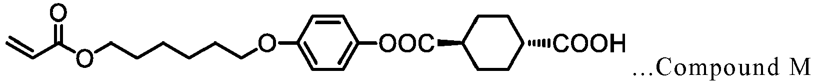

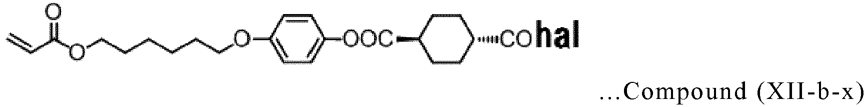

Examples of the solvent used for the reaction for obtaining the aforementioned compound M include the solvent which can be used when producing the compound (III). Thereamong, an ether-based solvent is preferable.

Further, examples of a solvent used for the reaction for obtaining the compound represented by the formula (XII-b-x) include an amide-based solvent such as N,N-dimethylformamide and N,N-dimethylacetamide; an aromatic hydrocarbon-based solvent such as benzene and toluene; a mixed solvent including two or more solvents among these solvents; and the like.

The amount of organic solvent used is not specifically limited, and may be used in an appropriate amount taking into account the type of compounds to be used, the reaction scale, and the like, but is normally used in an amount of 1 to 50 g per gram of trans-1,4-cyclohexanedicarboxylic acid.

### ((1-2) Method of Producing the Polymerizable Compound (Method of producing the second compound))

The method of producing the compound of the present disclosure (method of producing the second compound) includes the aforementioned Step 1 and the Step 2 for reacting and the compound (III) obtained in the Step 1 with the compound represented by the following (V) (hereinafter, referred to as "compound (V)") to obtain a polymerizable compound represented by the following formula (VI) (hereinafter, referred to as "compound (VI)").

**D-NH₂** (V)

In the aforementioned formula (V), D is represented by the following formula (V-I) or (V-II):

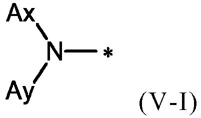

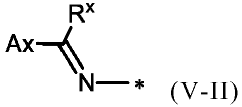

In the aforementioned formulas (V-I) and formula (V-II), * represents an amimo group.

In the aforementioned formulas (V-I) and (V-II), Ax represents an organic group having at least one aromatic ring selected from the group consisting of an aromatic hydrocarbon ring having 6 to 30 carbon atoms and an aromatic heterocyclic ring having 2 to 30 carbon atoms, the aromatic ring included in Ax may have a substituent, and Ay and R^{x} each independently represent a hydrogen atom or an organic group having 1 to 30 carbon atoms which may have a substituent.

The organic group including Ax having at least one aromatic ring selected from the group consisting of an aromatic hydrocarbon ring having 6 to 30 carbon atoms and an aromatic heterocyclic ring having 2 to 30 carbon atoms may have a plurality of aromatic rings, and may also have an aromatic hydrocarbon ring and an aromatic heterocyclic ring. Further, when there is a plurality of aromatic hydrocarbon rings and aromatic heterocyclic rings, each may be the same or different.

Note that, examples of the aromatic hydrocarbon ring of Ax includes a benzene ring, a naphthalene ring, an anthracene ring, a phenanthrene ring, a pyrene ring, a fluorene ring and the like.

Thereamong, a benzene ring, a naphthalene ring, or an anthracene ring are preferable as the aromatic hydrocarbon ring.

Further, examples of the aromatic heterocyclic ring of Ax include a 1H-isoindole-1,3 (2H)-dione ring, a 1-benzofuran ring, a 2-benzofuran ring, an acridine ring, an isoquinoline ring, an imidazole ring, an indole ring, an oxadiazole ring, an oxazole ring, an oxazolopyrazine ring, an oxazolopyridine ring, an oxazolopyridazyl ring, an oxazolopyrimidine ring, a quinazoline ring, a quinoxaline ring, a quinoline ring, a cinnoline ring, a thiadiazole ring, a thiazole ring, a thiazolopyrazine ring, a thiazolopyridine ring, a thiazolopyridazine ring, a thiazolopyrimidine ring, a thiophene ring, a triazine ring, a triazole ring, a naphthyridine ring, a pyrazine ring, a pyrazole ring, a pyranone ring, a pyran ring, a pyridine ring, a pyridazine ring, a pyrimidine ring, a pyrrole ring, a phenanthridine ring, a phthalazine ring, a furan ring, a benzo[c]thiophene ring, a benzisoxazole ring, a benzisothiazole ring, a benzimidazole ring, a benzoxadiazole ring, a benzoxazole ring, a benzothiadiazole ring, a benzothiazole ring, a benzothiophene ring, a benzotriazine ring, a benzotriazole ring, a benzopyrazole ring, a benzopyranone ring and the like.

Thereamong, a monocyclic aromatic heterocyclic ring such as a pyrrole ring, a furan ring, an oxazole ring, an oxadiazoyl ring, a thiazole ring, and a thiadiazole ring; a fused aromatic heterocyclic ring such as a benzothiazole ring, a benzoxazole ring, a quinoline ring, a 1-benzofuran ring, a 2-benzofuran ring, a benzothiophene ring, a 1H-isoindole-1,3 (2H)-dione ring, a benzo[c]thiophene ring, a thiazolopyridine ring, a thiazolopyrazine ring, a benzoisoxazole ring, a benzoxadiazole ring, a benzothiadiazole ring and the like are preferable as the aromatic heterocyclic ring.

The aromatic ring of Ax may have a substituent. Examples of such a substituent include a halogen atom such as a fluorine atom, and a chlorine atom; a cyano group; an alkyl group having 1 to 6 carbon atoms such as a methyl group, an ethyl group, and a propyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group; an N,N-dialkylamino group having 2 to 12 carbon atoms such as a dimethylamino group; an alkoxy group having 1 to 6 carbon atoms such as a methoxy group, an ethoxy group and an isopropoxy group; a nitro group; an aromatic hydrocarbon ring group having 6 to 20 carbon atoms such as a phenyl group and a naphthyl group; -OCF₃, -C(=O)-R^{b1}, -O-C(=O)-R^{b1}, -C(=O)-O-R^{b1}; and -SO₂R^{a1}; and the like. Here, R^{b1} and R^{a1} are the same as defined above, and the preferred examples thereof are the same.

Thereamong, the substituent of the aromatic ring of Ax is preferably a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, -OCF₃, -O-C(=O)-R^{b1}, or -C(=O)-O-R^{b1}, and more preferably a halogen atom, a cyano group, an alkyl group having 1 to 6 carbon atoms, and, an alkoxy group having 1 to 6 carbon atoms.

Note that, Ax may have a plurality of substituents selected from the aforementioned substituents. When Ax has a plurality of substituents, the substituents may be the same or different.

Here, the aromatic ring of Ax may have a plurality of substituents which are the same or different, and two adjacent substituents may be bonded to each other to form a ring. The ring formed by two adjacent substituents may be either a monocyclic ring or a fused polycyclic ring, and may be either an unsaturated ring or a saturated ring.

Note that, the "number of carbon atoms" of the organic group of Ax having at least one aromatic ring selected from the group consisting of an aromatic hydrocarbon ring having 6 to 30 carbon atoms and an aromatic heterocyclic ring having 2 to 30 carbon atoms means the number of carbon atoms of the aromatic hydrocarbon ring and the aromatic heterocyclic ring not including the number of carbon atoms of the substituents.

Moreover, the organic group of Ax having at least one aromatic ring selected from the group consisting of an aromatic hydrocarbon ring having 6 to 30 carbon atoms and an aromatic heterocyclic ring having 2 to 30 carbon atoms includes the following 1) to 5):
1) a hydrocarbon ring group having 6 to 40 carbon atoms having at least one aromatic hydrocarbon ring having 6 to 30 carbon atoms,
2) a heterocyclic ring group having 2 to 40 carbon atoms having at least one aromatic ring selected from the group consisting of an aromatic hydrocarbon ring having 6 to 30 carbon atoms and an aromatic heterocyclic ring having 2 to 30 carbon atoms,
3) an alkyl group having 1 to 12 carbon atoms substituted by at least one of an aromatic hydrocarbon ring group having 6 to 30 carbon atoms and an aromatic heterocyclic ring group having 2 to 30 carbon atoms,
4) an alkenyl group having 2 to 12 carbon atoms substituted by at least one of an aromatic hydrocarbon ring group having 6 to 30 carbon atoms and an aromatic heterocyclic ring group having 2 to 30 carbon atoms, and
5) an alkynyl group having 2 to 12 carbon atoms substituted by at least one of an aromatic hydrocarbon ring group having 6 to 30 carbon atoms and an aromatic heterocyclic ring group having 2 to 30 carbon atoms.

The specific examples of the aromatic hydrocarbon ring in the aforementioned 1) "a hydrocarbon ring group having 6 to 40 carbon atoms having at least one aromatic hydrocarbon ring having 6 to 30 carbon atoms" may include the same examples as those listed as the specific examples of the aromatic hydrocarbon ring of Ax. Moreover, examples of the hydrocarbon ring group of the aforementioned 1) include an aromatic hydrocarbon ring group having 6 to 30 carbon atoms (a phenyl group and an anthracenyl group, a phenanthrenyl group, a pyrenyl group, and a fluorenyl group), an indanyl group, a 1,2,3,4-tetrahydronaphthyl group, and, a 1,4-dihydronaphthyl group.

The specific examples of the aromatic hydrocarbon ring and the aromatic heterocyclic ring in the aforementioned 2) "a heterocyclic ring group having 2 to 40 carbon atoms having at least one aromatic ring selected from the group consisting of an aromatic hydrocarbon ring having 6 to 30 carbon atoms and an aromatic heterocyclic ring having 2 to 30 carbon atoms" may include the same examples as those listed as the specific examples of an aromatic hydrocarbon ring and an aromatic heterocyclic ring of Ax. Moreover, examples of the heterocyclic ring group of the aforementioned 2) include an aromatic heterocyclic ring group having 2 to 30 carbon atoms (a phthalimido group, a 1-benzofuranyl group, a 2-benzofuranyl group, an acridinyl group, an isoquinoryl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridinyl group, a thiazolopyridazinyl group, a thiazolopyrimidinyl group, a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxazolyl group, a benzothiadiazolyl group, benzothiazolyl group, a benzothiophenyl group, a benzotriazinyl group, a benzotriazolyl group, a benzopyrazolyl group, a benzopyranonyl group, a dihydropyranyl group, a tetrahydropyranyl group, a dihydrofuranyl group, a tetrahydrofuranyl group and the like), a 2,3-dihydroindolyl group, a 9,10-dihydroacridinyl group, a 1,2,3,4-tetrahydroquinolyl group, a dihydropyranyl group, a tetrahydropyranyl group, a dihydrofuranyl group and a tetrahydrofuranyl group.

The specific examples of the alkyl group having 1 to 12 carbon atoms in the aforementioned 3) "an alkyl group having 1 to 12 carbon atoms substituted by at least one of an aromatic hydrocarbon ring group having 6 to 30 carbon atoms and an aromatic heterocyclic ring group having 2 to 30 carbon atoms" include a methyl group, an ethyl group, a propyl group, an isopropyl group and the like. Moreover, the specific examples of the aromatic hydrocarbon ring group having 6 to 30 carbon atoms and the aromatic heterocyclic ring group having 2 to 30 carbon atoms in the aforementioned 3) may include the same examples as those listed as the specific examples of the aromatic hydrocarbon ring group having 6 to 30 carbon atoms and the aromatic heterocyclic ring group having 2 to 30 carbon atoms in the aforementioned 1) and 2).

The specific examples of the alkenyl group having 2 to 12 carbon atoms in the aforementioned 4) "an alkenyl group having 2 to 12 carbon atoms substituted by at least one of an aromatic hydrocarbon ring group having 6 to 30 carbon atoms and an aromatic heterocyclic ring group having 2 to 30 carbon atoms" include a vinyl group, an allyl group and the like. Moreover, the specific examples of the aromatic hydrocarbon ring group having 6 to 30 carbon atoms and the aromatic heterocyclic ring group having 2 to 30 carbon atoms in the aforementioned 4) may include the same examples as those listed as the specific examples of the aromatic hydrocarbon ring group having 6 to 30 carbon atoms and the aromatic heterocyclic ring group having 2 to 30 carbon atoms in the aforementioned 1) and 2).

The specific examples of the alkynyl group having 2 to 12 carbon atoms in the aforementioned 5) "an alkynyl group having 2 to 12 carbon atoms substituted by at least one of an aromatic hydrocarbon ring group having 6 to 30 carbon atoms and an aromatic heterocyclic ring group having 2 to 30 carbon atoms" include an ethynyl group, a propynyl group and the like. Moreover, the specific examples of the aromatic hydrocarbon ring group having 6 to 30 carbon atoms and the aromatic heterocyclic ring group having 2 to 30 carbon atoms in the aforementioned 5) may include the same examples as those listed as the specific examples of the aromatic hydrocarbon ring group having 6 to 30 carbon atoms and the aromatic heterocyclic ring group having 2 to 30 carbon atoms in the aforementioned 1) and 2).

Note that, the organic groups listed in the aforementioned 1) to 5) may have one or more substituents. When there is a plurality of substituents, the plurality of substituents may be the same or different.

Examples of such a substituent include a halogen atom such as a fluorine atom, and a chlorine atom; a cyano group; an alkyl group having 1 to 6 carbon atoms such as a methyl group, an ethyl group, and a propyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group; an N,N-dialkylamino group having 2 to 12 carbon atoms such as a dimethylamino group; an alkoxy group having 1 to 6 carbon atoms such as a methoxy group, an ethoxy group and an isopropoxy group; a nitro group; an aromatic hydrocarbon ring group having 6 to 20 carbon atoms such as a phenyl group and a naphthyl group; -OCF₃; -C(=O)-R^{b1}, -O-C(=O)-R^{b1}; -C(=O)-O-R^{b1}; and -SO₂R^{a1}; and the like. Here, R^{b1} and R^{a1} are the same as defined above, and the preferred examples thereof are the same.

Thereamong, the substituents having the organic groups listed in the aforementioned 1) to 5) are preferably a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, -OCF₃, -O-C(=O)-R^{b1}, or -C(=O)-O-R^{b1}, and more preferably a halogen atom, a cyano group, an alkyl group having 1 to 6 carbon atoms and an alkoxy group having 1 to 6 carbon atoms.

Preferred specific examples of the organic group having at least one aromatic ring selected from the group consisting of an aromatic hydrocarbon ring having 6 to 30 carbon atoms and an aromatic heterocyclic ring having 2 to 30 carbon atoms represented as Ax are given below. However, the present disclosure is not limited to the following examples. Note that, in the following formulas, "-" represents a bond with an N atom extending from any position of the ring (that is, the N atom bonding with Ax in formula (V-I)).
1) The specific examples of the hydrocarbon ring group having 6 to 40 carbon atoms having at least one aromatic hydrocarbon ring having 6 to 30 carbon atoms include the structures represented by the following formulas (1-1) to (1-21), and aromatic hydrocarbons ring having 6 to 30 carbon atoms represented by formulas (1-9) to (1-21) are preferable. 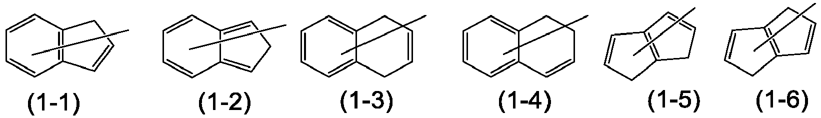 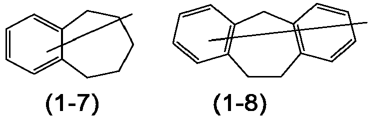 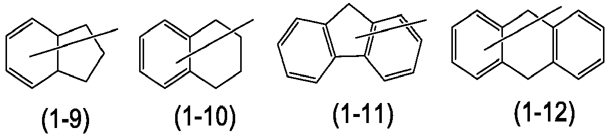 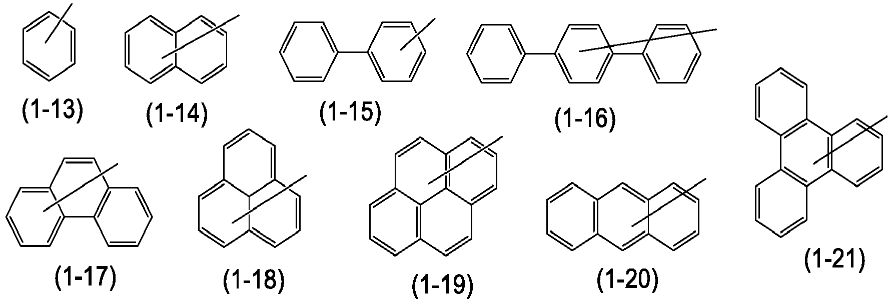
2) The specific examples of the heterocyclic ring group having 2 to 40 carbon atoms having at least one aromatic ring selected from the group consisting of an aromatic hydrocarbon ring having 6 to 30 carbon atoms and an aromatic heterocyclic ring having 2 to 30 carbon atoms include the structures represented by the following formulas (2-1) to (2-51), and aromatic heterocyclic ring groups having 2 to 30 carbon atoms represented by formulas (2-12) to (2-51) are preferable. 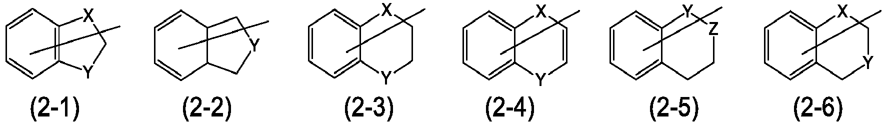 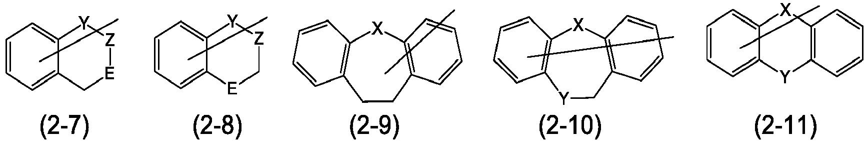 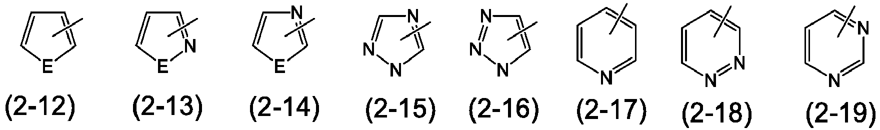 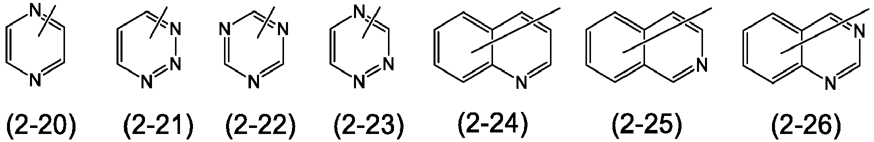 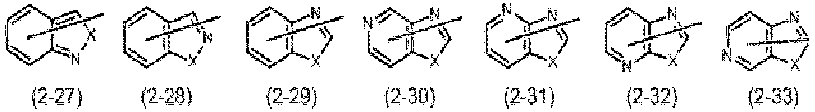 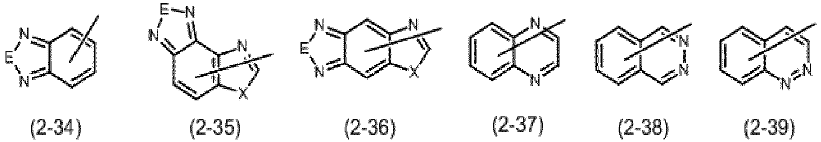 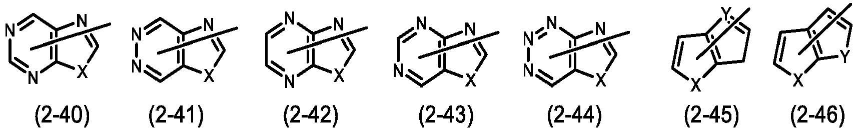 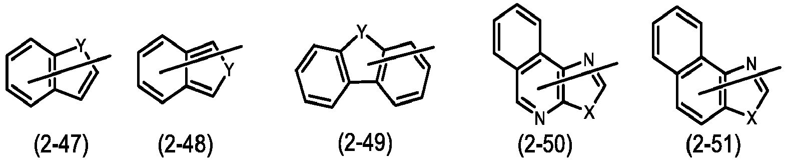
   where X represents -CH₂-, -NR^{c}-, an oxygen atom, a sulfur atom, -SO- or -SO₂-,
   Y and Z each independently represent -NR^{c}-, an oxygen atom, a sulfur atom, -SO- or -SO₂-, and
   E represents -NR^{c}-, an oxygen atom or a sulfur atom, where R^{c} represents a hydrogen atom, or an alkyl group having 1 to 6 carbon atoms such as a methyl group, an ethyl group and a propyl group (with the proviso that the oxygen atom, the sulfur atom, -SO-, or -SO₂- are not adjacent to each other in each of the formulas.
3) The specific examples of the alkyl group having 1 to 12 carbon atoms substituted with at least one of an aromatic hydrocarbon ring group having 6 to 30 carbon atoms and an aromatic heterocyclic ring group having 2 to 30 carbon atoms include the structures represented by the following formulas (3-1) to (3-8). 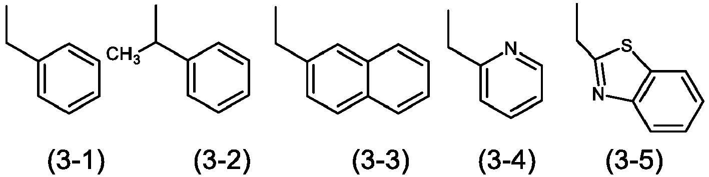 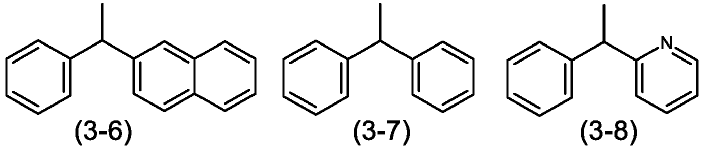
4) The specific examples of an alkenyl group having 2 to 12 carbon atoms substituted with at least one of an aromatic hydrocarbon ring group having 6 to 30 carbon atoms and an aromatic heterocyclic ring group having 2 to 30 carbon atoms include the structures represented by the following formulas (4-1) to (4-5). 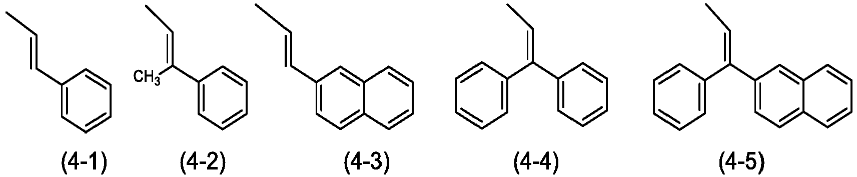
5) The specific examples of an alkynyl group having 2 to 12 carbon atoms substituted with at least one selected from the group consisting of an aromatic hydrocarbon ring and an aromatic heterocyclic ring include the structures represented by the following formulas (5-1) and (5-2).

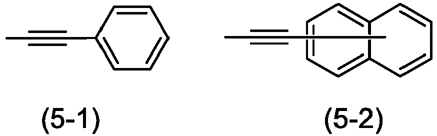

Note that, the ring of the preferred specific examples of the aforementioned Ax may have one or more substituents. Moreover, when there is a plurality of substituents, the plurality of substituents may be the same or different. Examples of such a substituent include a halogen atom such as a fluorine atom, and a chlorine atom; a cyano group; an alkyl group having 1 to 6 carbon atoms such as a methyl group, an ethyl group, and a propyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group; a N,N-dialkylamino group having 1 to 12 carbon atoms such as a dimethylamino group; an alkoxy group having 1 to 6 carbon atoms such as a methoxy group, an ethoxy group and an isopropoxy group; a nitro group; an aromatic hydrocarbon ring group having 6 to 20 carbon atoms such as a phenyl group and a naphthyl group; -OCF₃; -C(=O)-R^{b1}; -O-C(=O)-R^{b1}; -C(=O)-O-R^{b1}; and -SO₂R^{a1}; and the like. Here, R^{b1} and R^{a1} are the same as defined above, and the preferred examples thereof are the same.

Thereamong, the substituent having the aforementioned ring of Ax is preferably a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, -OCF₃, -O-C(=O)-R^{b1}, or -C(=O)-O-R^{b1}, and more preferably at least one substituent selected from a halogen atom, a cyano group, an alkyl group having 1 to 6 carbon atoms, and an alkoxy group having 1 to 6 carbon atoms.

Among these described above, Ax is preferably an aromatic hydrocarbon ring group having 6 to 30 carbon atoms, an aromatic heterocyclic ring group having 2 to 30 carbon atoms, or a group represented by the formula (1-9).

Ax is more preferably an aromatic hydrocarbon ring group having 6 to 20 carbon atoms or an aromatic heterocyclic ring group having 4 to 20 carbon atoms, and even more preferably a group represented by any of the aforementioned formulas (1-14), (1-20), (2-27) to (2-33), (2-35) to (2-43), and (2-50) to (2-51).

Note that, as described above, the aforementioned ring may have one or a plurality of substituents. When there is a plurality of substituents, the plurality of substituents may be the same or different. Examples of such a substituent include a halogen atom such as a fluorine atom, and a chlorine atom; a cyano group; an alkyl group having 1 to 6 carbon atoms such as a methyl group, an ethyl group, and a propyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group and a pentafluoroethyl group; an N,N-dialkylamino group having 1 to 12 carbon atoms such as a dimethylamino group; an alkoxy group having 1 to 6 carbon atoms such as a methoxy group, an ethoxy group and an isopropoxy group; a nitro group; an aromatic hydrocarbon ring group having 6 to 20 carbon atoms such as a phenyl group and a naphthyl group; -C(=O)-R^{b1}; -O-C(=O)-R^{b1}; -C(=O)-O-R^{b1}; -SO₂R^{a1}; and the like.

Here, R^{b1} and R^{a1} are the same as defined above, and the preferred examples thereof are the same.

Thereamong, the substituent having the aforementioned ring is preferably a halogen atom, a cyano group, an alkyl group having 1 to 6 carbon atoms, or an alkoxy group having 1 to 6 carbon atoms.

Moreover, a group represented by the following formula (XI) is even more preferable as Ax.

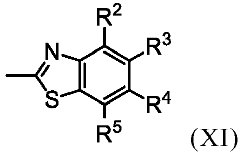

Here, in formula (XI), R² to R⁵ each independently represent a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, -OCF₃, -O-C(=O)-R^{b1}, or -C(=O)-O-R^{b1}, and R^{b1} represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring group having 5 to 12 carbon atoms which may have a substituent. Thereamong, all of R² to R⁵ are hydrogen atoms, at least one among R² to R⁵ is an alkoxy group having 1 to 6 carbon atoms which may have a substituent, and the remainder are preferably hydrogen atoms.

Moreover, C-R² to C-R⁵ may be the same or different, and one or more ring constituent C-R² to C-R⁵ may be replaced by a nitrogen atom.

Specific examples of groups represented by the aforementioned formula (XI) in which at least one among C-R² to C-R⁵ is replaced by a nitrogen atom are shown below. It is to be noted that groups represented by the aforementioned formula (XI) in which at least one among C-R² to C-R⁵ is replaced by a nitrogen atom are not limited thereto. 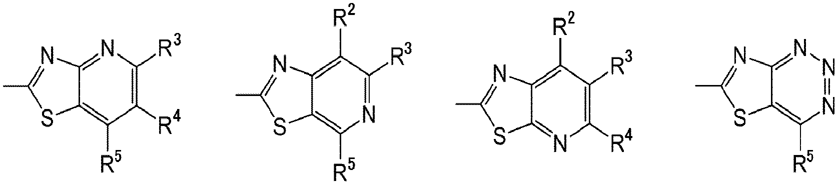 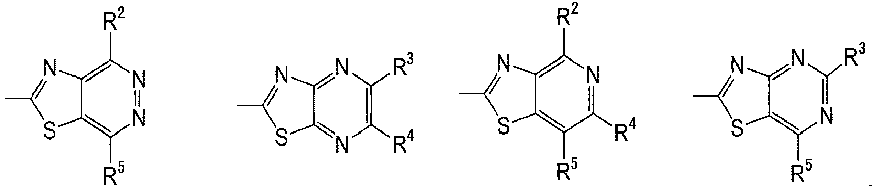 where R² to R⁵ are the same as defined above, and the preferred examples thereof are the same.

Further, the organic group having 1 to 30 carbon atoms which may have a substituent of Ay and R^{x} in the aforementioned formula (V-I) and formula (V-II) is not specifically limited, and examples thereof include an alkyl group having 1 to 20 carbon atoms which may have a substituent, an alkenyl group having 2 to 20 carbon atoms which may have a substituent, an alkynyl group having 2 to 20 carbon atoms which may have a substituent, a cycloalkyl group having 3 to 12 carbon atoms which may have a substituent, -SO₂R^{a1}, -C(=O)-R^{b1}, -CS-NH-R^{b1}, an aromatic hydrocarbon ring group having 3 to 30 carbon atoms which may have a substituent, or an aromatic heterocyclic ring group having 2 to 30 carbon atoms which may have a substituent.

Here, R^{a1} and R^{b1} are the same as defined above, and the preferred examples thereof are the same.

Note that, the examples of the alkenyl group having 2 to 20 carbon atoms of Ay and R^{x} in the case of an alkyl group having 1 to 20 carbon atoms which may have a substituent, the cycloalkyl group having 3 to 12 carbon atoms in the case of a cycloalkyl group having 3 to 12 carbon atoms which may have a substituent may include the same examples as those listed as the specific examples of the alkyl group having 1 to 20 carbon atoms in the case of an alkyl group having 1 to 20 carbon atoms which may have a substituent, the alkenyl group having 2 to 20 carbon atoms in the case of an alkenyl group having 2 to 20 carbon atoms which may have a substituent, and the cycloalkyl group having 3 to 12 carbon atoms in the case of a cycloalkyl group having 3 to 12 carbon atoms which may have a substituent of the aforementioned R^{b1}. Furthermore, the number of carbon atoms of an alkyl group having 1 to 20 carbon atoms which may have a substituent is preferably 1 to 10, the number of carbon atoms of an alkenyl group having 2 to 20 carbon atoms which may have a substituent is preferably 2 to 10, and the number of carbon atoms of a cycloalkyl group having 3 to 12 carbon atoms which may have a substituent is preferably 3 to 10.

Furthermore, the examples of the alkynyl group having 2 to 20 carbon atoms which may have a substituent of Ay and R^{x} in the case of an alkynyl group having 2 to 20 carbon atoms which may have a substituent include an ethynyl group, a propynyl group, a 2-propynyl group (propargyl group), a butynyl group, a 2-butynyl group, a 3-butynyl group, a pentinyl group, a 2-pentinyl group, a hexynyl group, a 5-hexynyl group, a heptinyl group, an octinyl group, a 2-octynyl group, a nonynyl group, a decynyl group, a 7-decynyl group and the like.

Moreover, the substituent in the case of an alkyl group having 1 to 20 carbon atoms which may have a substituent of Ay and R^{x} in the case of the alkenyl group having 2 to 20 carbon atoms which may have a substituent, in the case of the cycloalkyl group having 3 to 12 carbon atoms which may have a substituent, and in the case of the alkynyl group having 2 to 20 carbon atoms which may have a substituent includes a halogen atom such as a fluorine atom, and a chlorine atom, a cyano group; an N,N-dialkylamino group having 2 to 12 carbon atoms such as a dimethylamino group; an alkoxy group having 1 to 20 carbon atoms such as a methoxy group, an ethoxy group, an isopropoxy group; an alkoxy group having 1 to 12 carbon atoms substituted with an alkoxy group having 1 to 12 carbon atoms such as a methoxymethoxy group and a methoxyethoxy group; a nitro group; an aromatic hydrocarbon ring group having 6 to 20 carbon atoms such as a phenyl group and a naphthyl group; an aromatic heterocyclic ring group having 2 to 20 carbon atoms such as a triazolyl group, a pyrrolyl group, a furanyl group and a thiophenyl group; a cycloalkyl group having 3 to 8 carbon atoms such as a cyclopropyl group, a cyclopentyl group and a cyclohexyl group; a cycloalkyloxy group having 3 to 8 carbon atoms such as a cyclopentyloxy group and a cyclohexyloxy group; a cyclic ether group having 2 to 12 carbon atoms such as a tetrahydrofuranyl group, a tetrahydropyranyl group, a dioxolanyl group and a dioxanyl group; an aryloxy group having 6 to 14 carbon atoms such as a phenoxy group and a naphthoxy group; a fluoroalkyl group having 1 to 12 carbon atoms in which at least one hydrogen atom is substituted with a fluorine atom such as a trifluoromethyl group, a pentafluoroethyl group, and -CH₂CF₃; a benzofuryl group; a benzopyranyl group; a benzodioxolyl group; a benzodioxanyl group; an alkoxy group having 1 to 12 carbon atoms; a hydroxyl group substituted with -O-C(=O)-R^{b1}; -C(=O)-R^{b1}; -O-C(=O)-R^{b1}; C(=O)-O-R^{b1}; -SO₂R^{a1}; -SR^{b1}; -SR^{b1}; and the like. Here, R^{a1} and R^{b1} are the same as defined above, and the preferred examples thereof are the same.

Note that, when Ay and R^{x} are alkyl groups having 1 to 20 carbon atoms, are alkenyl groups having 2 to 20 carbon atoms, are cycloalkyl groups having 3 to 12 carbon atoms, or are alkynyl groups having 2 to 20 carbon atoms, they may have a plurality of substituents as described above, and when there is a plurality of substituents, the plurality of substituents may be the same or different.

Further, examples of the substituents, when Ay and R^{x} are aromatic hydrocarbon ring groups having 6 to 30 carbon atoms or are aromatic heterocyclic ring groups having 2 to 30 carbon atoms may include the same examples as those listed as the respective aromatic hydrocarbon ring group and an aromatic heterocyclic ring group of Ax, and, the substituents thereof. When Ay and R^{x} are aromatic hydrocarbon ring groups having 6 to 30 carbon atoms or aromatic heterocyclic ring groups having 2 to 30 carbon atoms, they may have a plurality of substituents selected from those listed above. When Ay and R^{x} are aromatic hydrocarbon ring groups or are aromatic heterocyclic ring groups, when there is a plurality of substituents thereof, the plurality of substituents may be the same or different. Furthermore, the number of carbon atoms of the aforementioned aromatic hydrocarbon ring groups of Ay and R^{x} is preferably 6 to 20, more preferably 6 to 18, and even more preferably 6 to 12. Further, the number of carbon atoms of the aforementioned aromatic heterocyclic ring group of Ay and R^{x} is preferably 2 to 20, and more preferably 2 to 18.

Among these described above, Ay and R^{x} are preferably a hydrogen atom, an alkyl group having 1 to 20 carbon atoms which may have a substituent, an alkenyl group having 2 to 20 carbon atoms which may have a substituent, an alkynyl group having 2 to 20 carbon atoms which may have a substituent, a cycloalkyl group having 3 to 12 carbon atoms which may have a substituent, an aromatic hydrocarbon ring group having 6 to 18 carbon atoms which may have a substituent, or an aromatic heterocyclic ring group having 2 to 18 carbon atoms which may have a substituent. Furthermore, Ay and R^{x} are more preferably a hydrogen atom, an alkyl group having 1 to 18 carbon atoms which may have a substituent, an alkenyl group having 2 to 18 carbon atoms which may have a substituent, an alkynyl group having 2 to 18 carbon atoms which may have a substituent, a cycloalkyl group having 3 to 10 carbon atoms which may have a substituent, an aromatic hydrocarbon ring group having 6 to 12 carbon atoms which may have a substituent, or an aromatic heterocyclic ring group having 2 to 18 carbon atoms which may have a substituent. Thereamong, an alkyl group having 1 to 18 carbon atoms which may have a substituent is particularly preferable as Ay and R^{x}, and thereamong, an alkyl group having 2 to 12 carbon atoms which may have a substituent is even more particularly preferable.

The polymerizable compound represented by formula (VI) will be described below. Here, the compound (VI) is formed by reacting the compound (III) with one of compound (V) or two of compound (V) having different structures. The compound (VI) can be suitably used as the material of an optical film or the like.

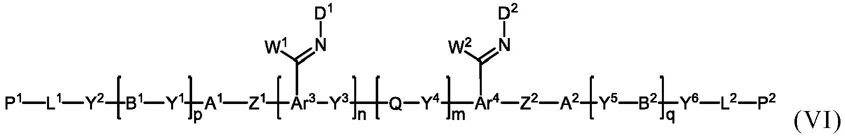

In the aforementioned formula (VI), W¹ and W² are the same as defined above, and the preferred examples thereof are the same, Ar³ and Ar⁴ are the same as defined above for Ar¹ and Ar², and the preferred examples thereof are the same, D¹ and D² are the same as defined above for D, and the preferred examples thereof are the same, A¹, A², B¹, B², Y¹ to Y⁶, L¹, L², P¹, P², Z¹, Z², Q, p, q, n and m are the same as defined above, and the preferred examples thereof are the same.

In the aforementioned formula (VI), Ar³-W¹C=N-D¹ and Ar⁴-W²C=N-D² is a group formed by reacting Ar¹-X¹ or Ar²-X² with the compound (V), and each preferably represent any of the following formulas (IX-1) to (IX-14).

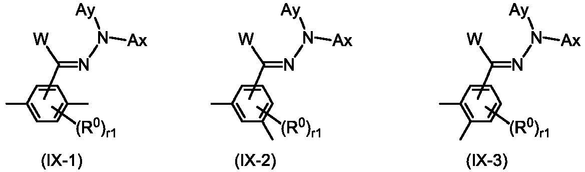

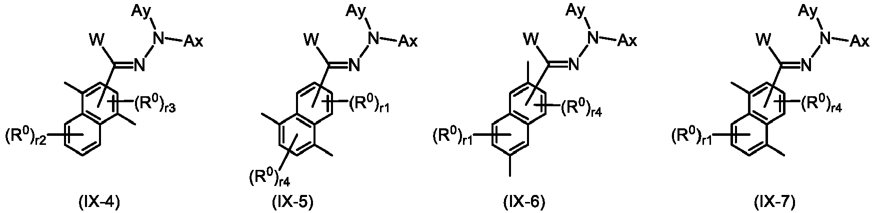

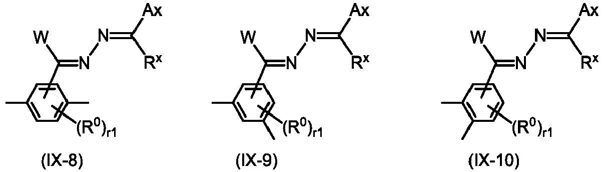

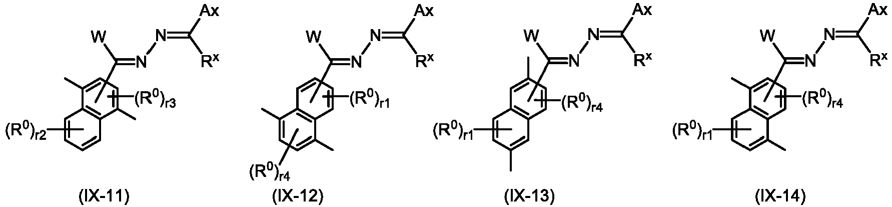

In the aforementioned formulas (IX-1) to (IX-14), Ax is the same as defined above, and the preferred examples thereof are the same, Ay and R^{x} are the same as defined above, and the preferred examples thereof are the same, W is the same as defined above, and the preferred examples thereof are the same, R⁰ is the same as defined above, and the preferred examples thereof are the same, with the proviso that when there is a plurality of R⁰, each R⁰ may be the same or may be different.

In the aforementioned formulas (IX-1) to (IX-14), r1 is the same as defined above, and the preferred examples thereof are the same, r2 is the same as defined above, and the preferred examples thereof are the same, r3 is the same as defined above, and the preferred examples thereof are the same, r4 is the same as defined above, and the preferred examples thereof are the same.

The polymerizable compound represented in the aforementioned formula (VI) is preferably represented by any of the following formulas (VI-1) to (VI-12).

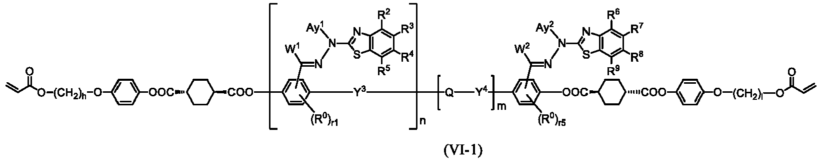

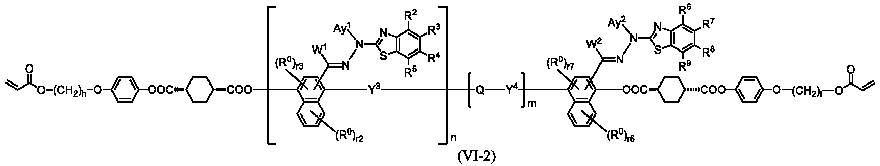

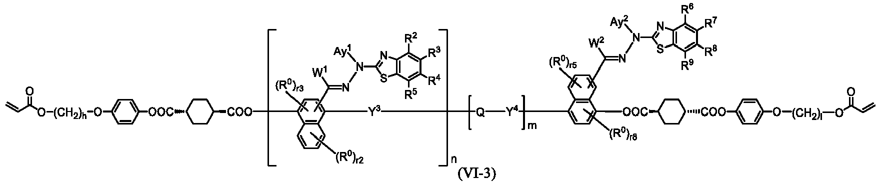

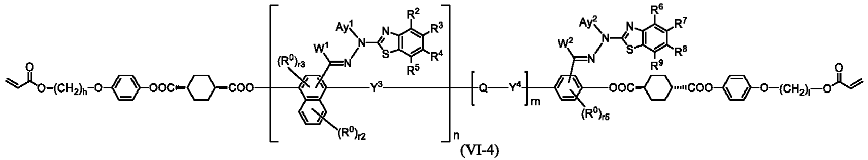

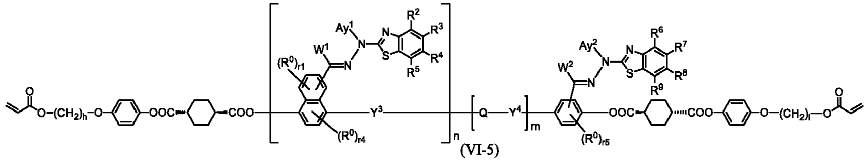

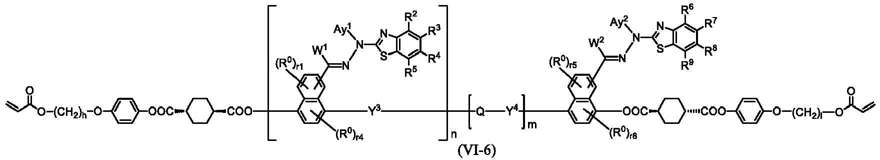

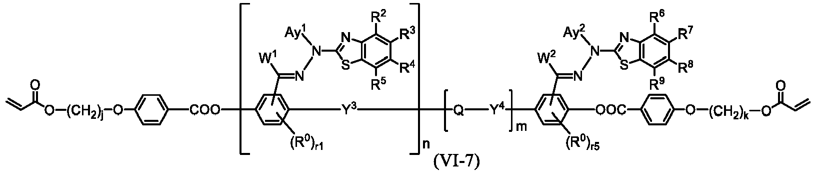

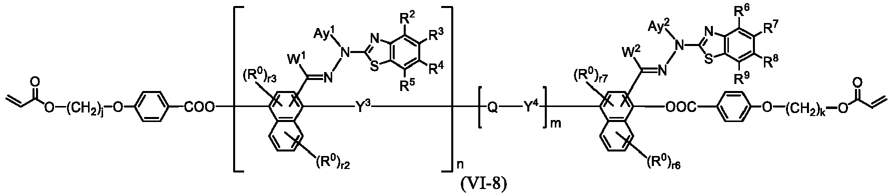

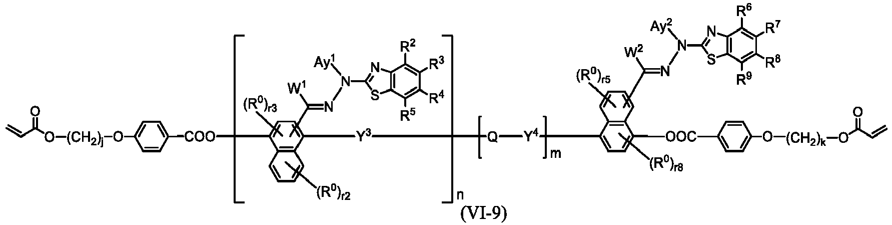

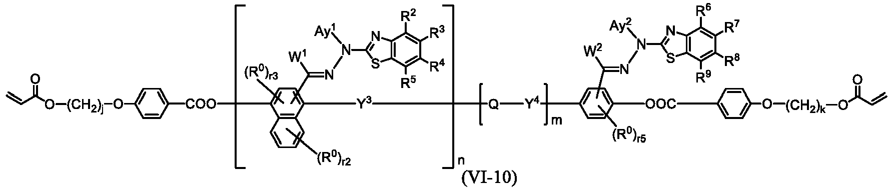

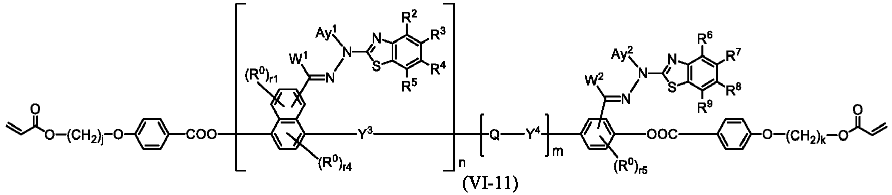

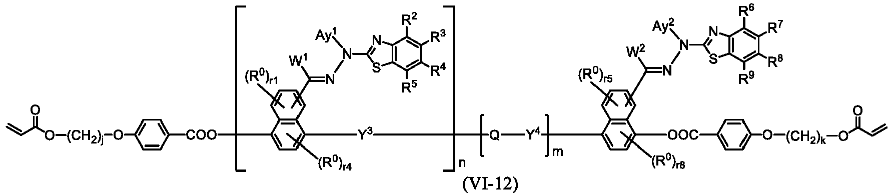

In the aforementioned formulas (VI-1) to (VI-12), W¹ and W² are the same as defined above, and the preferred examples thereof are the same, Ay¹ and Ay² are the same as defined above for Ay, and the preferred examples thereof are the same, n and m are the same as defined above, and the preferred examples thereof are the same, R² to R⁵ are the same as defined above, and the preferred examples thereof are the same, R⁶ to R⁹ are the same as defined above for R² to R⁵, and the preferred examples thereof are the same. The plurality of R² to R⁹ may be the same or different, and one or more ring constituent C-R² to C-R⁹ may be replaced by a nitrogen atom.

In the aforementioned formulas (VI-1) to (VI-12), R⁰ is the same as defined above, and the preferred examples thereof are the same. Here, when there is a plurality of R⁰, each R⁰ may be the same or may be different.

In the aforementioned formulas (VI-1) to (VI-12), r1 to r4 are the same as defined above, and the preferred examples thereof are the same, r5 to r8 are the same as defined above for r1 to r4, and the preferred examples thereof are the same.

In the aforementioned formulas (VI-1) to (VI-12), h, 1, j and k preferably each independently represent a group represented by an alkylene group having 1 to 20 carbon atoms which may be substituted with a fluorine atom, or a group represented by -(CH₂)X-C(=O)-O-(CH₂)_{y}- which may be substituted with a fluorine atom (in the formula, x and y respectively represents an integer from 2 to 12, and preferably, represent an integer from 2 to 8), more preferably an alkylene group having 2 to 12 carbon atoms which may be substituted with a fluorine atom, even more preferably an unsubstituted alkylene group having 2 to 12 carbon atoms, and a group represented by -(CH₂)z- (in the formula, z represents an integer from 2 to 12, and preferably, represents an integer from 2 to 8) is particularly preferable.

In the aforementioned formulas (VI-1) to (VI-12), Y³, Y⁴ and Q are the same as defined above, and the preferred examples thereof are the same.

### <Acid addition>

In the aforementioned Step 2, the reaction is preferably performed by adding the compound represented by the aforementioned formula (V) and an acid to the aforementioned reaction solution obtained in the Step 1.

Examples of the acid include an inorganic acid such as hydrochloric acid, sulfuric acid, phosphoric acid, carbonic acid, boric acid, perchloric acid, and nitric acid; and an organic acid such as a carboxylic acid such as formic acid, acetic acid, oxalic acid, citric acid, and trifluoroacetic acid; a sulfonic acid such as p-toluenesulfonic acid, methanesulfonic acid, trifluoromethanesulfonic acid, and 10-camphorsulfonic acid; and a sulfinic acid such as benzenesulfinic acid. These acids may be used either alone or in combination.

Thereamong, from the viewpoint that the target product can be obtained in high yield, an inorganic acid or an organic acid having 1 to 20 carbon atoms are preferable, hydrochloric acid, sulfuric acid, phosphoric acid, boric acid, sulfonic acid, sulfinic acid, formic acid, acetic acid and oxalic acid are preferable, and hydrochloric acid and sulfonic acid are particularly preferable.

Further, the acid is preferably used in the form of an acidic aqueous solution. The acidic aqueous solution to be used is not specifically limited, but the pH of the acidic aqueous solution is preferably 6 or less, and more preferably 2 or less. Here, by the acid being contained in the form of an acidic aqueous solution, and the organic solvent being a water-immiscible organic solvent which is described later, a high purity target product can be obtained in a higher yield.

The concentration of the acidic aqueous solution is preferably 0.1 mol/liter to 2 mol/liter.

The amount of the acidic aqueous solution used is preferably an amount that when the compound (V) and the acidic aqueous solution are added to the reaction solution, can sufficiently perform the reaction with the salt produced by the reaction being completely dissolved in the acidic aqueous solution. For example, when using a 1.0 N acidic aqueous solution, 1 to 20 parts by mass, and preferably 5 to 15 parts by mass is used based on 10 parts by mass of the compound (I).

### <Step 2>

The aforementioned Step 2 is the step for adding the compound (V) to the reaction solution obtained in the Step 1 to react the compound (III) with the compound (V).

By this reaction, the target compound (VI) can be obtained in high yield and at a high purity.

The amount of the compound (V) used as the ratio with the compound (III) in terms of molar ratio (compound (III):compound (V)) is preferably 1:1 to 1:2, more preferably 1:1 to 1:1.5, and 1:1.2 to 1:1.5 is particularly preferable.

The reaction of the Step 2 is performed by adding the compound (V) to the reaction solution obtained in the Step 1. As stated above, since the reaction solution obtained in the Step 1 is used as is without a post-treatment operation such as washing and extraction, it is possible to reduce the cost.

The compound (V) may be dissolved while adding into an organic solvent if desired. The organic solvent to be used can use the same solvent as listed in the Step 1.

A high purity compound (VI) having a very low ionic impurity content can be obtained in high yield by adding the compound (V) while the acidic aqueous solution in the reaction solution obtained in the Step 1 and performing the reaction with a salt produced by the reaction is completely dissolved in acidic aqueous solution. That is, by adding an acidic aqueous solution to the reaction solution containing the compound (III), the salt produced as a by-product by the reaction of the Step 1 is completely dissolved in the reaction solution and excluded from the reaction system, thus, it is considered that the ionic impurity content in the compound (VI) obtainable by the reaction between the compound (III) and the compound (V) can be reduced, and the target product can be obtained in high yield.

At least one of the organic solvents (first organic solvent) used in the Step 1, or the organic solvent (second organic solvent) used when adding the compound (V) in the Step 2 in the form of the organic solvent solution, or preferably both are preferably water-immiscible organic solvents. By using a water-immiscible organic solvent as the first organic solvent and/or the second organic solvent, a higher purity compound (VI) having a lower ionic impurity content can be obtained at a higher yield.

Here, "the term he water-immiscible organic solvent" refers to an organic solvent that has a solubility in 20°C water of 10 g (organic solvent)/100 mL (water) or less, preferably 1 g (organic solvent)/100 mL (water) or less, and more preferably 0.1 g (organic solvent)/100 mL (water) or less.

Examples of the water-immiscible organic solvent include an ester such as ethyl acetate, isopropyl acetate, butyl acetate, dimethyl carbonate, and diethyl carbonate; a halogenated hydrocarbon such as methylene chloride, chloroform, and 1,2-dichloroethane; an aromatic hydrocarbon such as benzene, toluene, and xylene; a saturated hydrocarbon such as pentane, hexane, and heptane; an ether such as diethyl ether and cyclopentyl methyl ether; an alicyclic hydrocarbon such as cyclopentane and cyclohexane; and the like.

The reaction temperature of the Step 2 is from -20°C to the boiling point of the solvent to be used, preferably 0°C to 80°C. The reaction time is normally several minutes to 10 hours based on the reaction scale.

When the reaction solution is separated into an organic layer and an aqueous layer after completion of the reaction, water (sodium chloride solution) and a water-immiscible organic solvent are optionally added to the reaction solution to effect separation, and the organic layer is collected.

When the reaction solution is not separated into two layers, water (sodium chloride solution) and a water-immiscible organic solvent are optionally added to the reaction solution to effect separation, and the organic layer is collected.

In either case, the obtained layer is subjected to a post-treatment operation that is normally employed in synthetic organic chemistry, optionally followed by a known separation-purification means such as precipitation, recrystallization, distillation, and column chromatography to isolate the target compound (VI).

Either or a combination of both of an adsorbent and a filter aid can be used to reduce the ionic impurity content and remove insoluble substances (high-molecular-weight substance).

Here, examples of the adsorbent include activated carbon, silica gel (main component: SiO₂), a synthetic adsorbent (main component: MgO, Al₂O₃, and SiO₂), activated clay, alumina, an ion-exchange resin, an adsorbent resin, and the like.

Examples of the filter aid include diatomaceous earth, silica gel (main component: SiO₂), a synthetic zeolite, pearlite, Radiolite, and the like.

Thereamong, from the viewpoint that the high-purity target product can be obtained in high yield using a simple operation, a method that concentrates the obtained organic layer, and precipitates crystals of the target product from the concentrate, or a method that concentrates the obtained organic layer, and adds a poor solvent to the concentrate to precipitate crystals of the target product is preferable.

Examples of the poor solvent used for the latter method include water; an alcohol such as methanol and ethanol; and the like; an aliphatic hydrocarbon such as hexane and heptane and the like.

It is also preferable to purify the obtained crystals using a recrystallization method or a precipitation method.

The recrystallization method is a method in which the obtained (crude) crystals are dissolved in a small amount of solvent (so that part of the crystals remains undissolved), the solution heated to effect complete dissolution, the resulting solution subjected to hot filtration to remove the insoluble substances, and then, the filtrate cooled to precipitate crystals.

Examples of the solvent used for recrystallization include an alcohol such as methanol, ethanol and isopropanol; an aliphatic hydrocarbon such as hexane and heptane; an aromatic hydrocarbon such as toluene and xylene; an ether such as tetrahydrofuran; and an ester such as ethyl acetate.

The precipitation method is a method in which the obtained (crude) crystals are dissolved in a small amount of a good solvent, and a poor solvent is added to precipitate the crystals. Examples of the good solvent used in the precipitation method include an ester such as tetrahydrofuran, an ester such as ethyl acetate, and examples of the poor solvent include water; an alcohol such as methanol, ethanol and isopropanol; an aliphatic hydrocarbon such as hexane and heptane; and an aromatic hydrocarbon such as toluene and xylene.

Further, it is also preferable to add an antioxidant such as 2,6-di-t-butyl-4-methylphenol to the recrystallization solvent and the good solvent in the precipitation method in order to obtain a high-purity product. The amount of antioxidant added is 1 to 500 mg based on 100 g of the crystals of the target product.

In the recrystallization method and the precipitation method, the temperature for precipitating or growing the crystals is not specifically limited as long as it is a temperature at which the crystals precipitate, but is normally -20°C to 50°C, preferably -5°C to 40°C, and 0°C to 25°C is particularly preferable.

The structure of the target product can be identified by measurements such as NMR spectrum, IR spectrum, or mass spectrum), elementary analysis, or the like.

### ((2-1) Solution (First Solution))

The solution (first solution) of the present disclosure is a solution including the polymerizable compound represented by the aforementioned formula (III) obtained using the disclosed method of producing a polymerizable compound, and including the polymerizable compound represented by the aforementioned formula (XII) in accordance with need.

The solution may also include the aforementioned organic solvent and other formulations.

### ((2-2) Solution (Second solution))

The solution (second solution) of the present disclosure is a solution including the polymerizable compound represented by the aforementioned formula (VI) obtained using the disclosed method of producing a polymerizable compound, and including the polymerizable compound represented by the aforementioned formula (XII) in accordance with need.

The solution may also include the aforementioned organic solvent and other formulations.

### EXAMPLES

The present disclosure will be described below in detail with reference to examples. However, the present disclosure is not limited to the following examples.

### (Synthesis Example 1) Synthesis of Compound B (Example of the compound represented by formula (II))

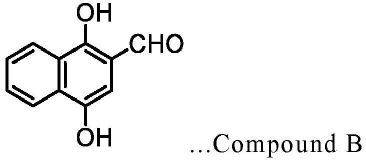

### <Step 1: Synthesis of Intermediate a>

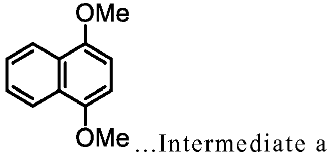

20.0 g (125 mmol) of 1,4-dihydroxynaphthalene was dissolved in 200 ml of N-N-dimethylformamide in a four-necked reactor equipped with a thermometer, under a nitrogen stream. 51.8 g (375 mmol) of potassium carbonate and 19.4 ml (312 mmol) of methyl iodide was added to the solution, and the solution was stirred at room temperature for 20 hours. After completion of the reaction, the reaction solution was filtered on Celite. 500 ml of the filtrate was charged in water, and extracted with 500 ml of ethyl acetate. The ethyl acetate layer was dried with anhydrous sodium sulfate. After the sodium sulfate was filtered off, and the ethyl acetate was evaporated under reduced pressure using a rotary evaporator to obtain a white solid. The white solid was recrystallized from (125 ml) hexane to obtain 20.3 g of Intermediate a as a colorless crystal. The yield was 86.3 mol%. The structure of Intermediate A was identified by ¹H-NMR. The ¹H-NMR spectral data is shown below.
¹H-NMR (500 MHz, CDCl₃, TMS, δppm):8.19-8.22 (m, 2H), 7.52-7.48 (m, 2H), 6.69 (s, 2H), 3.95 (s, 6H).

### <Step 2: Synthesis of Intermediate b>

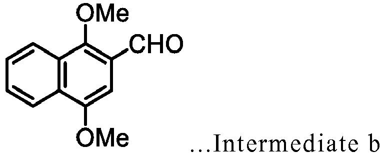

15.0 g (79.7 mmol)Intermediate a synthesized in the Step 1 was dissolved in 100 ml of dichloromethane in a four-necked reactor equipped with a thermometer, under a nitrogen stream, and cooled to 0°C. 91.7 ml (91.7 mmol) of titanium tetrachloride (1.0M dichloromethane solution), and 8.11 ml (91.7 mmol) of dichloromethyl methyl ether was added dropwise to the solution, and stirred at 0°C for 1 hour. After completion of the reaction, the reaction solution was charged in 300 ml of ice water, and extracted with 500 ml of ethyl acetate 500 ml. The ethyl acetate layer was dried with anhydrous magnesium sulfate. After filtering the magnesium sulfate, the ethyl acetate was evaporated under reduced pressure using a rotary evaporator to obtain a white solid. The white solid was recrystallized from 260 ml of hexane to obtain 16.6 g of Intermediate b as a colorless crystal. The yield was 96.4 mol%. The structure of Intermediate b was identified by ¹H-NMR. The ¹H-NMR spectral data is shown below.
¹H-NMR (500 MHz, CDCl₃, TMS, δppm):10.58 (s, 1H), 8.28-8.31 (m, 1H), 8.20-8.22 (m, 1H), 7.61-7.67 (m, 2H), 7.13 (s, 1H), 4.10 (s, 3H), 4.03 (s, 3H).

### <Step 3: Synthesis of Compound B (Example of the compound represented by formula (II))>

16.6 g (76.8 mmol) of Intermediate b synthesized in the Step 2 was dissolved in 100 ml of dichloromethane in a four-necked reactor equipped with a thermometer, under a nitrogen stream, and cooled to -40°C. 230 ml (230 mmol) of boron tribromide (17% dichloromethane solution) was added dropwise to the solution, the solution was heated to room temperature and stirred for 2 hours. After completion of the reaction, the reaction solution was charged in 500 ml of ice water, and extracted with 500 ml of dichloromethane. The dichloromethane layer was dried with anhydrous magnesium sulfate. After filtering the magnesium sulfate, the dichloromethane was evaporated from the filtrate under reduced pressure using a rotary evaporator to obtain a yellow solid. The yellow solid was purified by silica gel column chromatography (hexane:ethyl acetate = 70:30) to obtain 12.7 g of the compound B as a yellow solid The yield was 87.9 mol%. The structure of the target product (compound B) was identified by ¹H-NMR. The ¹H-NMR spectral data is shown below.
¹H-NMR (500 MHz, CDCl₃, TMS, δppm):12.31 (s, 1H), 9.88 (s, 1H), 8.45 (d, 1H, J = 8.5 Hz), 8.16 (d, 1H, J = 8.5 Hz), 7.72 (dd, 1H, J = 7.8 Hz, 8.5 Hz), 7.61 (dd, 1H, J = 7.8 Hz, 8.5 Hz), 6.83 (s, 1H), 5.17 (s, 1H).

### (Synthesis Example 2) Synthesis of Compound C (Another example of a compound represented by formula (II))

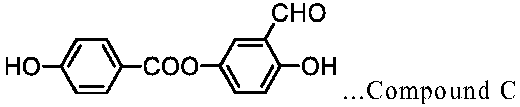

### <Step 1: Synthesis of Intermediate c>

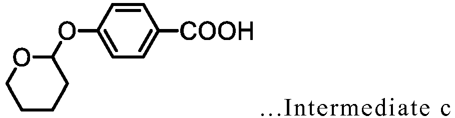

20 g (145 mmol) of 4-hydroxybenzoic acid, 14.62 g (145 mmol) of 3,4-dihydro-2H-pyran and 200 ml of tetrahydrofuran were added in a three-necked reactor equipped with a thermometer under a nitrogen stream to prepare a uniform solution. The reactor was immersed in a cold water bath to bring the internal temperature of the reaction solution to 15°C. 336 mg (1.45 mmol) of (±)-10-camphorsulfonic acid was added to the solution. Then, the entire solution as returned to 25°C and stirred for 6 hours. After completion of the reaction, 1 liter of distilled water and 100 ml of saturated saline solution were added to the obtained reaction solution, followed by extraction twice with 300 ml of ethyl acetate. The organic layer was collected, and dried with anhydrous sodium sulfate, and the sodium sulfate was filtered off. After removing the solvent using a rotary evaporator, the obtained residue was recrystallized as a solvent of ethyl acetate. The precipitated crystals were filtered. The obtained crystals were washed in cold ethyl acetate, and dried under a vacuum to obtain 9.0 g of Intermediate c as a white solid. The yield was 28 mol%. The structure of Intermediate c was identified by ¹H-NMR. The ¹H-NMR spectral data is shown below.
¹H-NMR (500 MHz, DMSO-d₆, TMS, δppm):12.66 (s, 1H), 7.89 (d, 2H, J = 9.0 Hz), 7.09 (d, 2H, J = 9.0 Hz), 5.58 (t, 1H, J = 3.5 Hz), 3.75-3.70 (m, 1H), 3.59-3.55 (m, 1H), 1.92-1.48 (m,6H).

### <Step 2: Synthesis of Intermediate d>

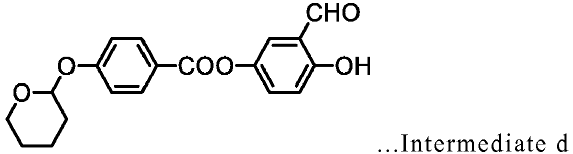

6.0 g (27 mmol) of Intermediate c synthesized in the Step 1, 3.73 g (27 mmol) of 2,5-dihydroxybenzaldehyde, 330 mg (2.7 mmol) of N-N-dimethylaminopyridine were added to 110 ml of chloroform in a three-necked reactor equipped with a thermometer under a nitrogen stream. 4.09 g (32.4 mmol) of N-N'-diisopropylcarbodiimide was gradually added therein dropwise at 25°C while stirring vigorously. Then, the solution was stirred at 25°C for 2 hours to perform the reaction. after completion of the reaction, 1 liter of distilled water and 100 ml of saturated saline solution were added to the obtained reaction solution, followed by extraction twice with 400 ml of ethyl acetate. The organic layer was collected and washed with 500 ml of saturated saline solution. The obtained organic layer was dried with anhydrous sodium sulfate, and the sodium sulfate was filtered off. After the solvent was removed using a rotary evaporator, the obtained residue was purified by silica gel column chromatography (toluene:ethyl acetate = 97:3 (volume ratio)) to obtain 4.5 g of Intermediate d as a white solid. The yield was 49 mol%. The stricture of Intermediate d was identified by ¹H-NMR. The ¹H-NMR spectral data is shown below.
¹H-NMR (500 MHz, CDCl₃, TMS, δppm):10.94 (s, 1H), 9.87 (d, 1H, J = 0.5 Hz), 8.13 (d, 2H, J = 9.0 Hz), 7.44 (d, 1H, J = 3.0 Hz), 7.37 (dd, 1H, J = 3.0 Hz, 9.0 Hz), 7.15 (d, 2H, J = 9.0 Hz), 7.02 (d, 1H, J = 9.0 Hz), 5.55 (t, 1H, J = 3.0 Hz), 3.89-3.84 (m, 1H), 3.66-3.62 (m, 1H), 2.07-1.95 (m, 1H), 1.93-1.83 (m, 2H), 1.77-1.57 (m, 3H).

### <Step 3: Synthesis of Compound C (Another example of the compound represented by formula (II))>

3.0 g (8.76 mmol) of Intermediate d synthesized in the Step 2 was added to 40 ml of a mixed solution of acetic acid / tetrahydrofuran / water = 4/2/1 (mass ratio) in a three-necked reactor equipped with a thermometer under a nitrogen stream. Then, the entire solution as returned to 45°C and stirred for 6 hours. After completion of the reaction, 500 ml of distilled water was added to the obtained reaction solution, and extracted twice with 200 ml of ethyl acetate. The organic layer was collected, dried with anhydrous sodium sulfate, and the sodium sulfate was filtered off. After the solvent was removed using a rotary evaporator, the obtained residue was purified by silica gel column chromatography (toluene:ethyl acetate = 80:20 (volume ratio)) to obtain 1.5 g of the compound C as a white solid. The yield was 66 mol%. The structure of the target product (compound C) was identified by ¹H-NMR. The ¹H-NMR spectral data is shown below.
¹H-NMR (500 MHz, THF-d₈, TMS, δppm):10.91 (s, 1H), 10.07 (s, 1H), 9.36 (s, 1H), 8.15 (d, 2H, J = 9.0 Hz), 7.69 (d, 1H, J = 3.0 Hz), 7.51 (dd, 1H, J = 3.0 Hz, 9.0 Hz), 7.10 (d, 1H, J = 9.0 Hz), 6.98 (d, 2H, J = 9.0 Hz).

### (Synthesis Example 3) Synthesis of Compound D (Still another example of a compound represented by formula (II))

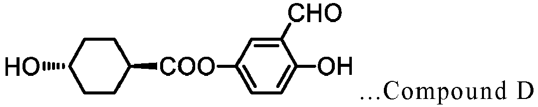

5.0 g (34.7 mmol) of trans-4-hydroxycyclohexanecarboxylic acid, 4.79 g (34.7 mmol) of 2,5-dihydroxybenzaldehyde, and 424 mg (3.47 mmol) of N-N-dimethylaminopyridine were added to 100 ml of chloroform in a three-necked reactor equipped with a thermometer under a nitrogen stream. 5.25 g (41.6 mmol) of N-N'-diisopropylcarbodiimide was slowly dripped therein at 15°C while stirring vigorously. Then, the solution was stirred at 25°C for 8 hours to perform the reaction. After completion of the reaction, 1 liter of distilled water and 100 ml of saturated saline solution were added to the obtained reaction solution, followed by extraction twice with 400 ml of ethyl acetate. The organic layer was collected and washed with 500 ml of saturated saline solution. The obtained organic layer was dried with anhydrous sodium sulfate, and the sodium sulfate was filtered off. After the solvent was removed using a rotary evaporator, the obtained residue was purified by silica gel column chromatography (toluene:ethyl acetate = 75:25 (volume ratio)) to obtain 5.0 g of the compound D as a white solid. The yield was 55 mol%. The structure of the target product (compound D) was identified by ¹H-NMR. The ¹H-NMR spectral data is shown below.
¹H-NMR (500 MHz, DMSO-d₆, TMS, δppm):10.77 (s, 1H), 10.25 (s, 1H), 7.31 (d, 1H, J = 3.0 Hz), 7.26 (dd, 1H, J = 3.0 Hz, 9.0 Hz), 7.02 (d, 1H, J = 9.0 Hz), 4.62 (d, 1H, J = 4.0 Hz), 3.44-3.36 (m, 1H), 2.49-2.45 (m, 1H), 2.04-1.99 (m, 2H), 1.90-1.86 (m, 2H), 1.52-1.43 (m, 2H), 1.27-1.19 (m, 2H).

### (Synthesis Example 4) Synthesis of Compound E (Still another example of a compound represented by formula (II))

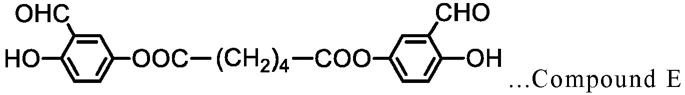

10 g (68.4 mmol) of adipic acid, 18.9 g (136.9 mmol) of 2,5-dihydroxybenzaldehyde, 836 mg (6.84 mmol) of N-N-dimethylaminopyridine, and 250 ml of chloroform 250 ml was charged in a three-necked reactor equipped with a thermometer under a nitrogen stream. 20.7 g (164.3 mmol) of N-N'-diisopropylcarbodiimide was added therein. Then, the solution was stirred at 25°C for 20 hours. After completion of the reaction, 500 ml of distilled water and 100 ml of saturated saline solution were added to the obtained reaction solution, followed by extraction twice with 300 ml of ethyl acetate. The organic layer was collected, dried with anhydrous sodium sulfate, and the sodium sulfate was filtered off. After the solvent was removed using a rotary evaporator, the obtained residue was purified by silica gel column chromatography (toluene:ethyl acetate = 90:10 (volume ratio)) to obtain 18 g of the compound E as a light yellow solid. The yield was 68.1 mol%. The structure of the target product (compound E) was identified by ¹H-NMR. The ¹H-NMR spectral data is shown below.
¹H-NMR (500 MHz, DMSO-d₆, TMS, δppm):10.84 (s, 2H), 10.25 (s, 2H), 7.35 (d, 2H, J = 3.0 Hz), 7.29 (dd, 2H, J = 3.0 Hz, 9.0 Hz), 7.02 (d, 2H, J = 9.0 Hz), 2.65-2.60 (m, 4H), 1.75-1.69 (m, 4H).

### (Synthesis Example 5) Synthesis of Compound F (Still another example of a compound represented by formula (II))

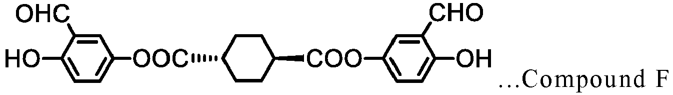

10 g (58.1 mmol) of trans-1,4-cyclohexanedicarboxylic acid, 16.0 g (116 mmol) of 2,5-dihydroxybenzaldehyde, and 710 mg (5.8 mmol) of N-N-dimethylaminopyridine were added to 350 ml of chloroform in a three-necked reactor equipped with a thermometer under a nitrogen stream. 17.6 g (139 mmol) of N-N'-diisopropylcarbodiimide was slowly dripped therein at 15°C while stirring vigorously. Then, the solution was stirred at 25°C for 6 hours to perform the reaction. after completion of the reaction, a Hirsch funnel covered with Celite was used to filter and remove the precipitate. The obtained reaction solution was dried with 0.1 N of aqueous hydrochloric acid 300 ml. Furthermore, 200 ml of saturated saline solution was added to the organic layer and washed. The obtained organic layer was dried with anhydrous sodium sulfate, and the sodium sulfate was filtered off. After the solvent was removed using a rotary evaporator, the obtained residue was purified by silica gel column chromatography (toluene:ethyl acetate = 90:10 (volume ratio)) to obtain 18 g of the compound F as a light yellow solid. The yield was 75.1 mol%. The structure of the target product (compound F) was identified by ¹H-NMR. The ¹H-NMR spectral data is shown below.
¹H-NMR (500 MHz, DMSO-d₆, TMS, δppm):10.78 (s, 2H), 10.26 (s, 2H), 7.34 (d, 2H, J = 3.0 Hz), 7.29 (dd, 2H, J = 3.0 Hz, 9.0 Hz), 7.03 (d, 2H, J = 9.0 Hz), 2.65-2.58 (m, 2H), 2.18-2.12 (m, 4H), 1.62-1.52 (m, 4H).

### (Synthesis Example 6) Synthesis of Compound G (Still another example of a compound represented by formula (II))

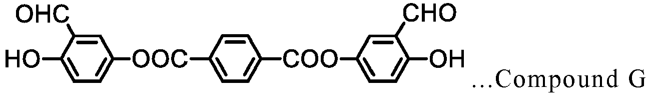

10 g (60.2 mmol) of terephthalic acid, 16.6 g (120 mmol) of 2,5-dihydroxybenzaldehyde, and 735 mg (6.0 mmol) of N-N-dimethylaminopyridine were added to 300 ml of chloroform 300 ml in a three-necked reactor equipped with a thermometer under a nitrogen stream. 18.2 g (144.5 mmol) of N-N'-diisopropylcarbodiimide was slowly dripped therein at 15°C while stirring vigorously. Then, the solution was stirred at 25°C for 12 hours to perform the reaction. After completion of the reaction, a Hirsch funnel covered with Celite was used to filter and remove the precipitate. The obtained reaction solution was dried in 0.1N of aqueous hydrochloric acid 100 ml. Furthermore, 100 ml of saturated saline solution was added to the organic layer and dried. The obtained organic layer was dried with anhydrous sodium sulfate, and the sodium sulfate was filtered off. After the solvent was removed using a rotary evaporator, the obtained residue was purified by silica gel column chromatography (toluene:ethyl acetate =85:15 (volume ratio)) to obtain 12.3 g of Compound G as a light yellow solid. The yield was 50.3 mol%. The structure of the target product (Compound G) was identified by ¹H-NMR. The ¹H-NMR spectral data is shown below.
¹H-NMR (500 MHz, DMSO-d₆, TMS, δppm):10.88 (s, 2H), 10.30 (s, 2H), 8.31 (s, 4H), 7.58 (d, 2H, J = 3.0 Hz), 7.52 (dd, 2H, J = 3.0 Hz, 9.0 Hz), 7.10 (d, 2H, J = 9.0 Hz).

### (Synthesis Example 7) Synthesis of Compound H (Still another example of a compound represented by formula (II))

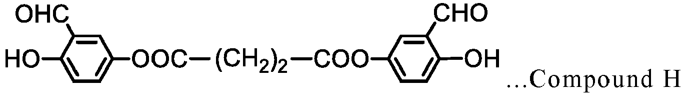

10 g (84.7 mmol) of succinic acid, 23.4 g (169.4 mmol) of 2,5-dihydroxybenzaldehyde, and 1.04 g (8.5 mmol) of N-N-dimethylaminopyridine were added to 250 ml of chloroform in a three-necked reactor equipped with a thermometer under a nitrogen stream. The solution was placed in a water bath and cooled to 0°C, and then, 25.7 g (203.3 mmol) of N-N' diisopropylcarbodiimide was added thereto. Then, the solution was stirred at 25°C for 20 hours to perform the reaction. After completion of the reaction, the resulting precipitate was filtered. The obtained filtered matter was charged into 500 ml of methanol, and was stirred and washed for 1 hour at room temperature. The filtration was performed again, and the filtered matter washed in 500 ml of methanol to obtain 19.6 g of the compound H as a white solid. The yield was 64.6 mol%. The structure of the target product (compound H) was identified by ¹H-NMR. The ¹H-NMR spectral data is shown below.
¹H-NMR (500 MHz, CDCl₃, TMS, δppm): 10.93 (s, 2H), 9.85 (s, 2H), 7.34 (d, 2H, J = 2.5 Hz), 7.27 (dd, 2H, J = 2.5 Hz, 9.0 Hz), 7.01 (d, 2H, J = 9.0 Hz), 3.01 (s, 4H).

### (Synthesis Example 8) Synthesis of Compound J (Another example of a compound represented by formula (II))

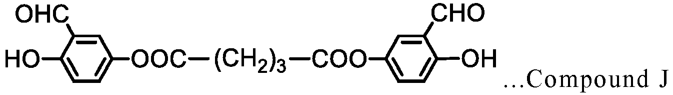

10 g (75.7 mmol) of gluraric acid, 20.9 g (151.4 mmol) of 2,5-dihydroxybenzaldehyde, and 928 mg (7.6 mmol) of N-N-dimethylaminopyridine were added to 250 ml of chloroform in a three-necked reactor equipped with a thermometer under a nitrogen stream. The solution was placed in a water bath and cooled to 0°C, and then, 22.9 g (181.7 mmol) of N-N'-diisopropylcarbodiimide was added thereto. Then, the solution was stirred at 25°C for 20 hours. After completion of the reaction, the concentration of the reaction solution was adjusted by evaporating the solvent by the rotary evaporator. 500 ml of methanol was added to the solution to precipitate a solid, and the produced solid was filtered off. The obtained filtered matter was charged into 500 ml of methanol, and was stirred and washed for 1 hour at room temperature. The filtration was performed again, and the filtered matter washed in 500 ml of methanol to obtain 18.3 g of the compound J as a white solid. The yield was 64.9 mol%. The structure of the target product (compound J) was identified by ¹H-NMR. The ¹H-NMR spectral data is shown below.
¹H-NMR (500 MHz, CDCl₃, TMS, δppm): 10.93 (brs, 2H), 9.85 (s, 1H), 9.85 (s, 1H), 7.34 (d, 2H, J = 3.0 Hz), 7.28-7.25 (m, 2H), 7.01 (d, 2H, J = 9.0 Hz), 2.75 (t, 4H, J = 7.5 Hz), 2.21 (quin, 2H, J = 7.0 Hz).

### (Synthesis Example 9) Synthesis of Compound K (Another example of a compound represented by formula (II))

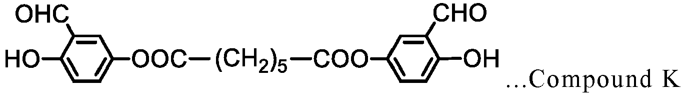

10 g (62.4 mmol) of pimelic acid, 17.2 g (124.9 mmol) of 2,5-dihydroxybenzaldehyde, and 757 mg (6.2 mmol) of N-N-dimethylaminopyridine were added to 250 ml of chloroform 250 ml in a three-necked reactor equipped with a thermometer under a nitrogen stream. The solution was placed in a water bath and cooled to 0°C, and then, 18.9 g (149.9 mmol) of N-N'-diisopropylcarbodiimide was added thereto. Then, the solution was stirred at 25°C for 20 hours. after completion of the reaction, 500 ml of distilled water and 100 ml of saturated saline solution were added to the obtained reaction solution, followed by extraction twice with 300 ml of chloroform. The organic layer was collected, dried with anhydrous sodium sulfate, and the sodium sulfate was filtered off. The concentration of the obtained organic layer was adjusted by evaporating the solvent by the rotary evaporator. 500 ml of methanol was added to the solution to precipitate a solid, and the produced solid was filtered off. The obtained filtered matter was charged into 500 ml of methanol, and was stirred and washed for 1 hour at room temperature. The filtration was performed again, and the filtered matter washed in 500 ml of methanol to obtain 16.7 g of the compound K as a white solid. The yield was 66.7 mol%. The structure of the target product (compound K) was identified by ¹H-NMR. The ¹H-NMR spectral data is shown below.
¹H-NMR (500 MHz, CDCl₃, TMS, δppm): 10.91 (s, 2H), 9.83 (s, 2H), 7.32 (d, 2H, J = 3.0 Hz), 7.24 (dd, 2H, J = 3.0 Hz, 9.0 Hz), 7.00 (d, 2H, J = 9.0 Hz), 2.62 (t, 4H, J = 7.5 Hz), 1.86-1.80 (m, 4H), 1.59-1.53 (m, 2H).

### (Synthesis Example 10) Synthesis of Compound L (Another example of a compound represented by formula (II))

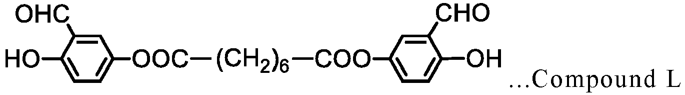

10 g (57.4 mmol) of 1,6-hexanedicarboxylic acid, 15.9 g (114.8 mmol) of 2,5-dihydroxybenzaldehyde, and 696 mg (5.7 mmol) of N-N-dimethylaminopyridine were added to 250 ml of chloroform in a three-necked reactor equipped with a thermometer under a nitrogen stream. The solution was placed in a water bath and cooled to 0°C, and then, 17.4 g (137.8 mmol) of N-N'-diisopropylcarbodiimide was added thereto. Then, the solution was stirred at 25°C for 20 hours. after completion of the reaction, 500 ml of distilled water and 100 ml of saturated saline solution were added to the obtained reaction solution, followed by extraction twice with 300 ml of chloroform. The organic layer was collected, dried with anhydrous sodium sulfate, and the sodium sulfate was filtered off. The concentration of the obtained organic layer was adjusted by evaporating the solvent by the rotary evaporator. 500 ml of methanol was added to the solution to precipitate a solid, and the produced solid was filtered off. The obtained filtered matter was charged into 500 ml of methanol, and was stirred and washed for 1 hour at room temperature. The filtration was performed again, and the filtered matter washed in 500 ml of methanol to obtain 13.8 g of the compound L as gray solid. The yield was 58.2 mol%. The structure of the target product (compound L) was identified by ¹H-NMR. The ¹H-NMR spectral data is shown below.
¹H-NMR (500 MHz, CDCl₃, TMS, δppm): 10.91 (s, 2H), 9.85 (s, 1H), 9.85 (s, 1H), 7.32 (d, 2H, J = 2.5 Hz), 7.24 (dd, 2H, J = 2.5 Hz, 9.0 Hz), 7.00 (d, 2H, J = 9.0 Hz), 2.59 (t, 4H, J = 7.5 Hz), 1.81-1.78 (m, 4H), 1.51-1.48 (m, 4H).

### (Synthesis Example 11) Synthesis of Compound M

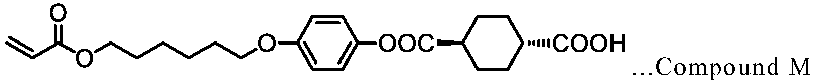

17.98 g (104.42 mmol) of trans-1,4-cyclohexanedicarboxylic acid and 180 ml of tetrahydrofuran (THF) were charged in a three-necked reactor equipped with a thermometer, under a nitrogen stream. After 6.58 g (57.43 mmol) of methanesulfonyl chloride was added therein, the reactor was immersed in a water bath to adjust the temperature of the reaction solution to 20°C. Next, 6.34 g (62.65 mmol) of triethylamine was added dropwise to the reaction solution over 10 minutes while maintaining the temperature of the reaction solution at 20 to 30°C. After the dropwise addition, the solution was stirred at 25°C for an additional 2 hours.

0.64 g (5.22 mmol) of 4-(dimethylamino)pyridine and 13.80 g (52.21 mmol) of 4-(6-acryloyloxyhex-1-yloxy)phenol (manufactured by DKSH) were added to the obtained reaction solution, and the reactor was again immersed in a water bath to adjust the temperature of the reaction solution to 15°C. 6.34 g (62.65 mmol) of triethylamine was dropped over 10 minutes in the solution while maintaining the internal temperature of the reaction solution at 20°C to 30°C, and after the dropwise addition, the solution was stirred at 25°C for an additional 2 hours. After completion of the reaction, 1000 ml of distilled water and 100 ml of saturated saline solution were added to the reaction solution, followed by extraction twice with 400 ml of ethyl acetate. The organic layer was collected, and dried with anhydrous sodium sulfate, and the sodium sulfate was filtered off. After the solvent was evaporated from the filtrate using a rotary evaporator, the obtained residue was purified by silica gel column chromatography (THF:toluene = 1:9 (volume ratio)) to obtain 14.11 g of the compound M as a white solid. The yield was 65 mol%. The structure of the target product (compound M) was identified by ¹H-NMR. The ¹H-NMR spectral data is shown below.
¹H-NMR (500 MHz, DMSO-d₆, TMS, δppm):12.12 (s,1H), 6.99 (d,2H,J = 9.0 Hz), 6.92 (d,2H,J = 9.0 Hz), 6.32 (dd,1H,J = 1.5 Hz,17.5 Hz), 6.17 (dd,1H,J = 10.0 Hz,17.5 Hz), 5.93 (dd,1H,J = 1.5 Hz,10.0 Hz), 4.11 (t,2H,J = 6.5 Hz), 3.94 (t,2H,J = 6.5 Hz), 2.48-2.56 (m,1H), 2.18-2.26 (m,1H), 2.04-2.10 (m,2H), 1.93-2.00 (m,2H), 1.59-1.75 (m,4H), 1.35-1.52 (m,8H).

### (Synthesis Example 12) Synthesis of Mixture O (Example of a compound represented by formula (I) and mixture of the polymerizable compound represented by formula (XII))

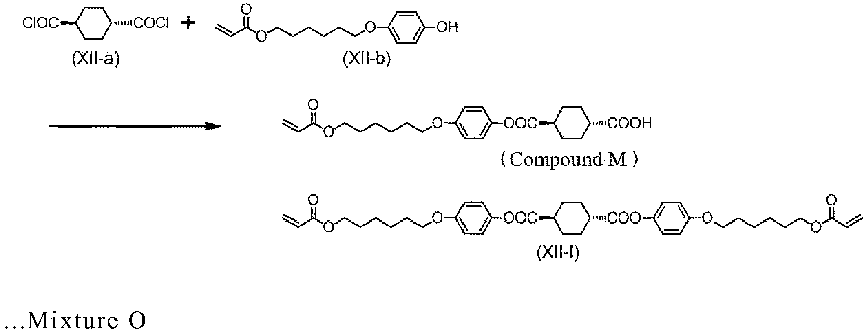

10.0 g (47.83 mmol) of trans-1,4-cyclohexanedicarboxylic acid dichloride represented by formula (XII-a), 84 ml of cyclopentyl methyl ether (CPME) and 31 ml of tetrahydrofuran (THF) were added in a three-necked reactor equipped with a thermometer, under a nitrogen stream. 12.04 g (45.55 mmol) of 4-(6-acryloyloxyhex-1-yloxy)phenol (manufactured by DKSH Management Ltd.) represented by formula (XII-b) were added to the solution and the reactor was immersed in an ice bath to bring the internal temperature of the reaction solution to 0°C. Next, 4.83 g (47.83 mmol) of triethylamine was gradually dropped over 5 minutes while maintaining the internal temperature of the reaction solution at 10°C or less. After the dropwise addition, the entire solution was maintained at 10°C or less while furthermore stirring for 1 hour.

30 ml of distilled water was added to the obtained solution. After raising the temperature of the reaction solution to 50°C, and after washing (hydrolysis reaction) for 2 hours, the water layer was extracted. Furthermore, after 30 ml of distilled water was added to the obtained organic layer, the entire solution was washed (hydrolysis reaction) at 50°C for 2 hours, and the water layer was extracted. After cooling the obtained organic layer to 40°C, 50 g of a buffer solution (pH: 5.5) consisting of acetic acid and sodium acetate at a concentration of 1 mol/liter was added and washed by stirring. Then, the buffer solution water layer was extracted and the organic layer was obtained. The washing operation for the buffer solution was performed a total of five times. After further washing the obtained organic layer in 30 ml of distilled water 30 ml, the water layer was extracted.

After adding 214 ml of n-hexane to the obtained organic layer at 40°C, the solution as cooled to 0°C and the crystals precipitated. Then, the precipitated crystals were filtered off by filtration. After washing the filtered material with n-hexane, the product was dried under a vacuum to obtain 16.78 g of the Mixture O as a white solid.

When the obtained crystals were analyzed by HPLC, and quantified by a calibration curve, and it is understood the target product (Mixture O) containing 11.49 g (27.45 mmol) of the compound M and 5.29 g (7.96 mmol) of the compound (example of polymerizable compound represented by formula (XII)) represented by the formula (XII-I) was obtained. Further, when the obtained crystals were analyzed by ¹³C-NMR (DMF-d₇), and the cyclohexanedicarboxylic acid content was calculated, it was determined to be under the detection limit. When the molar content is calculated from each composition ratio, the monoester content was 77.52 mol% and the diester content was 22.48 mol%.

### (Synthesis Example 13) Synthesis of Compound P (Example of a compound represented by formula (V-I))

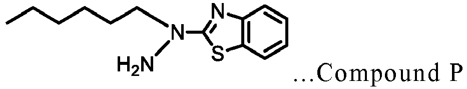

2.00 g (12.1 mmol) of 2-hydrazinobenzothiazole was dissolved in 20 ml of dimethylformamide in a four-necked reactor equipped with a thermometer, under a nitrogen stream. 8.36 g (60.5 mmol) of potassium carbonate and 3.08 g of 1-iodohexane were added to the solution and stirred at 50°C for 7 hours. After completion of the reaction, the reaction solution was cooled to 20°C, the reaction solution was charged in 200 ml of water, and extracted with 300 ml of ethyl acetate. The ethyl acetate layer was dried with anhydrous sodium sulfate. After the sodium sulfate was filtered off, and the ethyl acetate was evaporated under reduced pressure using a rotary evaporator to obtain a yellow solid. The yellow solid was purified by silica gel column chromatography (hexane:ethyl acetate = 75:25 (volume ratio)) to obtain 2.10 g of the compound P as a white solid. The yield was 69.6 mol%. The structure of the target product (compound P) was identified by ¹H-NMR.¹H-NMR spectral data is shown below.
¹H-NMR (500 MHz, CDCl₃, TMS, δppm):7.60 (dd, 1H, J = 1.0, 8.0 Hz), 7.53 (dd, 1H, J = 1.0, 8.0 Hz), 7.27 (ddd, 1H, J = 1.0, 8.0, 8.0 Hz), 7.06 (ddd, 1H, J = 1.0, 8.0, 8.0 Hz), 4.22 (s, 2H), 3.74 (t, 2H, J = 7.5 Hz), 1.69-1.76 (m, 2H), 1.29-1.42 (m, 6H), 0.89 (t, 3H, J = 7.0 Hz).

### (Synthesis Example 14) Synthesis of Compound R (Another example of a compound represented by formula (V-I))

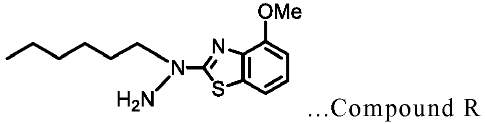

### <Step 1: Synthesis of Intermediate e>

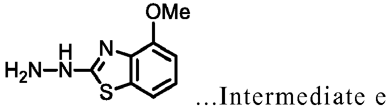

40 ml of 2-amino-4-methoxybenzothiazole, 4.00 g (22.2 mmol) of ethylene glycol and 15 ml of water were dissolved in a four-necked reactor equipped with a thermometer, under a nitrogen stream. 11.1 g (222 mmol) of hydrazine monohydrate and 2.8 ml (33.3 mmol) of a 12N hydrochloric acid were added to the solution and stirred at 120°C for 15 hours. After completion of the reaction, the reaction solution was cooled to 20°C, the reaction solution was charged in 200 ml of a 10% sodium bicarbonate water, and extracted with 800 ml of ethyl acetate. The ethyl acetate layer was dried with anhydrous sodium sulfate. After the sodium sulfate was filtered off, and the ethyl acetate was evaporated under reduced pressure using a rotary evaporator to obtain a yellow solid. The yellow solid was recrystallized from ethyl acetate to obtain 2.3 g of Intermediate e. The yield was 53.1 mol%. The structure of the target product (Intermediate e) was identified by ¹H-NMR. The ¹H-NMR spectral data is shown below.
¹H-NMR (500 MHz, DMSO-d6, TMS, δppm):8.93 (s, 1H), 7.27 (dd, 1H, J = 1.0 Hz, 8.0 Hz), 6.94 (dd, 1H, J = 8.0 Hz, 8.0 Hz), 6.82 (dd, 1H, J = 1.0 Hz, 8.0 Hz), 5.00 (s, 2H), 3.82 (s, 3H).

### <Step 2: Synthesis of Compound R (Another example of a compound represented by formula (V-I))>

2.00 g (10.2 mmol) of Intermediate e synthesized in the Step 1 was dissolved in 20 ml of dimethylformamide in a four-necked reactor equipped with a thermometer, under a nitrogen stream., 6.68 g (20.4 mmol) of cesium carbonate and 2.0 g of (12.2 mmol) 1-bromohexane was added to the solution, and stirred at 50°C for 6 hours. After completion of the reaction, the reaction solution was cooled to 20°C, the reaction solution was charged in 200 ml of water, and extracted with 300 ml of ethyl acetate. The ethyl acetate layer was dried with anhydrous sodium sulfate. After the sodium sulfate was filtered off, and the ethyl acetate was evaporated under reduced pressure using a rotary evaporator to obtain a yellow solid. The yellow solid was purified by silica gel column chromatography (hexane:THF = 80:20 (volume ratio))to obtain 2.0 g of the compound R as a white solid. The yield was 70.2 mol%. The structure of the compound R was identified by ¹H-NMR. The ¹H-NMR spectral data is shown below.
¹H-NMR (500 MHz, CDCl₃, TMS, δppm):7.22 (dd, 1H, J = 1.0 Hz, 8.0 Hz), 7.04 (dd, 1H, J = 8.0 Hz, 8.0 Hz), 6.81 (dd, 1H, J = 1.0 Hz, 8.0 Hz), 4.26 (s, 2H), 3.98 (s, 3H), 3.73 (t, 2H, J = 7.5 Hz), 1.75-1.69 (m, 2H), 1.41-1.27 (m, 6H), 0.89 (t, 3H, J = 7.0 Hz).

### (Example 1) Synthesis of Polymerizable compound (1-1) (Example of the polymerizable compound represented by formula (VI))

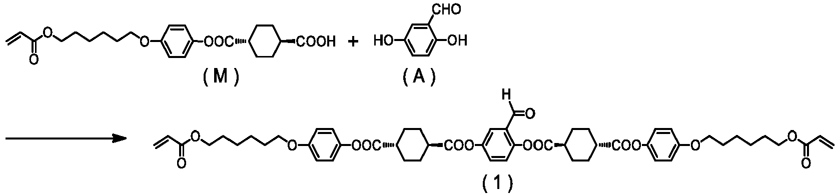

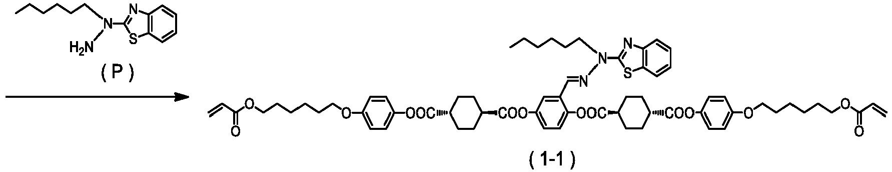

10.00 g (23.90 mmol) of the compound M synthesized in the Synthesis Example 11, 100 g of chloroform, and 3.49 g of dimethylformamide (DMF) were added in a three-necked reactor equipped with a thermometer, under a nitrogen stream, and cooled to 10°C or less. 3.27 g (27.48 mmol) of thionyl chloride was dropped in the solution while controlling the reaction temperature to 10°C or less. After the dropwise addition, the reaction solution was returned to 25°C and stirred for 1 hour. After completion of the reaction, the amount of the reaction solution was concentrated using an evaporator until the amount of the reaction solution became a quarter of the initial amount. Then, 25 g of chloroform was added to obtain the chloroform solution.

Separately, 1.50 g (10.86 mmol) of 2,5-dihydroxybenzaldehyde represented by the aforementioned formula (A), and 6.98 g (65.17 mmol) of 2,6-lutidine (pKa: 6.65) as a base were dissolved in 50 g of chloroform in a three-necked reactor equipped with a thermometer under a nitrogen stream, and cooled to 10°C or less. A chloroform solution was gradually dropped in the solution while maintaining the internal temperature of the reaction solution at 10°C or less. After the dropwise addition, the reaction solution was further reacted for 1 hour while maintaining at 10°C or less (Step 1).

When the obtained reaction solution was analyzed by high performance liquid chromatography (HPLC) and quantified by a calibration curve, it is understood that 9.77 g (10.40 mmol) of the polymerizable compound (polymerizable compound (1): example of the compound represented by formula (III)) represented by the aforementioned formulas (1) was contained. The yield was 95.75 mol%.

Furthermore, 3.52 g (14.12 mmol) of the compound P (example of the compound represented by formula (V-I)) synthesized in the Synthesis Example 13 was added to the obtained reaction solution at 10°C or less, and furthermore, 40 g of a 1.0N of aqueous hydrochloric acid was added. Then, the reaction solution was heated to 40°C and the reaction was performed for 3 hours (Step 2).

When the obtained reaction solution was analyzed by HPLC, and quantified by a calibration curve, it is understood that 11.76 g (10.05 mmol) of the polymerizable compound represented by the aforementioned formula (1-1) (polymerizable compound (1-1): example of the compound represented by formula (VI)) was contained. The yield was 92.49 mol%.

After completion of the reaction, the reaction solution was cooled to 25°C, and the liquid separation operation was performed.

After 0.50 g of ROKAHELP #479 (manufactured by Mitsui Mining and Smelting Co., Ltd.) was added to the obtained organic layer and stirred for 30 minutes, the ROKAHELP #479 was filtered off. Next, approximately 80% of the total weight was extracted from the obtained reaction solution and concentrated using an evaporator. After adding 20 g of THF to the solution THF 20 g, the solution was stirred for 1 hour. Next, after 80 g of n-hexane was dropped in the solution, the solution was cooled to 0°C to precipitate the crystals. Then, the precipitated crystals were filtered off by filtration.

After adding 108 g of THF, 1.8 g of ROKAHELP #479, and 100 mg of 2,6-di-t-butyl-4-methylphenol to the obtained crystals and stirring for 30 minutes, the ROKAHELP #479 was filtered off. Next, 36 g of THF was evaporated from the obtained reaction solution using an evaporator. After 117 g of methanol was dropped in the obtained solution, the solution was cooled to 0°C to precipitate the crystals. Then, the precipitated crystals were filtered off by filtration. After the filtered material was washed in methanol, it was dried under a vacuum to obtain 11.45 g of polymerizable compound (1-1) (example of the compound represented by formula (VI)) (Step 3). The isolated yield was 90.03 mol%.

### (Example 2) Synthesis of Polymerizable compound (1-1) (Example of a compound represented by formula (VI))

With the exception that 6.98 g (65.17 mmol) of 2,6-lutidine (pKa: 6.65) which is the base in the Step 1 of Example 1 was replaced with 6.98 g (65.17 mmol) of 2,4-lutidine (pKa: 6.99), the same operations as in Example 1 were performed. When the reaction solution was analyzed by the same method, and quantified with a calibration curve, it is understood to contain 9.57 g (10.20 mmol) of the polymerizable compound (1) (example of the compound represented by formula (III)). The yield was 93.86 mol%.

When the same operation was performed as in the Step 2 of Example 1, the reaction solution was analyzed by the same method, and quantified with a calibration curve, it is understood to contain 11.56 g (9.87 mmol) of the polymerizable compound (1-1) (example of the compound represented by formula (VI)) which is the target product. The yield was 90.91 mol%.

Furthermore, when the same operation as in the Step 3 of Example 1 was performed, 11.25 g of the polymerizable compound (1-1) (example of the compound represented by formula (VI)) was obtained. The isolated yield was 88.49 mol%.

### (Example 3) Synthesis of Polymerizable compound (1-1) (Example of the compound represented by formula (VI))

With the exception that 6.98 g (65.17 mmol) of 2,6-lutidine (pKa: 6.65) which is the base in the Step 1 of Example 1 was replaced with 7.90 g (65.17 mmol) of 2,4,6-collidine (pKa: 7.43), the same operation as in Example 1 was performed. When the reaction solution was analyzed by the same method, and quantified with a calibration curve, it is understood to contain 9.63 g (10.25 mmol) of the polymerizable compound (1) (example of the compound represented by formula (III)). The yield was 94.39 mol%.

When the same operation as in the Step 2 of Example 1 was performed, and the reaction solution was analyzed by the same method, and quantified with a calibration curve, it is understood to contain 11.57 g (9.89 mmol) of the polymerizable compound (1-1) (example of the compound represented by formula (VI)) which is the target product. The yield was 91.01 mol%.

Furthermore, when the same operation as in the Step 3 of Example 1 was performed, 11.26 g of the polymerizable compound (1-1) (example of the compound represented by formula (VI)) was obtained. The isolated yield was 88.59 mol%.

### (Example 4) Synthesis of Polymerizable compound (1-1) (Example of the compound represented by formula (VI))

With the exception that 6.98 g (65.17 mmol) of 2,6-lutidine (pKa: 6.65) which is the base in the Step 1 of Example 1 was replaced with 6.98 g (65.17 mmol) of 3,5-lutidine (pKa: 6.15), the same operation as in Example 1 was performed. When the reaction solution was analyzed by the same method, and quantified with a calibration curve, it is understood to contain 9.19 g (9.79 mmol) of the polymerizable compound (1) (example of the compound represented by formula (III)) which is the target product. The yield was 90.12 mol%.

When the same operation as in the Step 2 of Example 1 was performed, and the reaction solution was analyzed by the same method, and quantified with a calibration curve, it is understood to contain 11.18 g (9.56 mmol) of the polymerizable compound (1-1) (example of the compound represented by formula (VI)) which is the target product. The yield was 87.98 mol%.

Furthermore, when the same operation as in the Step 3 of Example 1 was performed, 10.89 g of the polymerizable compound (1-1) (example of the compound represented by formula (VI)) was obtained. The isolated yield was 85.63 mol%.

### (Example 5) Synthesis of Polymerizable compound (1-1) (Example of the compound represented by formula (VI))

With the exception that 6.98 g (65.17 mmol) of 2,6-lutidine (pKa: 6.65) which is the base in the Step 1 of Example 1 was replaced with 6.98 g (65.17 mmol) of 3,4-lutidine (pKa: 6.46), the same operation as in Example 1 was performed. When the reaction solution was analyzed by the same method, and quantified with a calibration curve, it is understood to contain 9.26 g (9.86 mmol) of the polymerizable compound (1) (example of the compound represented by formula (III)) which is the target product. The yield was 90.77 mol%.

When the same operation as in the Step 2 of Example 1 was performed, and the reaction solution was analyzed by the same method, and quantified with a calibration curve, it is understood to contain 10.96 g (9.37 mmol) of the polymerizable compound (1-1) (example of the compound represented by formula (VI)) which is the target product. The yield was 86.22 mol%.

Furthermore, when the same operation as in the Step 3 of Example 1 was performed, 10.67 g of the polymerizable compound (1-1) (example of the compound represented by formula (VI)) was obtained. The isolated yield was 83.92 mol%.

### (Example 6) Synthesis of Polymerizable compound (1-1) (Example of the compound represented by formula (VI))

With the exception that 10.00 g (23.90 mmol) of the compound M in the Step 1 of Example 1 was replaced with 14.60 g of Mixture O (example of the mixture of the compound represented by formula (I) and the compound represented by formula (XII)) synthesized in Synthesis Example 12, the same operation as in Example 1 was performed. When the reaction solution was analyzed by the same method, and quantified with a calibration curve, it is understood to contain 9.68 g (10.31 mmol) of the polymerizable compound (1) (example of the compound represented by formula (III)) which is the target product (it is understood that a solution containing the compound represented by formula (III) and the compound represented by formula (XII) could be obtained). The yield was 94.88 mol%.

When the same operation as in the Step 2 of Example 1 was performed, and the reaction solution was analyzed by the same method, and quantified with a calibration curve, it is understood to contain 11.65 g (9.96 mmol) of the polymerizable compound (1-1) (example of the compound represented by formula (VI)) which is the target product (it is understood that the solution containing the compound represented by formula (VI) and the compound represented by formula (XII) could be obtained). The yield was 91.66 mol%.

Furthermore, when the same operation as in the Step 3 of Example 1 was performed, 11.34 g of the polymerizable compound (1-1) (example of the compound represented by formula (VI)) was obtained. The isolated yield was 89.21 mol%.

### (Example 7) Synthesis of Polymerizable compound (1-1) (example of the compound represented by formula (VI))

After the same operation as in the Step 1 of Example 1 was performed, when the reaction solution was analyzed by the same method, and quantified with a calibration curve, it is understood to contain 9.77 g (10.40 mmol) of the polymerizable compound (1) (example of the compound represented by formula (III)) which is the target product. The yield was 95.75 mol%.

With the exception that 40 g of 1.0N aqueous hydrochloric acid in the Step 2 of Example 1 was replaced with 505 mg (2.17 mmol) of (±)-10-camphorsulfonic acid, the same operation as in Example 1 was performed. When the reaction solution was analyzed by the same method, and quantified with a calibration curve, it is understood to contain 11.58 g (9.90 mmol) of the polymerizable compound (1-1) (example of the compound represented by formula (VI)) which is the target product. The yield was 91.11 mol%.

Furthermore, when the same operation as in the Step 3 of Example 1 was performed, 11.27 g of the polymerizable compound (1-1) (example of the compound represented by formula (VI)) was obtained. The isolated yield was 88.68 mol%.

### (Example 8) Synthesis of Polymerizable compound (2-1) (Another example of a compound represented by formula (V-I))

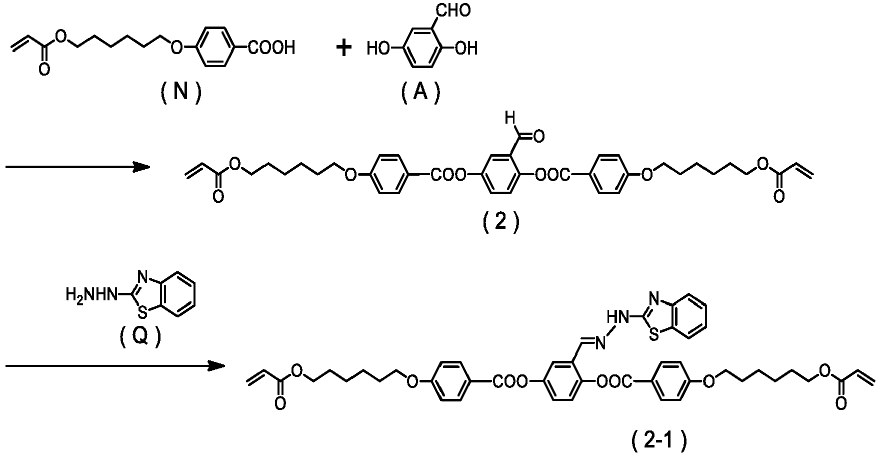

With the exception that 10.00 g (23.90 mmol) of the compound M in the Step 1 of Example 1was replaced with 6.99 g (23.90 mmol) of 4-(6-acryloylhex-1-yloxy)benzoic acid (manufactured by DKSH Management Ltd.) (another example of the compound represented by formula (I)) represented by the aforementioned formulas (N), the same operation as in Example 1 was performed. When the reaction solution was analyzed by the same method, and quantified with a calibration curve, it is understood to contain 7.00 g (10.20 mmol) of the polymerizable compound (polymerizable compound (2): another example of the compound represented by formula (III)) represented by the aforementioned formula (2). The yield was 93.91 mol%.

With the exception that 3.52 g (14.12 mmol) of Compound P (example of the compound represented by formula (V-I)) in the Step 2 of Example 1 was replaced with 2.33 g (14.12 mmol) of 2-hydrazinobenzothiazole represented by the aforementioned formula (Q), the same operation as in Example 1 was performed, the reaction solution was analyzed by the same method, and quantified with a calibration curve, it is understood to contain 8.22 g (9.85 mmol) of the polymerizable compound (2-1) represented by the aforementioned formula (2-1) (another example of a compound represented by formula (V-I)) which is the target product. The yield was 90.72 mol%.

Furthermore, when the same operation as in the Step 3 of Example 1 was performed, 8.00 g of the polymerizable compound (2-1) (another example of a compound represented by formula (V-I)) was obtained. The isolated yield was 88.30 mol%.

### (Example 9) Synthesis of Polymerizable compound (3-1) (Another example of the compound represented by formula (VI))

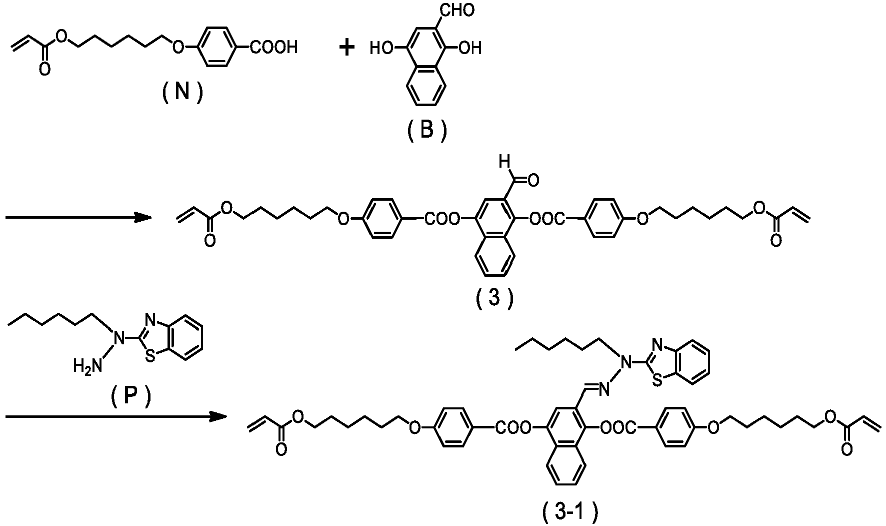

With the exception that 10.00 g (23.90 mmol) of the compound M in the Step 1 of Example 1 was replaced with 6.99 g (23.90 mmol) of 4-(6-acryloylhex-1-yloxy)benzoic acid (manufactured by DKSH Management Ltd.) (another example of the compound represented by formula (I)) represented by the aforementioned formula (N), and 1.50 g (10.86 mmol) of 2,5-dihydroxybenzaldehyde was replaced with 2.04 g (10.86 mmol) of the compound B (example of the compound represented by formula (II)) synthesized in Synthesis Example 1, the same operation as in Example 1 was performed. When the reaction solution was analyzed by the same method, and quantified with a calibration curve, it is understood to contain 7.25 g (9.83 mmol) of the polymerizable compound (polymerizable compound (3): another example of the compound represented by formula (III)) represented by the aforementioned formula (3). The yield was 90.54 mol%.

When the same operation as in the Step 2 of Example 1 was performed, and the reaction solution was analyzed by the same method, and quantified with a calibration curve, it is understood to contain 9.20 g (9.50 mmol) of the polymerizable compound (3-1) (another example of the compound represented by formula (VI)) which is the target product represented by the aforementioned formula (3-1). The yield was 87.46 mol%.

Furthermore, when the same operation as in the Step 3 of Example 1 was performed, 8.95 g of the Polymerizable compound (3-1) was obtained. The isolated yield was 85.13 mol%.

### (Example 10) Synthesis of Polymerizable compound (4-1) (Another example of the compound represented by formula (VI))

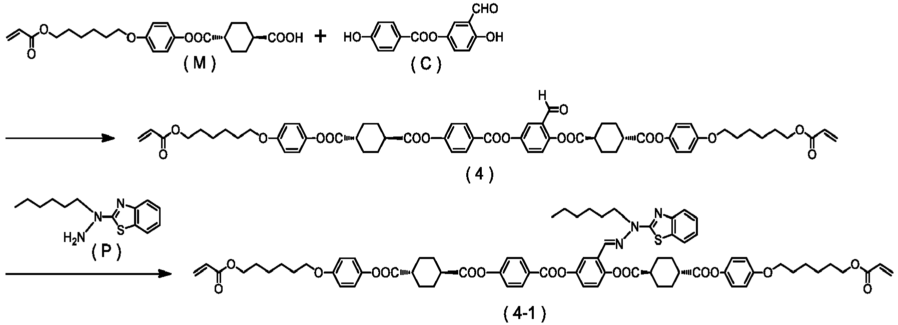

With the exception that 1.50 g (10.86 mmol) of 2,5-dihydroxybenzaldehyde in the Step 1 of Example 1 was replaced with 2.80 g (10.86 mmol) of the compound C (another example of the compound represented by formula (II)) synthesized in the Synthesis Example 2, the same operation as in Example 1 was performed. When the reaction solution was analyzed by the same method, and quantified with a calibration curve, it is understood to contain 10.83 g (10.22 mmol) of the polymerizable compound (polymerizable compound (4): another example of the compound represented by formula (III)) represented by the aforementioned formulas (4). The yield was 94.12 mol%).

When the same operation as in the Step 2 of Example 1 was performed, and the reaction solution was analyzed by the same method, and quantified with a calibration curve, it was understood to contain 12.74 (9.88 mmol) of the polymerizable compound (4-1) represented by the aforementioned formulas (4-1) (another example of the compound represented by formula (VI)) which is the target product. The yield was 90.92 mol%.

Furthermore, when the same operation as in the Step 3 of Example 1 was performed, 12.41 g of the polymerizable compound (4-1) (another example of the compound represented by formula (VI)) was obtained. The isolated yield was 88.50 mol%.

### (Example 11) Synthesis of Polymerizable compound (4-2) (Another example of the compound represented by formula (VI))

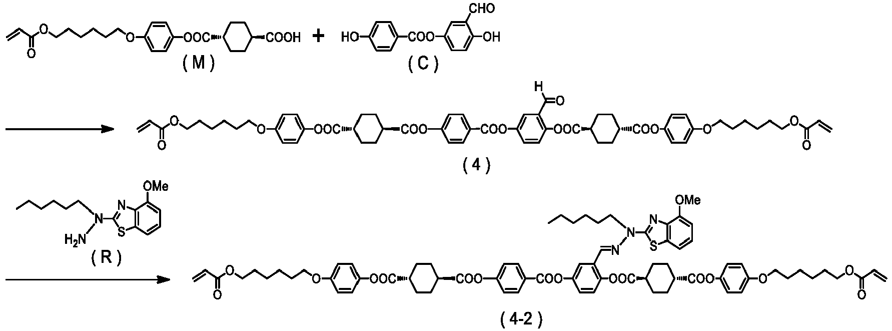

With the exception that 1.50 g (10.86 mmol) of 2,5-dihydroxybenzaldehyde in the Step 1 of Example 1 was replaced with 2.80 g (10.86 mmol) of the compound C (another example of the compound represented by formula (II)) synthesized in the Synthesis Example 2, the same operation as in Example 1 was performed. When the reaction solution was analyzed by the same method, and quantified with a calibration curve, it is understood to contain 10.83 g (10.22 mmol) of the polymerizable compound represented by the aforementioned formula (4) (polymerizable compound (4): another example of the compound represented by formula (III)). The yield was 94.1 mol%).

With the exception that 3.52 g (14.12 mmol) of the compound P (example of the compound represented by formula (V-I)) was replaced with 3.95 g (14.12 mmol) of the compound R (another example of a compound represented by formula (V-I)) synthesized in the Synthesis Example 14 in the Step 2 of Example 1, the same operation as in Example 1 was performed. When the reaction solution was analyzed by the same method, and quantified with a calibration curve, it is understood to contain 12.98 g (9.83 mmol) of the polymerizable compound (4-2) represented by the aforementioned formulas (4-2) (another example of the compound represented by formula (VI)) which is the target product. The yield was 90.48 mol%.

Furthermore, when the same operation as in the Step 3 of Example 1 was performed, 12.56 g of the polymerizable compound (4-2) (another example of the compound represented by formula (VI)) was obtained. The isolated yield was 87.56 mol%.

### (Example 12) Synthesis of Polymerizable compound (5-1) (Another example of the compound represented by formula (VI))

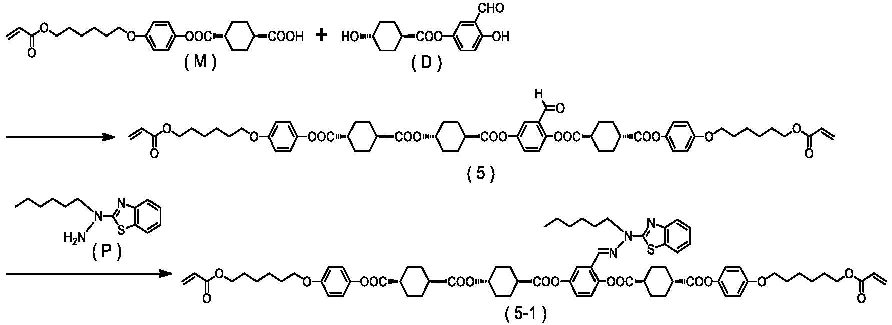

With the exception that 1.50 g (10.86 mmol) of 2,5-dihydroxybenzaldehyde in the Step 1 of Example 1 was replaced with 2.87 g (10.86 mmol) of the compound D (still another example of a compound represented by formula (II)) synthesized in the Synthesis Example 3, the same operation as in Example 1 was performed. When the reaction solution was analyzed by the same method, and quantified with a calibration curve, it is understood to contain 10.50 g (9.86 mmol) of the polymerizable compound represented by the aforementioned formula (5) (polymerizable compound (5): still another example of the compound represented by formula (III)). The yield was 90.79 mol%.

When the same operation as in the Step 2 of Example 1 was performed, and the reaction solution was analyzed by the same method, and quantified with a calibration curve, it is understood to contain 12.35 g (9.53 mmol) of the polymerizable compound (5-1) represented by the aforementioned formula (5-1) (still another example of the compound represented by formula (VI)) which is the target product. The yield was 87.70 mol%.

Furthermore, when the same operation as in the Step 3 of Example 1 was performed, 12.02 g of the polymerizable compound (5-1) (still another example of the compound represented by formula (VI)) was obtained. The isolated yield was 85.37 mol%.

### (Example 13) Synthesis of Polymerizable compound (6-1) (Still another example of the compound represented by formula (VI))

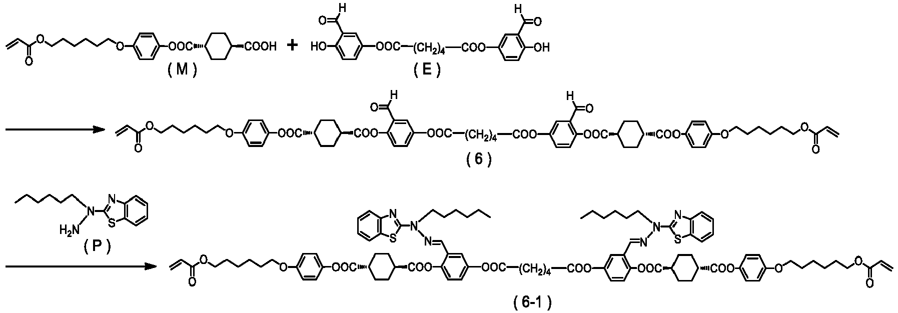

With the exception that 1.50 g (10.86 mmol) of 2,5-dihydroxybenzaldehyde in the Step 1 of Example 1 was replaced with 4.20 g (10.86 mmol) of the compound E (still another example of a compound represented by formula (II)) synthesized in Synthesis Example 4, the same operation as in Example 1 was performed. When the reaction solution was analyzed by the same method, and quantified with a calibration curve, it is understood to contain 12.05 g (10.15 mmol) of the polymerizable compound represented by the aforementioned formula (6) (polymerizable compound (6): still another example of the compound represented by formula (III)). The yield was 93.45 mol%.

With the exception of changing the amount of (14.12 mmol) of the compound P (example of the compound represented by formula (V-I)) added in the Step 2 of Example 1 from 3.52 g to 6.77 g (27.15 mmol), the same operation as in Example 1 was performed. When the reaction solution was analyzed by the same method, and quantified with a calibration curve, it is understood to contain 16.18 g (9.81 mmol) of the polymerizable compound (6-1) represented by the aforementioned formula (6-1) (still another example of the compound represented by formula (VI))which is the target product. The yield was 90.27 mol%.

Furthermore, when the same operation as in the Step 3 of Example 1 was performed, 15.75 g of the polymerizable compound (6-1) (still another example of the compound represented by formula (VI)) was obtained. The isolated yield was 87.87 mol%.

### (Example 14) Synthesis of Polymerizable compound (7-1) (Still another example of the compound represented by formula (VI))

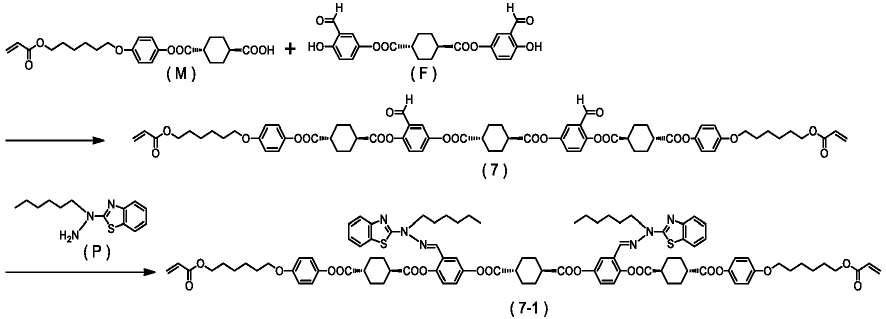

With the exception that 1.50 g (10.86 mmol) of 2,5-dihydroxybenzaldehyde in the Step 1 of Example 1 was replaced with 4.48 g (10.86 mmol) of the compound F (still another example of a compound represented by formula (II)) synthesized in Synthesis Example 5, the same operation as in Example 1 was performed. When the reaction solution was analyzed by the same method, and quantified with a calibration curve, it is understood to contain 12.44 g (10.26 mmol) of the polymerizable compound (7) represented by the aforementioned formula (7) (polymerizable compound (7): still another example of the compound represented by formula (III)). The yield was 94.42 mol%.

With the exception of changing the amount of the compound P (example of the compound represented by formula (V-I)) added in the Step 2 of Example 1 from 3.52 g (14.12 mmol) to 6.77 g (27.15 mmol), the same operation as in Example 1 was performed. When the reaction solution was analyzed by the same method, and quantified with a calibration curve, it is understood to contain 16.60 g (9.91 mmol) of the polymerizable compound (7-1) represented by the aforementioned formula (7-1) (still another example of the compound represented by formula (VI)) which is the target product. The yield was 91.21 mol%.

Furthermore, when the same operation as in the Step 3 of Example 1 was performed, 16.16 g of the polymerizable compound (7-1) was obtained. The isolated yield was 88.78 mol%.

### (Example 15) Synthesis of Polymerizable compound (8-1) (Still another example of the compound represented by formula (VI))

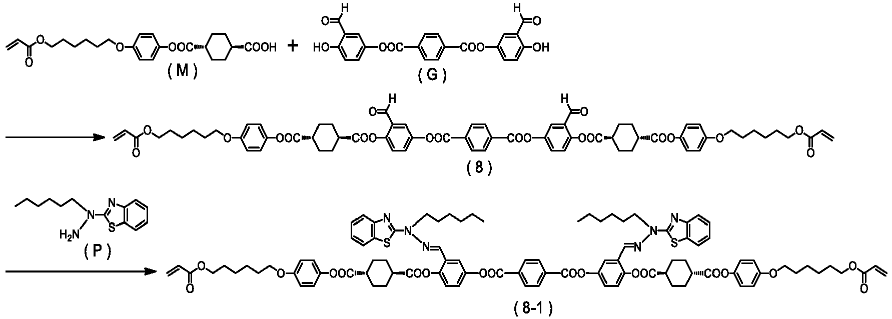

With the exception that 1.50 g (10.86 mmol) of 2,5-dihydroxybenzaldehyde in the Step 1 of Example 1 was replaced with 4.41 g (10.86 mmol) of the compound G (still another example of a compound represented by formula (II)) synthesized in Synthesis Example 6, the same operation as in Example 1 was performed. When the reaction solution was analyzed by the same method, and quantified with a calibration curve, it is understood to contain 12.09 g (10.01 mmol) of the polymerizable compound (8) represented by the aforementioned formulas (8) (polymerizable compound (8): still another example of the compound represented by formula (III)). The yield was 92.19 mol%.

With the exception of changing the amount of the compound P (example of the compound represented by formula (V-I)) added in the Step 2 of Example 1 from 3.52 g (14.12 mmol) to 6.77 g (27.15 mmol), the same operation as in Example 1 was performed. When the reaction solution was analyzed by the same method, and quantified with a calibration curve, it is understood to contain 16.15 g (9.67 mmol) of the polymerizable compound (8-1) represented by the aforementioned formulas (8-1) (still another example of the compound represented by formula (VI)) which is the target product. The yield was 89.06 mol%.

Furthermore, when the same operation as in the Step 3 of Example 1 was performed, 15.72 g of the polymerizable compound (8-1) was obtained. The isolated yield was 86.68 mol%.

### (Example 16) Synthesis of Polymerizable compound (9-1) (Still another example of the compound represented by formula (VI))

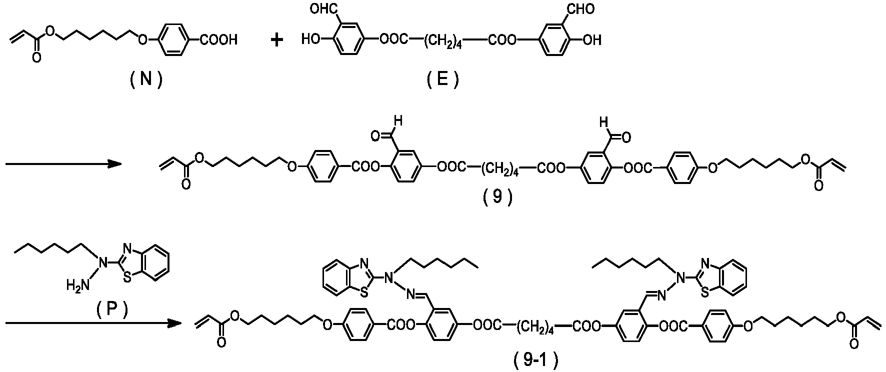

With the exception that 10.00 g (23.90 mmol) of the compound M in the Step 1 of Example 1 was replaced with 6.99 g (23.90 mmol) of 4-(6-acryloylhex-1-yloxy)benzoic acid (manufactured by DKSH Management Ltd.) (another example of a compound represented by formula (I)) represented by the aforementioned formula (N), and 1.50 g (10.86 mmol) of 2,5-dihydroxybenzaldehyde was replaced with 4.20 g (10.86 mmol) of the compound E (still another example of a compound represented by formula (II)) synthesized in Synthesis Example 4, the same operation as in Example 1 was performed. When the reaction solution was analyzed by the same method, and quantified with a calibration curve, it is understood to contain 9.63 g (10.29 mmol) of the polymerizable compound (polymerizable compound (9): still another example of the compound represented by formula (III)) represented by the aforementioned formula (9). The yield was 94.78 mol%.

With the exception of changing the amount of the compound P (example of a compound represented by formula (V-I)) added in the Step 2 of Example 1 from 3.52 g (14.12 mmol) to 6.77 g (27.15 mmol), the same operation as in Example 1 was performed. When the reaction solution was analyzed by the same method, and quantified with a calibration curve, it is understood to contain 13.90 g (9.94 mmol) of the polymerizable compound (9-1) represented by the aforementioned formulas (9-1) which is the target product. The yield was 91.56 mol%.

Furthermore, when the same operation as in the Step 3 of Example 1 was performed, 13.53 g of the polymerizable compound (9-1) was obtained. The isolated yield was 89.12 mol%.

### (Example 17) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 6.98 g (65.17 mmol) of 2,4-lutidine (pKa: 6.99), the same operation as in Example 16 was performed. When the reaction solution was analyzed by the same method, and quantified with a calibration curve, it is understood to contain 9.58 g (10.25 mmol) of the polymerizable compound (polymerizable compound (9): still another example of the compound represented by formula (III)) represented by the aforementioned formula (9). The yield was 94.34 mol%.

When the same operation as in the Step 2 of Example 16 was performed, the reaction solution was analyzed by the same method, and quantified with a calibration curve, it is understood to contain 13.84 g (9.90 mmol) of the polymerizable compound (9-1) represented by the aforementioned formula (9-1) which is the target product. The yield was 91.13 mol%.

Furthermore, when the same operation as in the Step 3 of Example 16 was performed, 13.47 g of the polymerizable compound (9-1) was obtained. The isolated yield was 88.71 mol%.

### (Example 18) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 7.90 g (65.17 mmol) of 2,4,6-collidine (pKa: 7.43), the same operation as in Example 16 was performed. When the reaction solution was analyzed by the same method, and quantified with a calibration curve, it is understood to contain 9.40 g (10.05 mmol) of the polymerizable compound (polymerizable compound (9): still another example of the compound represented by formula (III)) represented by the aforementioned formula (9). The yield was 92.52 mol%.

When the same operation as in the Step 2 of Example 16 was performed, the reaction solution was analyzed by the same method, and quantified with a calibration curve, it is understood to contain 13.57 g (9.71 mmol) of the polymerizable compound (9-1) represented by the aforementioned formula (9-1) which is which is the target product. The yield was 89.38 mol%.

Furthermore, when the same operation as in the Step 3 of Example 16 was performed, 13.21 g of the polymerizable compound (9-1) was obtained. The isolated yield was 86.99 mol%.

### (Example 19) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 6.98 g (65.17 mmol) of 3,5-lutidine (pKa: 6.15), the same operation as in Example 16 was performed. When the reaction solution was analyzed by the same method, and quantified with a calibration curve, it is understood to contain 9.47 g (10.13 mmol) of the polymerizable compound (polymerizable compound (9): still another example of the compound represented by formula (III)) represented by the aforementioned formula (9). The yield was 93.26 mol%.

When the same operation as in the Step 2 of Example 16 was performed, the reaction solution was analyzed by the same method, and quantified with a calibration curve, it is understood to contain 13.68 g (9.79 mmol) of the polymerizable compound (9-1) represented by the aforementioned formula (9-1) which is the target product. The yield was 90.09 mol%.

Furthermore, when the same operation as in the Step 3 of Example 16 was performed, 13.31 g of the polymerizable compound (9-1) was obtained. The isolated yield was 87.69 mol%.

### (Example 20) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 6.98 g (65.17 mmol) of 3,4-lutidine (pKa:6.46), the same operation as in Example 16 was performed. When the reaction solution was analyzed by the same method, and quantified with a calibration curve, it is understood to contain 9.32 g (9.97 mmol) of the polymerizable compound (polymerizable compound (9): still another example of the compound represented by formula (III)) represented by the aforementioned formula (9). The yield was 91.79 mol%.

When the same operation as in the Step 2 of Example 16 was performed, the reaction solution was analyzed by the same method, and quantified with a calibration curve, it is understood to contain 13.46 g (9.63 mmol) of the polymerizable compound (9-1) represented by the aforementioned formula (9-1) which is the target product. The yield was 88.67 mol%.

Furthermore, when the same operation as in the Step 3 of Example 16 was performed, 13.10 g of the polymerizable compound (9-1) was obtained. The isolated yield was 86.31 mol%.

### (Example 21) Synthesis of Polymerizable compound (10-1) (Still another example of the compound represented by formula (VI))

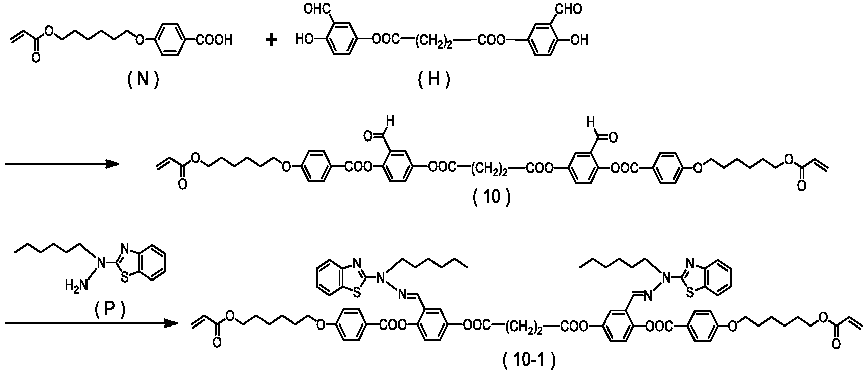

With the exception that 4.20 g (10.86 mmol) of the compound E (still another example of a compound represented by formula (II)) synthesized in Synthesis Example 4 in the Step 1 of Example 16 was replaced with 3.89 g (10.86 mmol) of Compound H (still another example of a compound represented by formula (II)) synthesized in Synthesis Example 7, the same operation as in Example 16 was performed. When the reaction solution was analyzed by the same method, and quantified with a calibration curve, it is understood to contain 9.28 g (10.24 mmol) of the polymerizable compound (polymerizable compound (10): still another example of the compound represented by formula (III)) represented by the aforementioned formula (10). The yield was 94.25 mol%.

When the same operation as in the Step 2 of Example 16 was performed, the reaction solution was analyzed by the same method, and quantified with a calibration curve, it is understood to contain 13.54 g (9.89 mmol) of the polymerizable compound (10-1) represented by the aforementioned formula (10-1) which is the target product. The yield was 91.05 mol%.

Furthermore, when the same operation as in the Step 3 of Example 16 was performed, 13.18 g of the polymerizable compound (10-1) was obtained. The isolated yield was 88.62 mol%.

### (Example 22) Synthesis of Polymerizable compound (11-1) (Still another example of the compound represented by formula (VI))

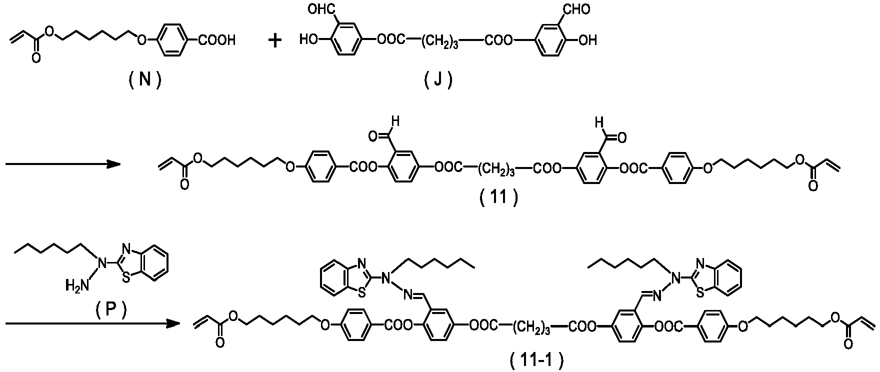

With the exception that 4.20 g (10.86 mmol) of the compound E (still another example of a compound represented by formula (II)) synthesized in Synthesis Example 4 in the Step 1 of Example 16 was replaced with 4.04 g (10.86 mmol) of Compound J (still another example of a compound represented by formula (II)) synthesized in Synthesis Example 8, the same operation as in Example 16 was performed. When the reaction solution was analyzed by the same method, and quantified with a calibration curve, it is understood to contain 9.40 g (10.21 mmol) of the polymerizable compound (polymerizable compound (11): still another example of the compound represented by formula (III)) represented by the aforementioned formula (11). The yield was 93.96 mol%.

When the same operation as in the Step 2 of Example 16 was performed, the reaction solution was analyzed by the same method, and quantified with a calibration curve, it is understood to contain 13.64 g (9.86 mmol) of the polymerizable compound (11-1) represented by the aforementioned formula (11-1) which is the target product. The yield was 90.77 mol%.

Furthermore, when the same operation as in the Step 3 of Example 16 was performed, 13.28 g of the polymerizable compound (11-1) was obtained. The isolated yield was 88.35 mol%.

### (Example 23) Synthesis of Polymerizable compound (12-1) (Still another example of the compound represented by formula (VI))

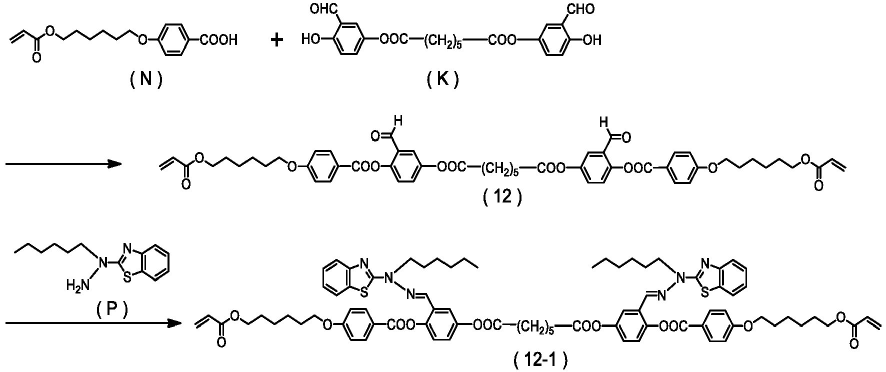

With the exception that 4.20 g (10.86 mmol) of the compound E (still another example of a compound represented by formula (II)) synthesized in Synthesis Example 4 in the Step 1 of Example 16 was replaced with 4.35 g (10.86 mmol) of the compound K (still another example of a compound represented by formula (II)) synthesized in Synthesis Example 9, the same operation as in Example 16 was performed. When the reaction solution was analyzed by the same method, and quantified with a calibration curve, it is understood to contain 9.79 g (10.31 mmol) of the polymerizable compound (polymerizable compound (12): still another example of the compound represented by formula (III)) represented by the aforementioned formula (12). The yield was 94.94 mol%.

When the same operation as in the Step 2 of Example 16 was performed, the reaction solution was analyzed by the same method, and quantified with a calibration curve, it is understood to contain 14.06 g (9.96 mmol) of the polymerizable compound (12-1) represented by the aforementioned formula (12-1) which is the target product. The yield was 91.71 mol%.

Furthermore, when the same operation as in the Step 3 of Example 16 was performed, 13.69 g of the polymerizable compound (12-1) was obtained. The isolated yield was 89.27 mol%.

### (Example 24) Synthesis of Polymerizable compound (13-1) (Still another example of the compound represented by formula (VI))

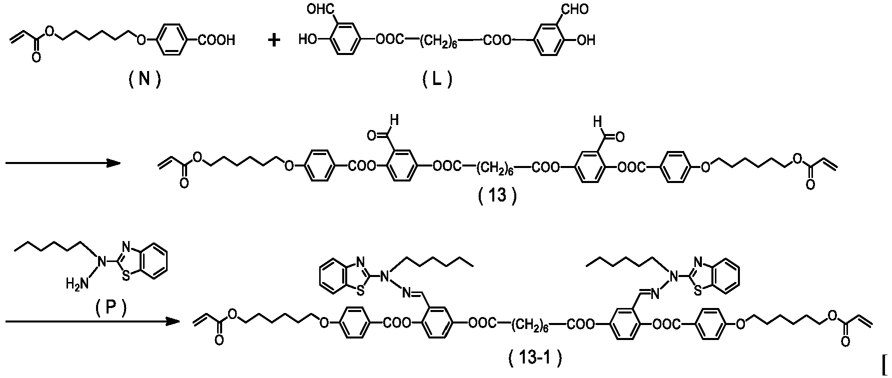

With the exception that 4.20 g (10.86 mmol) of the compound E (still another example of a compound represented by formula (II)) synthesized in Synthesis Example 4 in the Step 1 of Example 16 was replaced with 4.50 g (10.86 mmol) of the compound L (still another example of a compound represented by formula (II)) synthesized in Synthesis Example 10, the same operation as in Example 16 was performed. When the reaction solution was analyzed by the same method, and quantified with a calibration curve, it is understood to contain 9.68 g (10.06 mmol) of the polymerizable compound (polymerizable compound (13): still another example of the compound represented by formula (III)) represented by the aforementioned formula (13). The yield was 92.58 mol%.

When the same operation as in the Step 2 of Example 16 was performed, the reaction solution was analyzed by the same method, and quantified with a calibration curve, it is understood to contain 13.85 g (9.71 mmol) of the polymerizable compound (13-1) which is the target product represented by the aforementioned formula (13-1). The yield was 89.43 mol%.

Furthermore, when the same operation as in the Step 3 of Example 16 was performed, 13.48 g of the polymerizable compound (13-1) was obtained. The isolated yield was 87.05 mol%.

### (Example 25) Synthesis of Polymerizable compound (14-1) (Still another example of the compound represented by formula (VI))

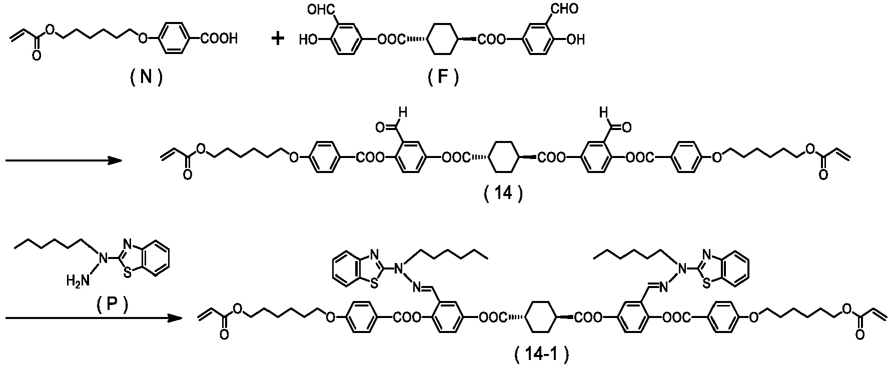

With the exception that 4.20 g (10.86 mmol) of the compound E (still another example of a compound represented by formula (II)) synthesized in Synthesis Example 4 in the Step 1 of Example 16 was replaced with 4.48 g (10.86 mmol) of the compound F (still another example of a compound represented by formula (II)) in Synthesis Example 5, the same operation as in Example 16 was performed. When the reaction solution was analyzed by the same method, and quantified with a calibration curve, it is understood to contain 9.74 g (10.14 mmol) of the polymerizable compound (polymerizable compound (14): still another example of the compound represented by formula (III)) represented by the aforementioned formula (14). The yield was 93.35 mol%.

When the same operation as in the Step 2 of Example 16 was performed, the reaction solution was analyzed by the same method, and quantified with a calibration curve, it is understood to contain 13.95 g (9.79 mmol) of the polymerizable compound (14-1) which is the target product represented by the aforementioned formula (14-1). The yield was 90.18 mol%.

Furthermore, when the same operation as in the Step 3 of Example 16 was performed, 13.57 g of the polymerizable compound (14-1) was obtained. The isolated yield was 87.77 mol%.

### (Example 26) Synthesis of Polymerizable compound (15-1) (Still another example of the compound represented by formula (VI))

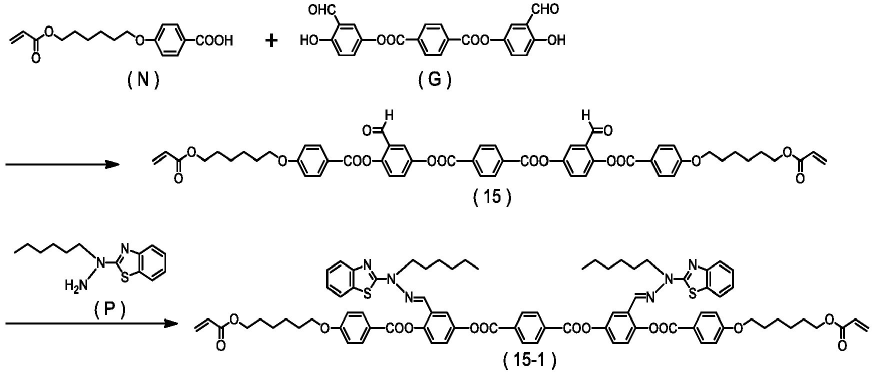

With the exception that 4.20 g (10.86 mmol) of the compound E (Still another example of a compound represented by formula (II)) in Synthesis Example 4 in the Step 1 of Example 16 was replaced with 4.41 g (10.86 mmol) of the compound G (still another example of a compound represented by formula (II)) synthesized in Synthesis Example 6, the same operation as in Example 16 was performed. When the reaction solution was analyzed by the same method, and quantified with a calibration curve, it is understood to contain 9.51 g (9.96 mmol) of the polymerizable compound (polymerizable compound (15): still another example of the compound represented by formula (III)) represented by the aforementioned formula (15). The yield was 91.71 mol%.

When the same operation as in the Step 2 of Example 16 was performed, the reaction solution was analyzed by the same method, and quantified with a calibration curve, it is understood to contain 13.64 g (9.62 mmol) of the polymerizable compound (15-1) which is the target product represented by the aforementioned formula (15-1). The yield was 88.59 mol%.

Furthermore, when the same operation as in the Step 3 of Example 16 was performed, 13.28 g of the polymerizable compound (15-1) was obtained. The isolated yield was 86.23 mol%.

### (Example 27) Synthesis of Polymerizable compound (16-1) (Still another example of the compound represented by formula (VI))

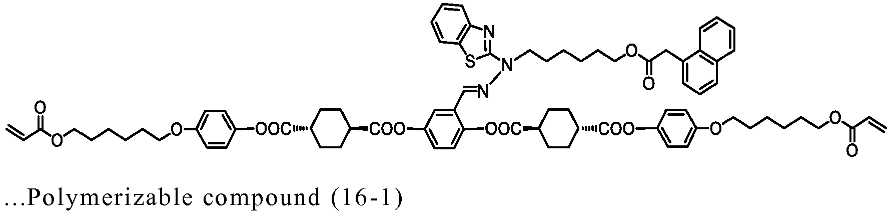

### <Step 1: Synthesis of Intermediate f>

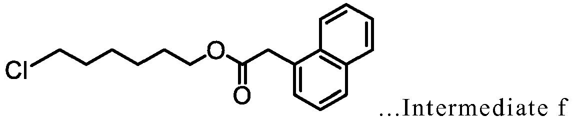

50 g (268.5 mmol) of 1-naphthylacetic acid was added to 110 g of toluene in a three-necked reactor equipped with a thermometer, under a nitrogen stream. Furthermore, 34.8 g (255 mmol) of 6-chloro-1-hexanol, 4.09 g (21.5 mmol) of p-toluenesulfonic acid mono hydrate were added to prepare a solution. A Dean Stark apparatus was used to heat the prepared solution and azeotropic dehydration (internal temperature approximately 95°C) was performed for 5 hours while discharging the generated water out of the reaction system. After completion of the reaction, 75 g of a 6 wt% sodium bicarbonate water was added to the reaction solution cooled to 25°C to separate and wash. After the separation, the organic layer was further washed with 80 g of water. After the washing, the organic layer was filtered off. The solvent was removed from the organic layer using a rotary evaporator to obtain 75 g of a light brown oil containing Intermediate f. Purification of the light brown oil was not performed, and the light brown oil was used in the following reaction (Step 2: Synthesis of Compound S) as is. The structure of Intermediate f was identified by ¹H-NMR. The ¹H-NMR spectral data is shown below.
¹H-NMR (500 MHz, CDCl₃, TMS, δppm): 8.00 (dd,1H,J = 1.0 Hz, 8.5 Hz), 7.86 (dd,1H,J = 1.5 Hz,8.5 Hz), 7.79 (dd,1H,J = 1.5 Hz,7.5 Hz), 7.54-7.47 (m,2H), 7.45-7.41 (m,2H), 4.09-4.06 (m,4H), 3.43 (t,2H,J = 7.0 Hz), 1.67-1.61 (m,2H), 1.58-1.53 (m,2H), 1.35-1.29 (m,2H), 1.22-1.15 (m,2H).

### <Step 2: Synthesis of Compound S>

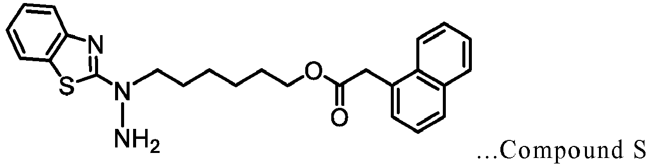

6.00 g (36.32 mmol) of 2-hydrazinobenzothiazole was dissolved in 65 ml of N-N-dimethylformamide in a three-necked reactor equipped with a thermometer under a nitrogen stream. 23.67 g (72.63 mmol) of cesium carbonate and 20 g of brown oil containing Intermediate f synthesized in the Step 1 were added to the solution, and the solution was stirred at 25°C for 15 hours. After completion of the reaction, the reaction solution was charged with 250 ml of distilled water, followed by extraction twice with 250 ml of ethyl acetate. After drying the ethyl acetate layer with anhydrous sodium sulfate, the sodium sulfate was filtered off. The organic layer was collected, and dried with anhydrous sodium sulfate, and the sodium sulfate was filtered off. After the solvent was evaporated from the filtrate using a rotary evaporator, the obtained residue was purified by silica gel column chromatography (hexane:THF = 80:20 (volume ratio)) to obtain 8.0 g of the compound S was obtained as a white solid. The yield was 51.0 mol%. The structure of the compound S was identified by ¹H-NMR. The ¹H-NMR spectral data is shown below.
¹H-NMR (500 MHz, CDCl₃, TMS, δppm):8.00 (d,1H,J = 8.5 Hz), 7.85 (dd,1H,J = 1.0 Hz,8.0 Hz), 7.78 (dd,1H,J = 1.5 Hz, 7.5 Hz), 7.60 (dd,1H,J = 1.0 Hz,7.5 Hz), 7.54-7.51 (m,2H), 7.49-7.40 (m,3H), 7.28 (ddd,1H,J = 1.0 Hz, 7.5 Hz,7.5 Hz), 7.07 (ddd,1H,J = 1.0 Hz,7.5 Hz,7.5 Hz), 4.16 (br,2H), 4.08 (t,2H,J = 6.5 Hz), 4.06 (s,2H), 3.66 (t,2H,J = 7.0 Hz), 1.63-1.54 (m,4H), 1.32-1.22 (m,4H).

### <Step 3: Synthesis of Polymerizable compound 16-1 (Still another example of the compound represented by formula (VI))>

When the same operation as in the Step 1 of Example 6 was performed, and the reaction solution was analyzed by the same method, it is understood to contain 9.68 g (10.31 mmol) of the polymerizable compound (1) (example of a compound represented by formula (III) which is the target product (it is understood that the solution containing the compound represented by formula (III) and the compound represented by formula (XII) could be obtained). The yield was 94.88 mol%.

With the exception that 3.52 g (14.12 mmol) of the compound P (example of a compound represented by formula (V-I)) in the Step 2 of Example 6 was replaced with 5.66 g (13.05 mmol) of the compound S synthesized in the aforementioned Step 2, the same operation as in Example 16 was performed, and when the reaction solution was analyzed by the same method, and quantified with a calibration curve, it is understood to contain 13.46 g (9.94 mmol) of the aforementioned formulas (16-1) (still another example of the compound represented by formula (VI)) which is the target product (it is understood that the solution containing the compound represented by formula (VI) and the compound represented by formula (XII) could be obtained). The yield was 91.50 mol%.

After completion of the reaction, the reaction solution was cooled to 25°C, and the liquid separation operation was performed.

After adding 0.50 g of ROKAHELP #479 (manufactured by Mitsui Mining and Smelting Co., Ltd.) to the obtained organic layer and stirring for 30 minutes, the ROKAHELP #479 was filtered off. Next, approximately 60% of the total amount was evaporated and concentrated from the obtained reaction solution using an evaporator. After 18 g of THF was added to the solution, the solution was cooled to 15°C and stirred for 30 minutes. Next, 70 g of n-hexane was dropped in the solution at 15°C to precipitate the crystals. Then, the precipitated crystals were filtered off by filtration.

After 72 g of THF, 1.2 g of ROKAHELP #479, and 100 mg of 2,6-di-t-butyl-4-methylphenol were added to the obtained crystals and stirred for 30 minutes, the ROKAHELP #479 was filtered off. Next, 36 g of THF was evaporated from the obtained reaction solution using an evaporator. After 66 g of methanol was dropped in the obtained solution, the solution was cooled to 0°C to precipitate the crystals. Then, the precipitated crystals were filtered off by filtration. After the filtered material was washed with methanol and dried under a vacuum, 13.24 g of the polymerizable compound (16-1) (still another example of the compound represented by formula (VI)) was obtained (Step 3). The isolated yield was 90.01 mol%.

### (Comparative Example 1) Synthesis of Polymerizable compound (1-1) (Example of a compound represented by formula (VI))

With the exception that 6.98 g (65.17 mmol) of 2,6-lutidine (pKa: 6.65) which is the base in the Step 1 of Example 1 was replaced with 6.59 g (65.17 mmol) of triethylamine (pKa: 10.75), the same operation as in Example 1 was performed. When the reaction solution was analyzed by the same method, and quantified with a calibration curve, it is understood to contain 8.82 g (9.39 mmol) of the polymerizable compound (1) (example of a compound represented by formula (III)). The yield was 86.45 mol%.

When the same operation as in the Step 2 of Example 1 was performed, the reaction solution was analyzed by the same method, and quantified with a calibration curve, it is understood to contain 10.76 g (9.19 mmol) of the polymerizable compound (1-1) which is the target product. The yield was 84.62 mol%.

Furthermore, when the same operation as in the Step 3 of Example 1 was performed, 10.47 g of the polymerizable compound (1-1) was obtained. The isolated yield was 82.37 mol%.

### (Comparative Example 2) Synthesis of Polymerizable compound (1-1) (Example of a compound represented by formula (VI))

With the exception that 6.98 g (65.17 mmol) of 2,6-lutidine (pKa: 6.65) which is the base in the Step 1 of Example 1 was replaced with 8.42 g (65.17 mmol) of N-N-diisopropylethylamine (pKa: 10.98), the same operation as in Example 1 was performed. When the reaction solution was analyzed by the same method, and quantified with a calibration curve, it is understood to contain 8.69 g (9.26 mmol) of the polymerizable compound (1) (example of a compound represented by formula (III)). The yield was 85.21 mol%.

When the same operation as in the Step 2 of Example 1 was performed, the reaction solution was analyzed by the same method, and quantified with a calibration curve, it is understood to contain 10.41 g (8.89 mmol) of the polymerizable compound (1-1) which is the target product. The yield was 81.86 mol%.

Furthermore, when the same operation as in the Step 3 of Example 1 was performed, 10.13 g of the polymerizable compound (1-1) was obtained. The isolated yield was 79.68 mol%.

### (Comparative Example 3) Synthesis of Polymerizable compound (1-1) (Example of a compound represented by formula (VI))

With the exception that 6.98 g (65.17 mmol) of 2,6-lutidine (pKa: 6.65) which is the base in the Step 1 of Example 1 was replaced with 6.07 g (65.17 mmol) of 2-methylpyridine (pKa: 6.00), the same operation as in Example 1 was performed. When the reaction solution was analyzed by the same method, and quantified with a calibration curve, it is understood to contain 4.04 g (4.30 mmol) of the polymerizable compound (1) (example of a compound represented by formula (III)). The yield was 39.58 mol%.

When the same operation as in the Step 2 of Example 1 was performed, the reaction solution was analyzed by the same method, and quantified with a calibration curve, it is understood to contain 4.53 g (3.87 mmol) of the polymerizable compound (1-1) which is the target product. The yield was 35.62 mol%.

Furthermore, when the same operation as in the Step 3 of Example 1 was performed, 4.41 g of the polymerizable compound (1-1) was obtained. The isolated yield was 34.67 mol%.

### (Comparative Example 4) Synthesis of Polymerizable compound (1-1) (Example of a compound represented by formula (VI))

With the exception that 6.98 g (65.17 mmol) of 2,6-lutidine (pKa: 6.65) which is the base in the Step 1 of Example 1 was replaced with 5.16 g (65.17 mmol) of pyridine (pKa: 5.23), the same operation as in Example 1 was performed. When the reaction solution was analyzed by the same method, and quantified with a calibration curve, it is understood to contain 8.23 g (8.77 mmol) of the Polymerizable compound (1) (example of the compound represented by formula (III)). The yield was 80.73 mol%.

When the same operation as in the Step 2 of Example 1 was performed, and the reaction solution was analyzed by the same method, and quantified with a calibration curve, it is understood to contain 9.82 g (8.39 mmol) of the polymerizable compound (1-1) which is the target product. The yield was 77.21 mol%.

Furthermore, when the same operation as in the Step 3 of Example 1 was performed, 9.55 g of the polymerizable compound (1-1) was obtained. The isolated yield was 75.15 mol%.

### (Comparative Example 5) Synthesis of Polymerizable compound (1-1) (Example of a compound represented by formula (VI))

With the exception that 6.98 g (65.17 mmol) of 2,6-lutidine (pKa: 6.65) which is the base in the Step 1 of Example 1 was replaced with 8.42 g (65.17 mmol) of quinoline (pKa: 4.93), the same operation as in Example 1 was performed. When the reaction solution was analyzed by the same method, and quantified with a calibration curve, it is understood to contain 8.49 g (9.04 mmol) of the polymerizable compound (1) (example of a compound represented by formula (III)). The yield was 83.21 mol%.

When the same operation as in the Step 2 of Example 1 was performed, and the reaction solution was analyzed by the same method, and quantified with a calibration curve, it is understood to contain 10.19 g (8.71 mmol) of the polymerizable compound (1-1) which is the target product. The yield was 80.17 mol%.

Furthermore, when the same operation as in the Step 3 of Example 1 was performed, 9.92 g of the polymerizable compound (1-1) was obtained. The isolated yield was 78.03 mol%.

### (Comparative Example 6) Synthesis of Polymerizable compound (1-1) (Example of a compound represented by formula (VI))

With the exception that 6.98 g (65.17 mmol) of 2,6-lutidine (pKa: 6.65) which is the base in the Step 1 of Example 1 was replaced with 9.92 g (65.17 mmol) of diazabicycloundecene (pKa: 13.20), the same operation as in Example 1 was performed. When the reaction solution was analyzed by the same method, and quantified with a calibration curve, it is understood to contain 0.19 g (0.20 mmol) of the polymerizable compound (1) (example of a compound represented by formula (III)). The yield was 1.86 mol%.

When the same operation as in the Step 2 of Example 1 was performed, and the reaction solution was analyzed by the same method, it is understood to contain 0.15 g (0.13 mmol) of the polymerizable compound (1-1) which is the target product. The yield was 1.16 mol%.

Furthermore, the same operation as in the Step 3 of Example 1 was performed, but the polymerizable compound (1-1) could not be isolated.

### (Comparative Example 7) Synthesis of Polymerizable compound (1-1) (Example of a compound represented by formula (VI))

With the exception that 6.98 g (65.17 mmol) of 2,6-lutidine (pKa: 6.65) which is the base in the Step 1 of Example 1 was replaced with 8.09 g (65.17 mmol) of diazabicyclononane (pKa: 13.40), the same operation as in Example 1 was performed. When the reaction solution was analyzed by the same method, and quantified with a calibration curve, it is understood to contain 0.22 g (0.24 mmol) of the polymerizable compound (1) (example of a compound represented by formula (III)). The yield was 2.19 mol%.

When the same operation as in the Step 2 of Example 1 was performed, and the reaction solution was analyzed by the same method, it is understood to contain 0.24 g (0.20 mmol) of the polymerizable compound (1-1) which is the target product. The yield was 1.85 mol%.

Furthermore, the same operation as in the Step 3 of Example 1 was performed, but the polymerizable compound (1-1) could not be isolated.

### (Comparative Example 8) Synthesis of Polymerizable compound (1-1) (Example of a compound represented by formula (VI))

With the exception that 6.98 g (65.17 mmol) of 2,6-lutidine (pKa: 6.65) which is the base in the Step 1 of Example 1 was replaced with 7.96 g (65.17 mmol) of 4-dimethyl aminopyridine (pKa: 9.52), the same operation as in Example 1 was performed. When the reaction solution was analyzed by the same method, and quantified with a calibration curve, it is understood to contain 8.06 g (8.58 mmol) of the polymerizable compound (1) which is the target product (example of a compound represented by formula (III)). The yield was 79.01 mol%.

When the same operation as in the Step 2 of Example 1 was performed and the reaction solution was analyzed by the same method, and quantified with a calibration curve, it is understood to contain 9.66 g (8.25 mmol) of the polymerizable compound (1-1) which is the target product (example of a compound represented by formula (VI)). The yield was 75.99 mol%.

Furthermore, when the same operation as in the Step 3 of Example 1 was performed, 9.18 g of the polymerizable compound (1-1) (example of a compound represented by formula (VI)) was obtained. The isolated yield was 72.18 mol%.

### (Comparative Example 9) Synthesis of Polymerizable compound (1-1) (Example of a compound represented by formula (VI))

With the exception that 6.98 g (65.17 mmol) of 2,6-lutidine (pKa: 6.65) which is the base in the Step 1 of Example 6 was replaced with 6.59 g (65.17 mmol) of triethylamine (pKa: 10.75), the same operation as is Example 6 was performed. When the reaction solution was analyzed by the same method, and quantified with a calibration curve, it is understood to contain 8.67 g (9.23 mmol) of the polymerizable compound (1) (example of a compound represented by formula (III)). The yield was 85.02 mol%.

When the same operation as in the Step 2 of Example 6 was performed and the reaction solution was analyzed by the same method, it is understood to contain 10.49 g (8.97 mmol) of the polymerizable compound (1-1) which is the target product. The yield was 82.54 mol%.

Furthermore, when the same operation as in the Step 3 of Example 6 was performed, 10.21 g of the polymerizable compound (1-1) was obtained. The isolated yield was 80.34 mol%.

### (Comparative Example 10) Synthesis of Polymerizable compound (1-1) (example of a compound represented by formula (VI))

With the exception that 6.98 g (65.17 mmol) of 2,6-lutidine (pKa: 6.65) which is the base in the Step 1 of Example 7 was replaced with 6.59 g (65.17 mmol) of triethylamine (pKa: 10.75), the same operation as in Example 7 was performed. When the reaction solution was analyzed by the same method, and quantified with a calibration curve, it is understood to contain 8.82 g (9.39 mmol) of the polymerizable compound (1) (example of a compound represented by formula (III)). The yield was 86.45 mol%.

When the same operation as in the Step 2 of Example 7 was performed and the reaction solution was analyzed by the same method, it is understood to contain 10.32 g (8.82 mmol) of the polymerizable compound (1-1) which is the target product. The yield was 81.21 mol%.

Furthermore, when the same operation as in the Step 3 of Example 7 was performed, 10.05 g of the polymerizable compound (1-1) was obtained. The isolated yield was 79.05 mol%.

### (Comparative Example 11) Synthesis of Polymerizable compound (2-1) (Another example of a compound represented by formula (V-I))

With the exception that 6.98 g (65.17 mmol) of 2,6-lutidine (pKa: 6.65) which is the base in the Step 1 of Example 8 was replaced with 6.59 g (65.17 mmol) of triethylamine (pKa: 10.75), the same operation as in Example 8 was performed. When the reaction solution was analyzed by the same method, and quantified with a calibration curve, it is understood to contain 6.47 g (9.42 mmol) of the polymerizable compound (2) (another example of the compound represented by formula (III)). The yield was 86.72 mol%.

When the same operation as in the Step 2 of Example 8 was performed and the reaction solution was analyzed by the same method, it is understood to contain 7.59 g (9.10 mmol) of the polymerizable compound (2-1) which is the target product. The yield was 83.77 mol%.

Furthermore, when the same operation as in the Step 3 of Example 8 was performed, 7.39 g of the polymerizable compound (2-1) was obtained. The isolated yield was 81.54 mol%.

### (Comparative Example 12) Synthesis of Polymerizable compound (3-1) (Still another example of the compound represented by formula (VI))

With the exception that 6.98 g (65.17 mmol) of 2,6-lutidine (pKa: 6.65) which is the base in the Step 1 of Example 9 was replaced with 6.59 g (65.17 mmol) of triethylamine (pKa: 10.75), the same operation as in Example 9 was performed. When the reaction solution was analyzed by the same method, and quantified with a calibration curve, it is understood to contain 6.81 g (9.25 mmol) of the polymerizable compound (3) (still another example of the compound represented by formula (III)). The yield was 85.14 mol%.

When the same operation as in the Step 2 of Example 9 was performed and the reaction solution was analyzed by the same method, it is understood to contain 8.65 g (8.93 mmol) of the polymerizable compound (3-1) which is the target product. The yield was 82.25 mol%.

Furthermore, when the same operation as in the Step 3 of Example 9 was performed, 8.42 g of the polymerizable compound (3-1) was obtained. The isolated yield was 80.06 mol%.

### (Comparative Example 13) Synthesis of Polymerizable compound (4-1) (Still another example of the compound represented by formula (VI))

With the exception that 6.98 g (65.17 mmol) of 2,6-lutidine (pKa: 6.65) which is the base in the Step 1 of Example 10 was replaced with 6.59 g (65.17 mmol) of triethylamine (pKa: 10.75), the same operation as in Example 10 was performed. When the reaction solution was analyzed by the same method, and quantified with a calibration curve, it is understood to contain 9.80 g (9.25 mmol) of the polymerizable compound (4) (still another example of the compound represented by formula (III)). The yield was 85.15 mol%.

When the same operation as in the Step 2 of Example 10 was performed and the reaction solution was analyzed by the same method, it is understood to contain 11.39 g (8.83 mmol) the polymerizable compound (4-1) which is the target product. The yield was 81.26 mol%.

Furthermore, when the same operation as in the Step 3 of Example 10 was performed, 11.20 g of the polymerizable compound (4-1) was obtained. The isolated yield was 79.88 mol%.

### (Comparative Example 14) Synthesis of Polymerizable compound (4-2) (Still another example of the compound represented by formula (VI))

With the exception that 6.98 g (65.17 mmol) of 2,6-lutidine (pKa: 6.65) which is the base in the Step 1 of Example 11 was replaced with 6.59 g (65.17 mmol) of triethylamine (pKa: 10.75), the same operation as in Example 11 was performed. When the reaction solution was analyzed by the same method, and quantified with a calibration curve, it is understood to contain 9.80 g (9.25 mmol) of the polymerizable compound (4) (still another example of the compound represented by formula (III)). The yield was 85.15 mol%.

When the same operation as in the Step 2 of Example 11 was performed and the reaction solution was analyzed by the same method, it is understood to contain 11.62 g (8.80 mmol) of the polymerizable compound (4-2) which is the target product. The yield was 80.98 mol%.

Furthermore, when the same operation as in the Step 3 of Example 11 was performed, 11.14 g of the polymerizable compound (4-2) was obtained. The isolated yield was 77.69 mol%.

### (Comparative Example 15) Synthesis of Polymerizable compound (5-1) (Still another example of the compound represented by formula (VI))

With the exception that 6.98 g (65.17 mmol) of 2,6-lutidine (pKa: 6.65) which is the base in the Step 1 of Example 12 was replaced with 6.59 g (65.17 mmol) of triethylamine (pKa: 10.75), the same operation as in Example 12 was performed. When the reaction solution was analyzed by the same method, and quantified with a calibration curve, it is understood to contain 9.82 g (9.22 mmol) of the polymerizable compound (5) (still another example of the compound represented by formula (III)). The yield was 84.89 mol%.

When the same operation as in the Step 2 of Example 12 was performed and the reaction solution was analyzed by the same method, it is understood to contain 11.55 g (8.91 mmol) of the polymerizable compound (5-1) which is the target product. The yield was 82.00 mol%.

Furthermore, when the same operation as in the Step 3 of Example 12 was performed, 11.24 g of the polymerizable compound (5-1) was obtained. The isolated yield was 79.82 mol%.

### (Comparative Example 16) Synthesis of Polymerizable compound (6-1) (Still another example of the compound represented by formula (VI))

With the exception that 6.98 g (65.17 mmol) of 2,6-lutidine (pKa: 6.65) which is the base in the Step 1 of Example 13 was replaced with 6.59 g (65.17 mmol) of triethylamine (pKa: 10.75), the same operation as in Example 13 was performed. When the reaction solution was analyzed by the same method, and quantified with a calibration curve, it is understood to contain 11.09 g (9.34 mmol) of the polymerizable compound (6) (still another example of the compound represented by formula (III)). The yield was 86.01 mol%.

When the same operation as in the Step 2 of Example 13 was performed and the reaction solution was analyzed by the same method, it is understood to contain 14.89 g (9.02 mmol) of the polymerizable compound (6-1) which is the target product. The yield was 83.09 mol%.

Furthermore, when the same operation as in the Step 3 of Example 13 was performed, 14.49 g of the polymerizable compound (6-1) was obtained. The isolated yield was 80.87 mol%.

### (Comparative Example 17) Synthesis of Polymerizable compound (7-1) (Still another example of the compound represented by formula (VI))

With the exception that 6.98 g (65.17 mmol) of 2,6-lutidine (pKa: 6.65) which is the base in the Step 1 of Example 14 was replaced with 6.59 g (65.17 mmol) of triethylamine (pKa: 10.75), the same operation as in Example 14 was performed. When the reaction solution was analyzed by the same method, and quantified with a calibration curve, it is understood to contain 11.29 g (9.31 mmol) of the polymerizable compound (7) (still another example of the compound represented by formula (III)). The yield was 85.67 mol%.

When the same operation as in the Step 2 of Example 14 was performed and the reaction solution was analyzed by the same method, it is understood to contain 15.42 g (9.20 mmol) of the polymerizable compound (7-1) which is the target product. The yield was 84.69 mol%.

Furthermore, when the same operation as in the Step 3 of Example 14 was performed, 15.01 g of the polymerizable compound (7-1) was obtained. The isolated yield was 82.43 mol%.

### (Comparative Example 18) Synthesis of Polymerizable compound (8-1) (Still another example of the compound represented by formula (VI))

With the exception that 6.98 g (65.17 mmol) of 2,6-lutidine (pKa: 6.65) which is the base in the Step 1 of Example 15 was replaced with 6.59 g (65.17 mmol) of triethylamine (pKa: 10.75), the same operation as in Example 15 performed. When the reaction solution was analyzed by the same method, and quantified with a calibration curve, it is understood to contain 11.41 g (9.45 mmol) of the polymerizable compound (8) (still another example of the compound represented by formula (III)). The yield was 86.99 mol%.

When the same operation as in the Step 2 of Example 15 was performed and the reaction solution was analyzed by the same method, it is understood to contain 15.24 g (9.13 mmol) of the polymerizable compound (8-1) which is the target product. The yield was 84.03 mol%.

Furthermore, when the same operation as in the Step 3 of Example 15 was performed, 14.84 g of the polymerizable compound (8-1) was obtained. The isolated yield was 81.79 mol%.

### (Comparative Example 19) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 6.59 g (65.17 mmol) of triethylamine (pKa: 10.75), the same operation as in Example 16 was performed. When the reaction solution was analyzed by the same method, and quantified with a calibration curve, it is understood to contain 8.75 g (9.35 mmol) of the polymerizable compound (9) (still another example of the compound represented by formula (III)). The yield was 86.12 mol%.

When the same operation as in the Step 2 of Example 16 was performed and the reaction solution was analyzed by the same method, it is understood to contain 12.63 g (9.04 mmol) of the polymerizable compound (9-1) which is the target product. The yield was 83.19 mol%.

Furthermore, when the same operation as in the Step 3 of Example 16 was performed, 12.29 g of the polymerizable compound (9-1) was obtained. The isolated yield was 80.98 mol%.

### (Comparative Example 20) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 8.42 g (65.17 mmol) of N-N-diisopropylethylamine (pKa: 10.98), the same operation as in Example 16 was performed. When the reaction solution was analyzed by the same method, and quantified with a calibration curve, it is understood to contain 8.59 g (9.18 mmol) of the polymerizable compound (9) (still another example of the compound represented by formula (III)). The yield was 84.56 mol%.

When the same operation as in the Step 2 of Example 16 was performed and the reaction solution was analyzed by the same method, it is understood to contain 12.40 g (8.87 mmol) of the polymerizable compound (9-1) which is the target product. The yield was 81.69 mol%.

Furthermore, when the same operation as in the Step 3 of Example 16 was performed, 12.07 g of the polymerizable compound (9-1) was obtained. The isolated yield was 79.51 mol%.

### (Comparative Example 21) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 6.07 g (65.17 mmol) of 2-methylpyridine (pKa: 6.00), the same operation as in Example 16 was performed. When the reaction solution was analyzed by the same method, and quantified with a calibration curve, it is understood to contain 3.59 g (3.84 mmol) of the polymerizable compound (9) (still another example of the compound represented by formula (III)). The yield was 35.34 mol%.

When the same operation as in the Step 2 of Example 16 was performed and the reaction solution was analyzed by the same method, it is understood to contain 5.18 g (3.71 mmol) of the polymerizable compound (9-1) which is the target product. The yield was 34.14 mol%.

Furthermore, when the same operation as in the Step 3 of Example 16 was performed, 5.04 g of the polymerizable compound (9-1) was obtained. The isolated yield was 33.23 mol%.

### (Comparative Example 22) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 5.16 g (65.17 mmol) of pyridine (pKa: 5.23), the same operation as in Example 16 was performed. When the reaction solution was analyzed by the same method, and quantified with a calibration curve, it is understood to contain 7.15 g (7.65 mmol) of the polymerizable compound (9) (still another example of the compound represented by formula (III)). The yield was 70.41 mol%.

When the same operation as in the Step 2 of Example 16 was performed and the reaction solution was analyzed by the same method, it is understood to contain 10.33 g (7.39 mmol) of the polymerizable compound (9-1) which is the target product. The yield was 68.02 mol%.

Furthermore, when the same operation as in the Step 3 of Example 16 was performed, 10.05 g of the polymerizable compound (9-1) was obtained. The isolated yield was 66.20 mol%.

### (Comparative Example 23) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 8.42 g (65.17 mmol) of quinoline (pKa: 4.93), the same operation as in Example 16 was performed. When the reaction solution was analyzed by the same method, and quantified with a calibration curve, it is understood to contain 7.92 g (8.47 mmol) of the polymerizable compound (9) (still another example of the compound represented by formula (III)). The yield was 78.00 mol%.

When the same operation as in the Step 2 of Example 16 was performed and the reaction solution was analyzed by the same method, it is understood to contain 11.44 g (8.18 mmol) of the polymerizable compound (9-1) which is the target product. The yield was 75.35 mol%.

Furthermore, when the same operation as in the Step 3 of Example 16 was performed, 11.13 g of the polymerizable compound (9-1) was obtained. The isolated yield was 73.34 mol%.

### (Comparative Example 24) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 9.92 g (65.17 mmol) of diazabicycloundecene (pKa: 13.20), the same operation as in Example 16 was performed. When the reaction solution was analyzed by the same method, and quantified with a calibration curve, it is understood to contain 0.09 g (0.10 mmol) of the polymerizable compound (9) (still another example of the compound represented by formula (III)). The yield was 0.89 mol%.

When the same operation as in the Step 2 of Example 16 was performed and the reaction solution was analyzed by the same method, it is understood to contain 0.07 g (0.05 mmol) of the polymerizable compound (9-1) which is the target product. The yield was 0.44 mol%.

Furthermore, the same operation as in the Step 3 of Example 16 was performed, but the polymerizable compound (9-1) could not be isolated.

### (Comparative Example 25) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 8.09 g (65.17 mmol) of diazabicyclononane (pKa: 13.40), the same operation as in Example 16 was performed. When the reaction solution was analyzed by the same method, and quantified with a calibration curve, it is understood to contain 0.07 g (0.07 mmol) of the polymerizable compound (9) (still another example of the compound represented by formula (III)). The yield was 0.65 mol%.

When the same operation as in the Step 2 of Example 16 was performed and the reaction solution was analyzed by the same method, it is understood to contain 0.02 g (0.01 mmol) of the polymerizable compound (9-1) which is the target product. The yield was 0.12 mol%.

Furthermore, the same operation as in the Step 3 of Example 16 was performed, but the polymerizable compound (9-1) could not be isolated.

### (Comparative Example 26) Synthesis of Polymerizable compound (9-1) (Still another example of the compound represented by formula (VI))

With the exception that 6.98 g (65.17 mmol) of 2,6-lutidine (pKa: 6.65) which is the base in the Step 1 of Example 16 was replaced with 7.96 g (65.17 mmol) of 4-dimethyl aminopyridine (pKa: 9.52), the same operation as in Example 16 was performed. When the reaction solution was analyzed by the same method, and quantified with a calibration curve, it is understood to contain 8.20 g (8.77 mmol) of the polymerizable compound represented by the aforementioned formula (9) (polymerizable compound (9): still another example of the compound represented by formula (III)). The yield was 80.78 mol%.

The same operation as in the Step 2 of Example 16 was performed, the reaction solution was analyzed by the same method, and quantified with a calibration curve, it is understood to contain 11.80 g (8.44 mmol) of the polymerizable compound (9-1) represented by the aforementioned formula (9-1) which is the target product. The yield was 77.69 mol%.

Furthermore, when the same operation as in the Step 3 of Example 16 was performed, 11.44 g of the polymerizable compound (9-1) was obtained. The isolated yield was 75.36 mol%.

### (Comparative Example 27) Synthesis of Polymerizable compound (10-1) (Still another example of the compound represented by formula (VI))

With the exception that 6.98 g (65.17 mmol) of 2,6-lutidine (pKa: 6.65) which is the base in the Step 1 of Example 21 was replaced with 6.59 g (65.17 mmol) of triethylamine (pKa: 10.75), the same operation as in Example 21 was performed. When the reaction solution was analyzed by the same method, and quantified with a calibration curve, it is understood to contain 8.48 g (9.35 mmol) of the polymerizable compound (10) (still another example of the compound represented by formula (III). The yield was 86.10 mol%.

When the same operation as in the Step 2 of Example 21 was performed and the reaction solution was analyzed by the same method, it is understood to contain 12.37 g (9.03 mmol) of the polymerizable compound (10-1) which is the target product. The yield was 83.17 mol%.

Furthermore, when the same operation as in the Step 3 of Example 21 was performed, 12.04 g of the polymerizable compound (10-1) was obtained. The isolated yield was 80.96 mol%.

### (Comparative Example 28) Synthesis of Polymerizable compound (11-1) (Still another example of the compound represented by formula (VI))

With the exception that 6.98 g (65.17 mmol) of 2,6-lutidine (pKa:6.65) which is the base in the Step 1 of Example 22 was replaced with 6.59 g (65.17 mmol) of triethylamine (pKa: 10.75), the same operation as in Example 22 was performed. When the reaction solution was analyzed by the same method, and quantified with a calibration curve, it is understood to contain 8.51 g (9.24 mmol) of the polymerizable compound (11) (still another example of the compound represented by formula (III)). The yield was 85.04 mol%.

When the same operation as in the Step 2 of Example 22 was performed and the reaction solution was analyzed by the same method, it is understood to contain 12.35 g (8.92 mmol) of the polymerizable compound (11-1) which is the target product. The yield was 82.15 mol%.

Furthermore, when the same operation as in the Step 3 of Example 22 was performed, 12.02 g of the polymerizable compound (11-1). The isolated yield was 79.96 mol%.

### (Comparative Example 29) Synthesis of Polymerizable compound (12-1) (Still another example of the compound represented by formula (VI))

With the exception that 6.98 g (65.17 mmol) of 2,6-lutidine (pKa: 6.65) which is the base in the Step 1 of Example 23 was replaced with 6.59 g (65.17 mmol) of triethylamine (pKa: 10.75), the same operation as in Example 23 was performed. When the reaction solution was analyzed by the same method, and quantified with a calibration curve, it is understood to contain 8.77 g (9.24 mmol) of the polymerizable compound (12) (still another example of the compound represented by formula (III)). The yield was 85.06 mol%.

When the same operation as in the Step 2 of Example 23 was performed and the reaction solution was analyzed by the same method, it is understood to contain 12.90 g (9.13 mmol) of the polymerizable compound (12-1) which is the target product. The yield was 84.10 mol%.

Furthermore, when the same operation as in the Step 3 of Example 23 was performed, 12.55 g of the polymerizable compound (12-1) was obtained. The isolated yield was 81.86 mol%.

### (Comparative Example 30) Synthesis of Polymerizable compound (13-1) (Still another example of the compound represented by formula (VI))

With the exception that 6.98 g (65.17 mmol) of 2,6-lutidine (pKa: 6.65) which is the base in the Step 1 of Example 24 was replaced with 6.59 g (65.17 mmol) of triethylamine (pKa: 10.75), the same operation as in Example 24 was performed. When the reaction solution was analyzed by the same method, and quantified with a calibration curve, it is understood to contain 9.01 g (9.35 mmol) of the polymerizable compound (13) (still another example of the compound represented by formula (III)). The yield was 86.12 mol%.

When the same operation as in the Step 2 of Example 24 was performed and the reaction solution was analyzed by the same method, it is understood to contain 12.88 g (9.04 mmol) of the polymerizable compound (13-1) which is the target product. The yield was 83.19 mol%.

Furthermore, when the same operation as in the Step 3 of Example 24 was performed, 12.54 g of the polymerizable compound (13-1) was obtained. The isolated yield was 80.98 mol%.

### (Comparative Example 31) Synthesis of Polymerizable compound (14-1) (Still another example of the compound represented by formula (VI))

With the exception that 6.98 g (65.17 mmol) of 2,6-lutidine (pKa: 6.65) which is the base in the Step 1 of Example 25 was replaced with 6.59 g (65.17 mmol) of triethylamine (pKa: 10.75), the same operation as in Example 25 was performed. When the reaction solution was analyzed by the same method, and quantified with a calibration curve, it is understood to contain 8.90 g (9.26 mmol) of the polymerizable compound (14) (still another example of the compound represented by formula (III)). The yield was 85.24 mol%.

When the same operation as in the Step 2 of Example 25 was performed and the reaction solution was analyzed by the same method, it is understood to contain 12.73 g (8.94 mmol) of the polymerizable compound (14-1) which is the target product. The yield was 82.34 mol%.

Furthermore, when the same operation as in the Step 3 of Example 25 was performed, 12.39 g of the polymerizable compound (14-1) was obtained. The isolated yield was 80.15 mol%.

### (Comparative Example 32) Synthesis of Polymerizable compound (15-1) (Still another example of the compound represented by formula (VI))

With the exception that 6.98 g (65.17 mmol) of 2,6-lutidine (pKa: 6.65) which is the base in the Step 1 of Example 26 was replaced with 6.59 g (65.17 mmol) of triethylamine (pKa: 10.75), the same operation as in Example 26 was performed. When the reaction solution was analyzed by the same method, and quantified with a calibration curve, it is understood to contain 8.71 g (9.12 mmol) of the polymerizable compound (15) (still another example of the compound represented by formula (III)). The yield was 83.95 mol%.

When the same operation as in the Step 2 of Example 26 was performed, and the reaction solution was analyzed by the same method, it is understood to contain 12.49 g (8.81 mmol) of the polymerizable compound (15-1) which is the target product. The yield was 81.10 mol%.

Furthermore, when the same operation as in the Step 3 of Example 26 was performed, 12.16 g of the polymerizable compound (15-1) was obtained. The isolated yield was 78.94 mol%.

The aforementioned results are shown together in the following Table 1 and Table 1-2.

Note that, with respect to the value of "pKa" of the bases in the tables, 4-dimethyl aminopyridine, N-N-diisopropylethylamine, diazabicycloundecene, diazabicyclononane show the values described in SciFinder (Chemical Abstracts Service, American -Chemical Society), and the other bases shown the values described in CRC Handbook Of Chemistry and Physics 87th Edition (CRC Press).

Further, all of the yields in the Steps 1 to 3 were calculated based on the compound represented by formula (II) which was used in the Step 1.

**Table 1**

| | Base | | Step 1 | | Step 2 | | Step 3 | |
|---|---|---|---|---|---|---|---|---|
| | Type | pKa | Polymerizable compound | Yield (%) | Polymerizable compound | Yield (%) | Polymerizable compound | Isolated yield (%) |
| Ex. 1 | 2,6-lutidine | 6.65 | 1 | 95.75 | 1-1 | 92.49 | 1-1 | 90.03 |
| Ex. 2 | 2,4-lutidine | 6.99 | | 93.86 | | 90.91 | | 88.49 |
| Ex. 3 | 2,4,6-collidine | 7.43 | | 94.39 | | 91.01 | | 88.59 |
| Ex. 4 | 3,5-lutidine | 6.15 | | 90.12 | | 87.98 | | 85.63 |
| Ex. 5 | 3,4-lutidine | 6.46 | | 90.77 | | 86.22 | | 83.92 |
| Ex. 6 | 2,6-lutidine | 6.65 | | 94.88 | | 91.66 | | 89.21 |
| Ex. 7 | | | | 95.75 | | 91.11 | | 88.68 |
| Ex. 8 | | | 2 | 93.91 | 2-1 | 90.72 | 2-1 | 88.30 |
| Ex. 9 | | | 3 | 90.54 | 3-1 | 87.46 | 3-1 | 85.13 |
| Ex. 10 | | | 4 | 94.12 | 4-1 | 90.92 | 4-1 | 88.50 |
| Ex. 11 | | | 4 | 94.12 | 4-2 | 90.48 | 4-2 | 87.56 |
| Ex. 12 | | | 5 | 90.79 | 5-1 | 87.70 | 5-1 | 85.37 |
| Ex. 13 | | | 6 | 93.45 | 6-1 | 90.27 | 6-1 | 87.87 |
| Ex. 14 | | | 7 | 94.42 | 7-1 | 91.21 | 7-1 | 88.78 |
| Ex. 15 | | | 8 | 92.19 | 8-1 | 89.06 | 8-1 | 86.68 |
| Ex. 16 | | | 9 | 94.78 | 9-1 | 91.56 | 9-1 | 89.12 |
| Ex. 17 | 2,4-lutidine | 6.99 | | 94.34 | | 91.13 | | 88.71 |
| Ex. 18 | 2,4,6-collidine | 7.43 | | 92.52 | | 89.38 | | 86.99 |
| Ex. 19 | 3,5-lutidine | 6.15 | | 93.26 | | 90.09 | | 87.69 |
| Ex. 20 | 3,4-lutidine | 6.46 | | 91.79 | | 88.67 | | 86.31 |
| Ex. 21 | | | 10 | 94.25 | 10-1 | 91.05 | 10-1 | 88.62 |
| Ex. 22 | | | 11 | 93.96 | 11-1 | 90.77 | 11-1 | 88.35 |
| Ex. 23 | | | 12 | 94.94 | 12-1 | 91.71 | 12-1 | 89.27 |
| Ex. 24 | 2,6-lutidine | 6.65 | 13 | 92.58 | 13-1 | 89.43 | 13-1 | 87.05 |
| Ex. 25 | | | 14 | 93.35 | 14-1 | 90.18 | 14-1 | 87.77 |
| Ex. 26 | | | 15 | 91.71 | 15-1 | 88.59 | 15-1 | 86.23 |
| Comp. Ex. 1 | Triethylamine | 10.75 | 1 | 86.45 | 1-1 | 84.62 | 1-1 | 82.37 |
| Comp. Ex. 2 | N,N-diisopropylethylamine | 10.98 | | 85.21 | | 81.86 | | 79.68 |
| Comp. Ex. 3 | 2-methylpyridine | 6.00 | | 39.58 | | 35.62 | | 34.67 |
| Comp. Ex. 4 | Pyridine | 5.23 | | 80.73 | | 77.21 | | 75.15 |
| Comp. Ex. 5 | Quinoline | 4.93 | | 83.21 | | 80.17 | | 78.03 |
| Comp. Ex. 6 | Diazabicycloundecene | 13.20 | | 1.86 | | 1.16 | | Could not be isolated |
| Comp. Ex. 7 | Diazabicyclononene | 13.40 | | 2.19 | | 1.85 | | Could not be isolated |
| Comp. Ex. 8 | 4-dimethylaminopyridine | 9.52 | | 79.01 | | 75.99 | | 72.18 |
| Comp. Ex. 9 | Triethylamine | 10.75 | | 85.02 | | 82.54 | | 80.34 |
| Comp. Ex. 10 | | | | 86.45 | | 81.21 | | 79.05 |
| Comp. Ex. 11 | | | 2 | 86.72 | 2-1 | 83.77 | 2-1 | 81.54 |
| Comp. Ex. 12 | | | 3 | 85.14 | 3-1 | 82.25 | 3-1 | 80.06 |
| Comp. Ex. 13 | | | 4 | 85.15 | 4-1 | 81.26 | 4-1 | 79.88 |
| Comp. Ex. 14 | | | 4 | 85.15 | 4-2 | 80.98 | 4-2 | 77.69 |
| Comp. Ex. 15 | | | 5 | 84.89 | 5-1 | 82.00 | 5-1 | 79.82 |
| Comp. Ex. 16 | | | 6 | 86.01 | 6-1 | 83.09 | 6-1 | 80.87 |
| Comp. Ex. 17 | | | 7 | 85.67 | 7-1 | 84.69 | 7-1 | 82.43 |
| Comp. Ex. 18 | | | 8 | 86.99 | 8-1 | 84.03 | 8-1 | 81.79 |
| Comp. Ex. 19 | | | 9 | 86.12 | 9-1 | 83.19 | 9-1 | 80.98 |
| Comp. Ex. 20 | N,N-diisopropylethylamine | 10.98 | | 84.56 | | 81.69 | | 79.51 |
| Comp. Ex. 21 | 2-methylpyridine | 6.00 | | 35.34 | | 34.14 | | 33.23 |
| Comp. Ex. 22 | Pyridine | 5.23 | | 70.41 | | 68.02 | | 66.20 |
| Comp. Ex. 23 | Quinoline | 4.93 | | 78.00 | | 75.35 | | 73.34 |
| Comp. Ex. 24 | Diazabicycloundecene | 13.20 | | 0.89 | | 0.44 | | Could not be isolated |
| Comp. Ex. 25 | Diazabicyclononene | 13.40 | | 0.65 | | 0.12 | | Could not be isolated |
| Comp. Ex. 26 | 4-dimethylaminopyridine | 9.52 | | 80.78 | | 77.69 | | 75.36 |
| Comp. Ex. 27 | Triethylamine | 10.75 | 10 | 86.10 | 10-1 | 83.17 | 10-1 | 80.96 |
| Comp. Ex. 28 | | | 11 | 85.04 | 11-1 | 82.15 | 11-1 | 79.96 |
| Comp. Ex. 29 | | | 12 | 85.06 | 12-1 | 84.10 | 12-1 | 81.86 |
| Comp. Ex. 30 | | | 13 | 86.12 | 13-1 | 83.19 | 13-1 | 80.98 |
| Comp. Ex. 31 | | | 14 | 85.24 | 14-1 | 82.34 | 14-1 | 80.15 |
| Comp. Ex. 32 | | | 15 | 83.95 | 15-1 | 81.10 | 15-1 | 78.94 |

**Table 1-2**

| | Base | | Step 1 | | Step 2 | | Step 3 | |
|---|---|---|---|---|---|---|---|---|
| | Type | pKa | Polymerizable compound | Yield (%) | Polymerizable compound | Yield (%) | Polymerizable compound | Isolated yield (%) |
| Ex. 27 | 2,6-lutidine | 6.65 | 16 | 94.88 | 16-1 | 91.50 | 16-1 | 90.01 |

It is understood from Table 1 and Table 1-2 that when a base having a pKa from 6.1 to 9.5 is used in the reaction, the polymerizable compounds 1 to 15 and the polymerizable compounds 1-1 to 15-1 can be obtained at a good yield (Examples 1 to 27).

On the one hand, it is understood that when a base having a pKa of less than 6.1 or greater than 9.5 is used in the reaction, the polymerizable compounds 1 to 15 and the polymerizable compounds 1-1 to 15-1 cannot be obtained at a good yield (Comparative Examples 1 to 32)..

## Claims

1. A method of producing a polymerizable compound, comprising reacting a compound represented by formula (I) with a compound represented by formula (II) in an organic solvent in which a base having a pKa from 6.1 to 9.5 is present, so as to obtain a reaction solution containing a polymerizable compound represented by formula (III): 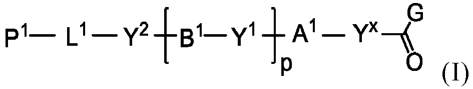 where in the formula (I),
Y^{x} represents a single bond, -CH₂-, -CH₂-CH₂-, or -CH=CH-,
A¹ and B¹ each independently represent a cyclic aliphatic group which may have a substituent, or an aromatic group which may have a substituent,
Y¹ and Y² each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, -NR²¹-C(=O)-NR²²-, -O-CH₂-, -O-CH₂-CH₂-, -CH₂-O-, -CH₂-CH₂-O-, -CH₂-O-CH₂-, -CH₂-CH₂-O-CH₂-, -CH₂-O-CH₂-CH₂-, -CH₂-CH₂-O-CH₂-CH₂-, -C(=O)-CH₂-, -C(=O)-CH₂-CH₂-, -CH₂-C(=O)-, -CH₂-CH₂-C(=O)-, -O-C(=O)-CH₂-, -CH₂-C(=O)-O-, -O-C(=O)-CH₂-CH₂-, -CH₂-CH₂-C(=O)-O-, -NR²¹-C(=O)-CH₂-, -NR²¹-C(=O)-CH₂-CH₂-, -CH₂-C(=O)-NR²¹-, -CH₂-CH₂-C(=O)-NR²¹-, -O-C(=O)-O-CH₂-, -O-C(=O)-O-CH₂-CH₂-, -CH₂-O-C(=O)-O-, -CH₂-CH₂-O-C(=O)-O-, -CH₂-O-C(=O)-O-CH₂-, -CH₂-CH₂-O-C(=O)-O-CH₂-, -CH₂-O-C(=O)-O-CH₂-CH₂-, -CH₂-CH₂-O-C(=O)-O-CH₂-CH₂-, -NR²¹-C(=O)-NR²²-CH₂-, -NR²¹-C(=O)-NR²²-O-CH₂-CH₂-, -CH₂-CH₂-O-NR²¹-C(=O)-NR²²-, -CH₂-NR²¹-C(=O)-NR²²-, -CH₂-CH₂-NR²¹-C(=O)-NR²²-, -NR²¹-C(=O)-NR²²-CH₂-CH₂-, -CH₂-NR²¹-C(=O)-NR²²-CH₂-, -CH₂-CH₂-NR²¹-C(=O)-NR²²-CH₂-, -CH₂-NR²¹-C(=O)-NR²²-CH₂-CH₂-, -CH₂-NR²¹-C(=O)-NR²²-O-CH₂-CH₂-, -CH₂-CH₂-O-NR²¹-C(=O)-NR²²-CH₂-, -CH₂-CH₂-NR²¹-C(=O)-NR²²-O-CH₂-CH₂-, or -CH₂-CH₂-O-NR²¹-C(=O)-NR²²-CH₂-CH₂-, where R²¹ and R²² each independently represent a hydrogen atom or an alkyl group having 1 to 6 carbon atoms,
L¹ is an organic group which is either an alkylene group having 1 to 20 carbon atoms, or an alkylene group having 3 to 20 carbon atoms in which at least one methylene group (-CH₂-) contained in the alkylene group is substituted by -O- or -C(=O)-, and the hydrogen atom included in the organic group of L¹ may be substituted by an alkyl group having 1 to 5 carbon atoms, an alkoxy group having 1 to 5 carbon atoms, or a halogen atom, with the proviso that the methylene groups (-CH₂-) on both ends of L¹ are not substituted with -O- or -C(=O)-,
P¹ represents a hydrogen atom or a polymerizable group,
p is an integer from 0 to 3, and
G represents a leaving group, 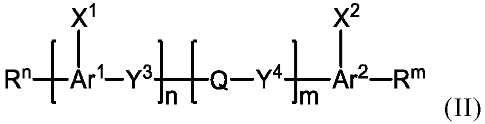
where in the formula (II),
Ar¹ and Ar² each independently represent an aromatic hydrocarbon ring group which may have a substituent, or an aromatic heterocyclic ring group which may have a substituent,
X¹ and X² each independently represent -CHO, or -C(=O)-R^{a}, where R^{a} represents an organic group having 1 to 20 carbon atoms which may have a substituent,
Y³ and Y⁴ each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, or -NR²¹-C(=O)-NR²²-, where R²¹ and R²² each independently represent a hydrogen atom or an alkyl group having 1 to 6 carbon atoms,
Q represents an organic group having 1 to 20 carbon atoms which may have a substituent,
n and m each independently represent an integer from 0 to 3,
Rⁿ and R^{m} each independently represent -CH₂-CH₂-OR^{b}, -CH₂-OR^{b}, -CH₂-CH₂-OH, -CH₂-OH, -OR^{b}, -COOR^{b}, -NHR²⁰, -SH, a hydroxyl group, or a carboxyl group, where R²⁰ represents a hydrogen atom or an alkyl group having 1 to 6 carbon atoms, R^{b} represents a protecting group, and when Rⁿ or R^{m} is -CH₂-CH₂-OR^{b}, -CH₂-OR^{b}, -OR^{b}, or -COOR^{b}, at least one of Rⁿ and R^{m} is -CH₂-CH₂-OH, -CH₂-OH, -NHR²⁰, -SH, a hydroxyl group, or a carboxyl group, 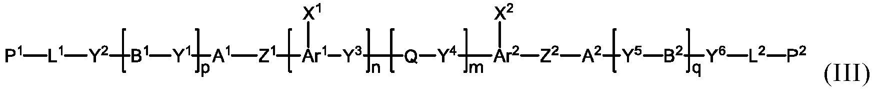
where in the formula (III),
A¹, A², B¹ and B² each independently represent a cyclic aliphatic group which may have a substituent, or an aromatic group which may have a substituent,
Ar¹ and Ar² each independently represent an aromatic hydrocarbon ring group which may have a substituent, or an aromatic heterocyclic ring group which may have a substituent,
X¹ and X² each independently represent -CHO, or -C(=O)-R^{a}, where R^{a} represents an organic group having 1 to 20 carbon atoms which may have a substituent,
Z¹ and Z² each independently represent -C(=O)-O-, -O-C(=O)-, -C(=O)-S-, -S-C(=O)-, -NR²⁰-C(=O)-, -C(=O)-NR²⁰-, -CH=CH-C(=O)-O-, -O-C(=O)-CH=CH-, -CH₂-C(=O)-O-, -O-C(=O)-CH₂-, -CH₂-O-C(=O)-, -C(=O)-O-CH₂-, -CH₂-CH₂-C(=O)-O-, -O-C(=O)-CH₂-CH₂-, -CH₂-CH₂-O-C(=O)-, -C(=O)-O-CH₂-CH₂-, or -C(=O)-O-C(=O)-, where R²⁰ represents a hydrogen atom or an alkyl group having 1 to 6 carbon atoms,
Y¹ to Y⁶ each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, -NR²¹-C(=O)-NR²²-, -O-CH₂-, -O-CH₂-CH₂-, -CH₂-O-, -CH₂-CH₂-O-, -CH₂-O-CH₂-, -CH₂-CH₂-O-CH₂-, -CH₂-O-CH₂-CH₂-, -CH₂-CH₂-O-CH₂-CH₂-, -C(=O)-CH₂-, -C(=O)-CH₂-CH₂-, -CH₂-C(=O)-, -CH₂-CH₂-C(=O)-, -O-C(=O)-CH₂-, -CH₂-C(=O)-O-, -O-C(=O)-CH₂-CH₂-, -CH₂-CH₂-C(=O)-O-, -NR²¹-C(=O)-CH₂-, -NR²¹-C(=O)-CH₂-CH₂-, -CH₂-C(=O)-NR²¹-, -CH₂-CH₂-C(=O)-NR²¹-, -O-C(=O)-O-CH₂-, -O-C(=O)-O-CH₂-CH₂-, -CH₂-O-C(=O)-O-, -CH₂-CH₂-O-C(=O)-O-, -CH₂-O-C(=O)-O-CH₂-, -CH₂-CH₂-O-C(=O)-O-CH₂-, -CH₂-O-C(=O)-O-CH₂-CH₂-, -CH₂-CH₂-O-C(=O)-O-CH₂-CH₂-, -NR²¹-C(=O)-NR²²-CH₂-, -NR²¹-C(=O)-NR²²-O-CH₂-CH₂-, -CH₂-CH₂-O-NR²¹-C(=O)-NR²²-, -CH₂-NR²¹-C(=O)-NR²²-, -CH₂-CH₂-NR²¹-C(=O)-NR²²-, -NR²¹-C(=O)-NR²²-CH₂-CH₂-, -CH₂-NR²¹-C(=O)-NR²²-CH₂-, -CH₂-CH₂-NR²¹-C(=O)-NR²²-CH₂-, -CH₂-NR²¹-C(=O)-NR²²-CH₂-CH₂-, -CH₂-NR²¹-C(=O)-NR²²-O-CH₂-CH₂-, -CH₂-CH₂-O-NR²¹-C(=O)-NR²²-CH₂-, -CH₂-CH₂-NR²¹-C(=O)-NR²²-O-CH₂-CH₂-, or -CH₂-CH₂-O-NR²¹-C(=O)-NR²²-CH₂-CH₂-, where R²¹ and R²² each independently represent a hydrogen atom or an alkyl group having 1 to 6 carbon atoms,
L¹ and L² each independently represent an organic group which is either an alkylene group having 1 to 20 carbon atoms, or an alkylene group having 3 to 20 carbon atoms in which at least one methylene group (-CH₂-) contained in the alkylene group is substituted by -O- or -C(=O)-, and the hydrogen atom included in the organic group of L¹ and L² may be substituted by an alkyl group having 1 to 5 carbon atoms, an alkoxy group having 1 to 5 carbon atoms, or a halogen atom, with the proviso that the methylene groups (-CH₂-) on both ends of L¹ and L² are not substituted with -O- or -C(=O)-,
Q represents an organic group having 1 to 20 carbon atoms which may have a substituent,
P¹ and P² each independently represent a hydrogen atom or a polymerizable group, and at least one of P¹ and P² represents a polymerizable group, and
p, q, n and m each independently represent an integer from 0 to 3.

2. The method of producing a polymerizable compound according to claim 1, wherein the base is a tertiary amine.

3. The method of producing a polymerizable compound according to claim 1 or 2, wherein the base has a pKa from 6.5 to 7.5.

4. The method of producing a polymerizable compound according to any one of claims 1 to 3, wherein at least one pyridine having at least two alkyl groups having 1 to 6 carbon atoms is used as the base.

5. The method of producing a polymerizable compound according to any one of claims 1 to 4, wherein at least one pyridine where at least two hydrogen atoms among hydrogen atoms at the 2-position, 4-position and 6-position in the pyridine are substituted with an alkyl group having 1 to 6 carbon atoms is used as the base.

6. The method of producing a polymerizable compound according to any one of claims 1 to 5, wherein at least one compound selected from the group consisting of 2,4-lutidine, 2,6-lutidine, and 2,4,6-collidine is used as the base.

7. The method of producing a polymerizable compound according to any one of claims 1 to 6, wherein the Ar¹-X¹ and Ar²-X² each independently are represented by any of the following formulas (VIII-1) to (VIII-7): 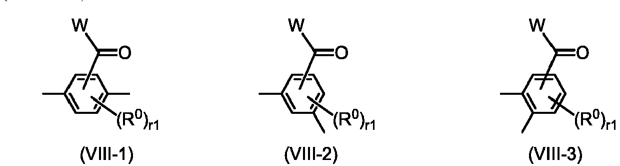 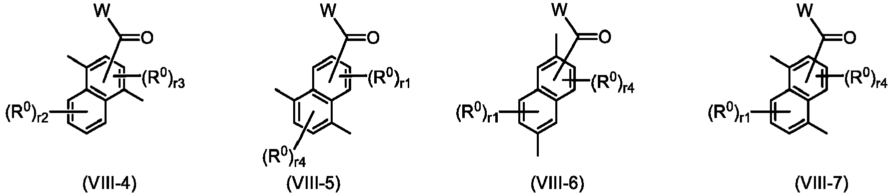 where in the formulas (VIII-1) to (VIII-7),
W represents a hydrogen atom or an alkyl group having 1 to 6 carbon atoms, and
R⁰ represents a halogen atom; a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N,N-dialkylamino group having 2 to 12 carbon atoms, an alkoxy group having 1 to 6 carbon atoms, a nitro group, -C(=O)-R^{a1}, -O-C(=O)-R^{a1}, -C(=O)-O-R^{a1}, or -SO₂R^{a1}, where R^{a1} represents an alkyl group having 1 to 6 carbon atoms, or an aromatic hydrocarbon ring group having 6 to 20 carbon atoms which may have an alkyl group having 1 to 6 carbon atoms or an alkoxy group having 1 to 6 carbon atoms as a substituent, r1 is an integer from 0 to 3, r2 is an integer from 0 to 4, r3 is 0 or 1, and r4 is an integer from 0 to 2, with the proviso that when there is a plurality of R⁰, each R⁰ may be the same or may be different.

8. The method of producing a polymerizable compound according to any one of claims 1 to 7, wherein the polymerizable compound represented by formula (III) is represented by any of the following formulas (III-1) to (III-6): 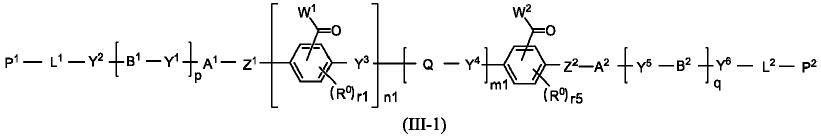 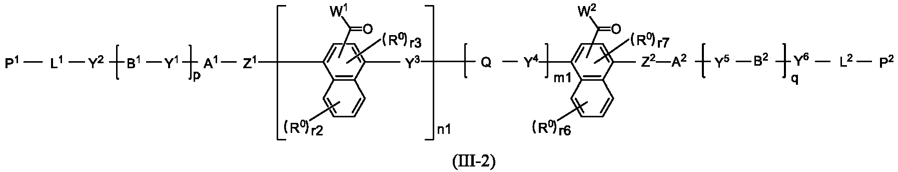 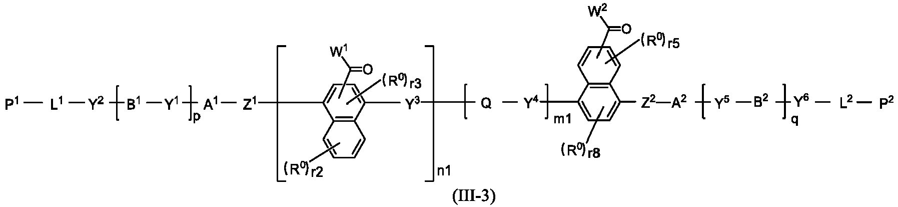 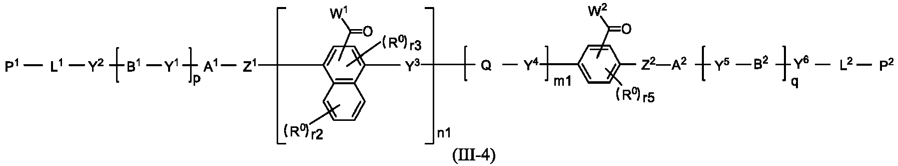 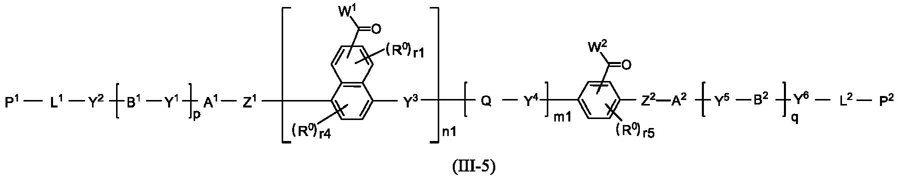 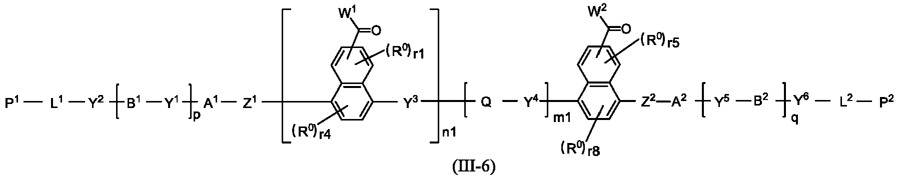 where in the formulas (III-1) to (III-6),
W¹ and W² each independently represent a hydrogen atom or an organic group having 1 to 20 carbon atoms which may have a substituent,
n1 is an integer of 0 or 1,
m1 is an integer of 0 or 1,
R⁰ represents a halogen atom; a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N,N-dialkylamino group having 2 to 12 carbon atoms, an alkoxy group having 1 to 6 carbon atoms, a nitro group, -C(=O)-R^{a1}, -O-C(=O)-R^{a1}, -C(=O)-O-R^{a1}, or -SO₂R^{a1}, where R^{a1} represents an alkyl group having 1 to 6 carbon atoms, or an aromatic hydrocarbon ring group having 6 to 20 carbon atoms which may have an alkyl group having 1 to 6 carbon atoms or an alkoxy group having 1 to 6 carbon atoms as a substituent, r1 and r5 each independently represent an integer from 0 to 3, r2 and r6 each independently represent an integer from 0 to 4, r3 and r7 each independently are 0 or 1, and r4 and r8 each independently represent an integer from 0 to 2, wherein, when there is a plurality of R⁰, each R⁰ may be the same or may be different, and
A¹, A², B¹, B², Y¹ to Y⁶, L¹, L², P¹, P², Z¹, Z², Q, p and q are the same as defined above.

9. The method of producing a polymerizable compound according to any one of claims 1 to 8, wherein P¹ and P² each independently are represented by the following formula (IV): 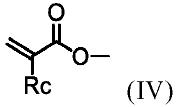 where in the formula (IV), Rc represents a hydrogen atom, a methyl group or a chlorine atom.

10. The method of producing a polymerizable compound according to any one of claims 1 to 9, wherein Q is represented by any of the following formulas (VII-1) to (VII-29):

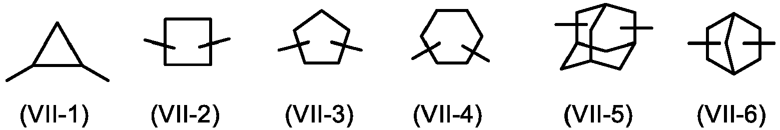

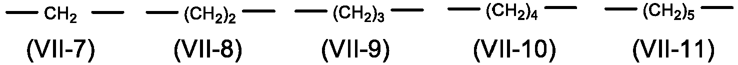

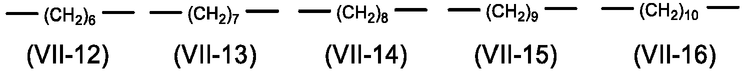

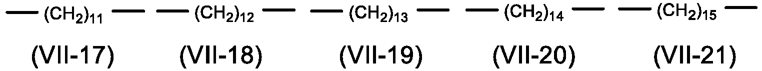

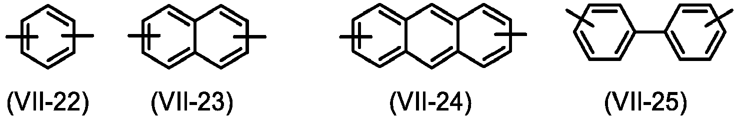

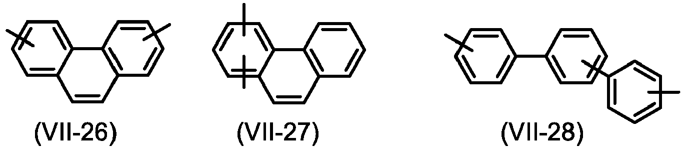

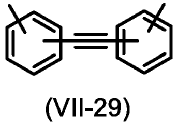

11. The method of producing a polymerizable compound according to any one of claims 1 to 10, wherein the compound represented by formula (I) is reacted with the compound represented by the formula (II) in the presence of a polymerizable compound represented by the following formula (XII): 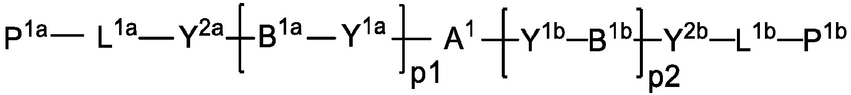 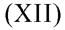 where in the formula (XII),
A¹, B^{1a} and B^{1b} each independently represent a cyclic aliphatic group which may have a substituent, or an aromatic group which may have a substituent,
Y^{1a}, Y^{1b}, Y^{2a} and Y^{2b} each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, -NR²¹-C(=O)-NR²²-, -O-CH₂-, -O-CH₂-CH₂-, -CH₂-O-, -CH₂-CH₂-O-, -CH₂-O-CH₂-, -CH₂-CH₂-O-CH₂-, -CH₂-O-CH₂-CH₂-, -CH₂-CH₂-O-CH₂-CH₂-, -C(=O)-CH₂-, -C(=O)-CH₂-CH₂-, -CH₂-C(=O)-, -CH₂-CH₂-C(=O)-, -O-C(=O)-CH₂-, -CH₂-C(=O)-O-, -O-C(=O)-CH₂-CH₂-, -CH₂-CH₂-C(=O)-O-, -NR²¹-C(=O)-CH₂-, -NR²¹-C(=O)-CH₂-CH₂-, -CH₂-C(=O)-NR²¹-, -CH₂-CH₂-C(=O)-NR²¹-, -O-C(=O)-O-CH₂-, -O-C(=O)-O-CH₂-CH₂-, -CH₂-O-C(=O)-O-, -CH₂-CH₂-O-C(=O)-O-, -CH₂-O-C(=O)-O-CH₂-, -CH₂-CH₂-O-C(=O)-O-CH₂-, -CH₂-O-C(=O)-O-CH₂-CH₂-, -CH₂-CH₂-O-C(=O)-O-CH₂-CH₂-, -NR²¹-C(=O)-NR²²-CH₂-, -NR²¹-C(=O)-NR²²-O-CH₂-CH₂-, -CH₂-CH₂-O-NR²¹-C(=O)-NR²²-, -CH₂-NR²¹-C(=O)-NR²²-, -CH₂-CH₂-NR²¹-C(=O)-NR²²-, -NR²¹-C(=O)-NR²²-CH₂-CH₂-, -CH₂-NR²¹-C(=O)-NR²²-CH₂-, -CH₂-CH₂-NR²¹-C(=O)-NR²²-CH₂-, -CH₂-NR²¹-C(=O)-NR²²-CH₂-CH₂-, -CH₂-NR²¹-C(=O)-NR²²-O-CH₂-CH₂-, -CH₂-CH₂-O-NR²¹-C(=O)-NR²²-CH₂-, -CH₂-CH₂-NR²¹-C(=O)-NR²²-O-CH₂-CH₂-, or -CH₂-CH₂-O-NR²¹-C(=O)-NR²²-CH₂-CH₂-, where R²¹ and R²² each independently represent a hydrogen atom or an alkyl group having 1 to 6 carbon atoms,
L^{1a} and L^{1b} each independently represent an organic group which is either an alkylene group having 1 to 20 carbon atoms, or an alkylene group having 3 to 20 carbon atoms in which at least one methylene group (-CH₂-) contained in the alkylene group is substituted by -O- or -C(=O)-, and the hydrogen atom included in the organic group of L^{1a} and L^{1b} may be substituted by an alkyl group having 1 to 5 carbon atoms, an alkoxy group having 1 to 5 carbon atoms, or a halogen atom, with the proviso that the methylene groups (-CH₂-) on both ends of L^{1a} and L^{1b} are not substituted with -O- or -C(=O)-,
P^{1a} and P^{1b} each independently represent a polymerizable group, and
p1 and p2 each independently represent an integer from 0 to 3.

12. A method of producing a polymerizable compound, comprising: a Step 1 which uses the method of producing a polymerizable compound according to any one of claims 1 to 11 to obtain the polymerizable compound represented by the formula (III); and a Step 2 which reacts the polymerizable compound represented by the formula (III) obtained in the Step 1 with a compound represented by the following formula (V) to obtain a polymerizable compound represented by the following formula (VI):
**D-NH₂** (V)
where in the formula (V),
D is represented by the following formula (V-I) or (V-II): 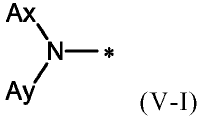 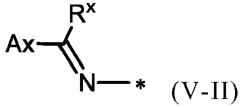
where * represents an amino group,
Ax represents an organic group having at least one aromatic ring selected from the group consisting of an aromatic hydrocarbon ring having 6 to 30 carbon atoms and an aromatic heterocyclic ring having 2 to 30 carbon atoms, and the aromatic ring included in Ax may have a substituent,
Ay represents a hydrogen atom or an organic group having 1 to 30 carbon atoms which may have a substituent, and
R^{x} represents a hydrogen atom or an organic group having 1 to 30 carbon atoms which may have a substituent, 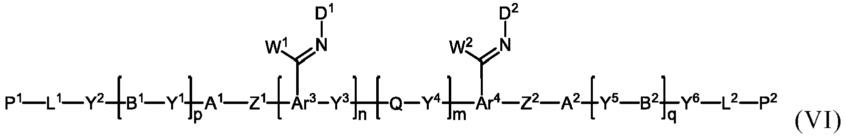
where in the formula (VI),
W¹ and W² each independently represent a hydrogen atom or an organic group having 1 to 20 carbon atoms which may have a substituent,
Ar³ and Ar⁴ each independently represent an aromatic hydrocarbon ring group which may have a substituent, or an aromatic heterocyclic ring group which may have a substituent,
D¹ and D² each independently represent the following formula (V-I) or (V-II), 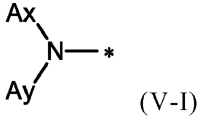 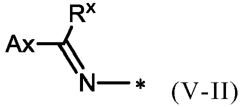
where * represents an amino group,
Ax represents an organic group having at least one aromatic ring selected from the group consisting of an aromatic hydrocarbon ring having 6 to 30 carbon atoms and an aromatic heterocyclic ring having 2 to 30 carbon atoms, and the aromatic ring included in Ax may have a substituent,
Ay represents a hydrogen atom or an organic group having 1 to 30 carbon atoms which may have a substituent,
R^{x} represents a hydrogen atom or an organic group having 1 to 30 carbon atoms which may have a substituent, and
A¹, A², B¹, B², Y¹ to Y⁶, L¹, L², P¹, P², Z¹, Z², Q, p, q, n and m are the same as defined above.

13. The method of producing a polymerizable compound according to claim 12, wherein the Ar³-W¹C=N-D¹ and Ar⁴-W²C=N-D² each independently are represented by any of the following formulas (IX-1) to (IX-14): 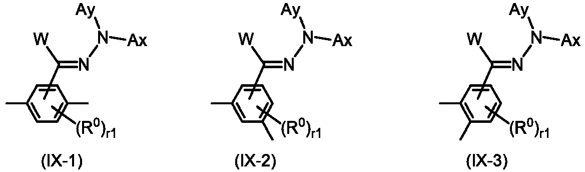 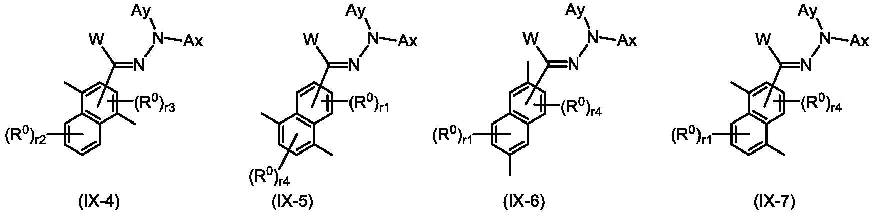 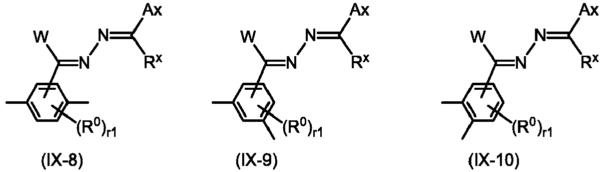 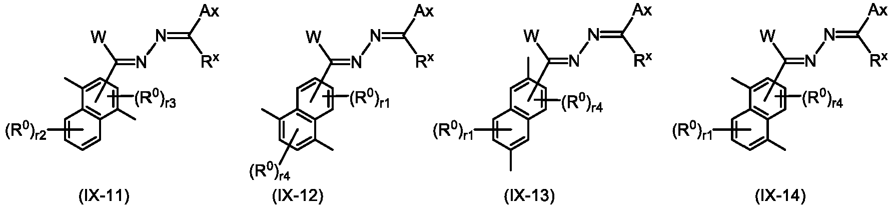 where in the formulas (IX-1) to (IX-14),
Ax represents an organic group having at least one aromatic ring selected from the group consisting of an aromatic hydrocarbon ring having 6 to 30 carbon atoms and an aromatic heterocyclic ring having 2 to 30 carbon atoms, and the aromatic ring included in Ax may have a substituent,
Ay represents a hydrogen atom or an organic group having 1 to 30 carbon atoms which may have a substituent,
R^{x} represents a hydrogen atom or an organic group having 1 to 30 carbon atoms which may have a substituent,
W represents a hydrogen atom or an alkyl group having 1 to 6 carbon atoms, and
R⁰ represents a halogen atom; a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N,N-dialkylamino group having 2 to 12 carbon atoms, an alkoxy group having 1 to 6 carbon atoms, a nitro group, -C(=O)-R^{a1}, -O-C(=O)-R^{a1}, -C(=O)-O-R^{a1}, or -SO₂R^{a1}, where R^{a1} represents an alkyl group having 1 to 6 carbon atoms, or an aromatic hydrocarbon ring group having 6 to 20 carbon atoms which may have an alkyl group having 1 to 6 carbon atoms or an alkoxy group having 1 to 6 carbon atoms as a substituent, r1 is an integer from 0 to 3, r2 is an integer from 0 to 4, r3 is 0 or 1, and r4 is an integer from 0 to 2, with the proviso that when there is a plurality of R⁰, each R⁰ may be the same or may be different.

14. The method of producing a polymerizable compound according to claim 12 or 13, wherein the Ax each independently represents the following formula (XI): 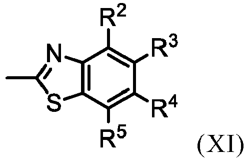 where in the formula (XI),
R² to R⁵ each independently represent a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, -OCF₃, -O-C(=O)-R^{b1}, or -C(=O)-O-R^{b1},
R^{b1} represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 18 carbon atoms which may have a substituent, and
each of R² to R⁵ may be the same or different, one or more ring constituent C-R² to C-R⁵ may be replaced by a nitrogen atom.

15. The method of producing a polymerizable compound according to any one of claims 12 to 14, wherein the polymerizable compound represented by formula (VI) is represented by any of the following formulas (VI-1) to (VI-12): 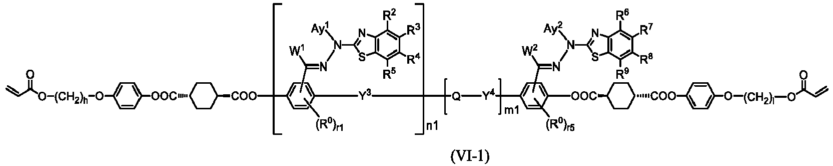 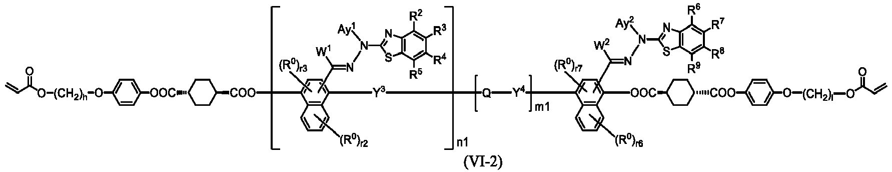 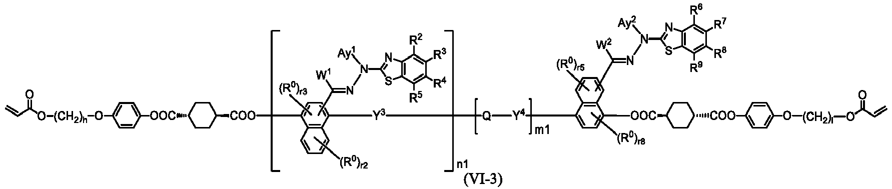 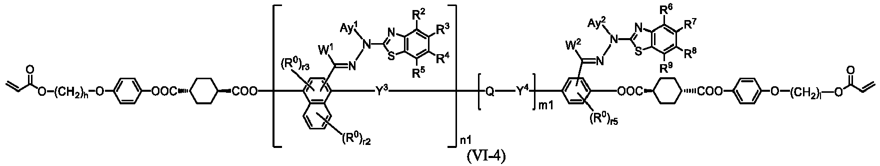 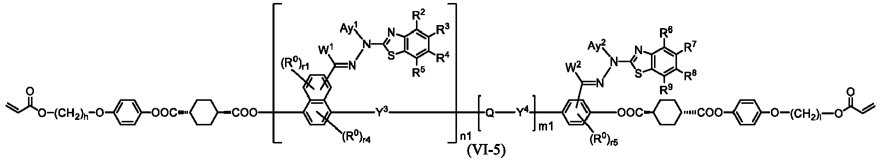 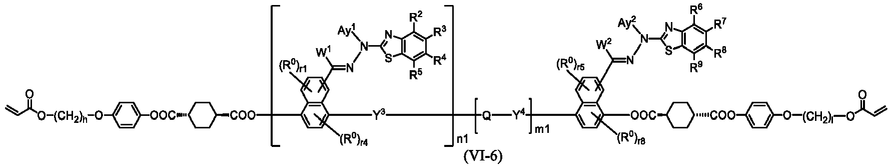 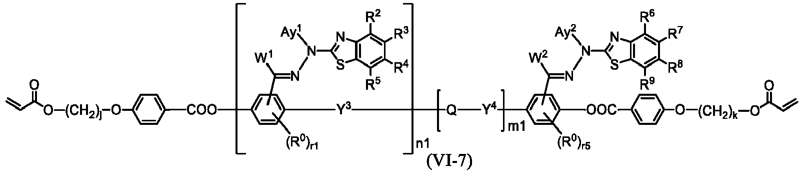 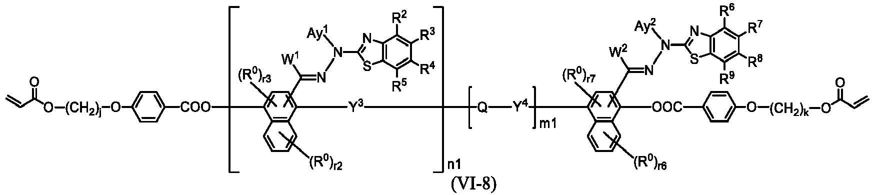 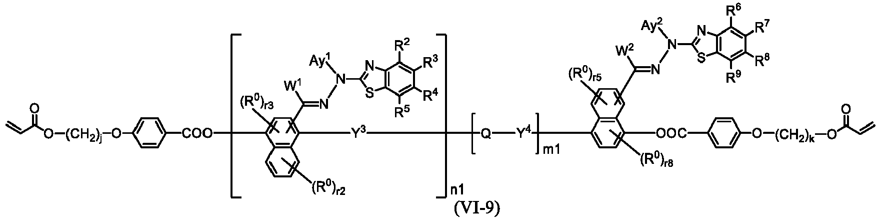 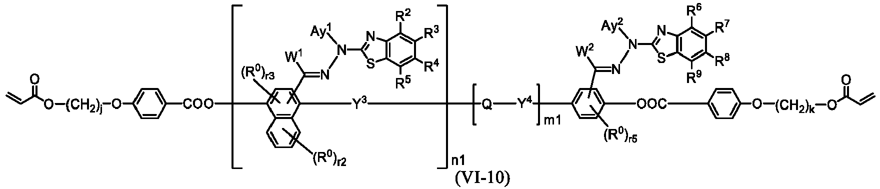 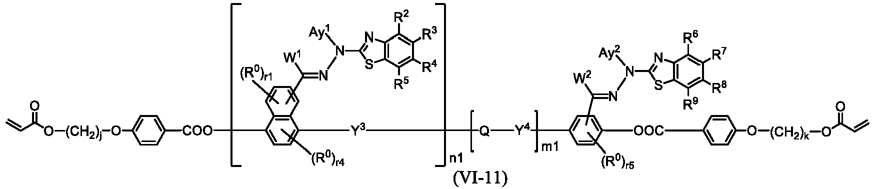 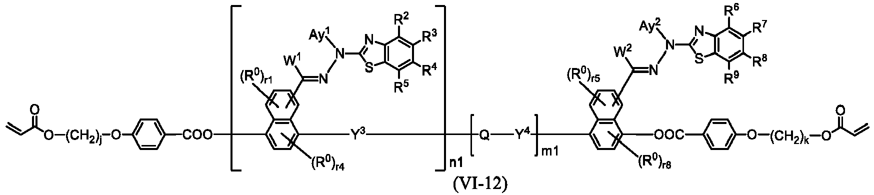 where in the formulas (VI-1) to (VI-12),
W¹ and W² each independently represent a hydrogen atom or an organic group having 1 to 20 carbon atoms which may have a substituent,
Ay¹ and Ay² each independently represent a hydrogen atom or an organic group having 1 to 30 carbon atoms which may have a substituent,
n1 is an integer of 0 or 1,
m1 is an integer of 0 or 1,
R² to R⁹ each independently represent a hydrogen atom, a halogen atom, an alkyl group having 1 to 6 carbon atoms, a cyano group, a nitro group, a fluoroalkyl group having 1 to 6 carbon atoms, an alkoxy group having 1 to 6 carbon atoms, -OCF₃, -O-C(=O)-R^{b1}, or -C(=O)-O-R^{b1},
R^{b1} represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring group having 5 to 18 carbon atoms which may have a substituent,
the plurality of R² to R⁹ may be the same or different, and one or more ring constituent C-R² to C-R⁹ may be replaced by a nitrogen atom,
R⁰ represents a halogen atom; a cyano group, an alkyl group having 1 to 6 carbon atoms, an alkenyl group having 2 to 6 carbon atoms, an alkyl group having 1 to 6 carbon atoms in which at least one hydrogen atom is substituted with a halogen atom, an N,N-dialkylamino group having 2 to 12 carbon atoms, an alkoxy group having 1 to 6 carbon atoms, a nitro group, -C(=O)-R^{a1}, -O-C(=O)-R^{a1}, -C(=O)-O-R^{a1}, or -SO₂R^{a1}, where R^{a1} represents an alkyl group having 1 to 6 carbon atoms, or an aromatic hydrocarbon ring group having 6 to 20 carbon atoms which may have an alkyl group having 1 to 6 carbon atoms or an alkoxy group having 1 to 6 carbon atoms as a substituent, r1 and r5 each independently represent an integer from 0 to 3, r2 and r6 each independently represent an integer from 0 to 4, r3 and r7 each independently are 0 or 1, and r4 and r8 each independently represent an integer from 0 to 2, wherein, when there is a plurality of R⁰, each R⁰ may be the same or may be different,
h, 1, j and k each independently represent an integer from 1 to 18, and
Y³, Y⁴, and Q are the same as defined above.

16. The method of producing a polymerizable compound according to any one of claims 12 to 15, wherein the compound represented by formula (V) and an acid are added to a reaction solution obtained in the Step 1 to perform a reaction in the Step 2.

17. The method of producing a polymerizable compound according to claim 16, wherein the acid is an inorganic acid or an organic acid having 1 to 20 carbon atoms.

18. The method of producing a polymerizable compound according to claim 16 or 17, wherein the acid is an acidic aqueous solution, and the organic solvent is a water-immiscible organic solvent.

19. The method of producing a polymerizable compound according to any one of claims 16 to 18, wherein the acid is at least one compound selected from the group consisting of hydrochloric acid, sulfuric acid, phosphoric acid, boric acid, sulfonic acid, sulfinic acid, formic acid, acetic acid and oxalic acid.

20. A solution comprising a polymerizable compound represented by the formula (III) obtained using the method according to any one of claims 1 to 11, and a polymerizable compound represented by the following formula (XII) : 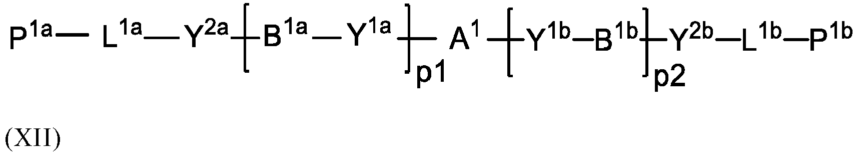 where in the formula (XII),
A¹, B^{1a} and B^{1b} each independently represent a cyclic aliphatic group which may have a substituent, or an aromatic group which may have a substituent,
Y^{1a}, Y^{1b}, Y^{2a} and Y^{2b} each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, -NR²¹-C(=O)-NR²²-, -O-CH₂-, -O-CH₂-CH₂-, -CH₂-O-, -CH₂-CH₂-O-, -CH₂-O-CH₂-, -CH₂-CH₂-O-CH₂-, -CH₂-O-CH₂-CH₂-, -CH₂-CH₂-O-CH₂-CH₂-, -C(=O)-CH₂-, -C(=O)-CH₂-CH₂-, -CH₂-C(=O)-, -CH₂-CH₂-C(=O)-, -O-C(=O)-CH₂-, -CH₂-C(=O)-O-, -O-C(=O)-CH₂-CH₂-, -CH₂-CH₂-C(=O)-O-, -NR²¹-C(=O)-CH₂-, -NR²¹-C(=O)-CH₂-CH₂-, -CH₂-C(=O)-NR²¹-, -CH₂-CH₂-C(=O)-NR²¹-, -O-C(=O)-O-CH₂-, -O-C(=O)-O-CH₂-CH₂-, -CH₂-O-C(=O)-O-, -CH₂-CH₂-O-C(=O)-O-, -CH₂-O-C(=O)-O-CH₂-, -CH₂-CH₂-O-C(=O)-O-CH₂-, -CH₂-O-C(=O)-O-CH₂-CH₂-, -CH₂-CH₂-O-C(=O)-O-CH₂-CH₂-, -NR²¹-C(=O)-NR²²-CH₂-, -NR²¹-C(=O)-NR²²-O-CH₂-CH₂-, -CH₂-CH₂-O-NR²¹-C(=O)-NR²²-, -CH₂-NR²¹-C(=O)-NR²²-, -CH₂-CH₂-NR²¹-C(=O)-NR²²-, -NR²¹-C(=O)-NR²²-CH₂-CH₂-, -CH₂-NR²¹-C(=O)-NR²²-CH₂-, -CH₂-CH₂-NR²¹-C(=O)-NR²²-CH₂-, -CH₂-NR²¹-C(=O)-NR²²-CH₂-CH₂-, -CH₂-NR²¹-C(=O)-NR²²-O-CH₂-CH₂-, -CH₂-CH₂-O-NR²¹-C(=O)-NR²²-CH₂-, -CH₂-CH₂-NR²¹-C(=O)-NR²²-O-CH₂-CH₂-, or -CH₂-CH₂-O-NR²¹-C(=O)-NR²²-CH₂-CH₂-, where R²¹ and R²² each independently represent a hydrogen atom or an alkyl group having 1 to 6 carbon atoms,
L^{1a} and L^{1b} each independently represent an organic group which is either an alkylene group having 1 to 20 carbon atoms, or an alkylene group having 3 to 20 carbon atoms in which at least one methylene group (-CH₂-) contained in the alkylene group is substituted by -O- or -C(=O)-, and the hydrogen atom included in the organic group of L^{1a} and L^{1b} may be substituted by an alkyl group having 1 to 5 carbon atoms, an alkoxy group having 1 to 5 carbon atoms, or a halogen atom, with the proviso that the methylene groups (-CH₂-) on both ends of L^{1a} and L^{1b} are not substituted with -O- or -C(=O)-,
P^{1a} and P^{1b} each independently represent a polymerizable group, and
p1 and p2 each independently represent an integer from 0 to 3.

21. A solution comprising the polymerizable compound represented by formula (VI) obtained using the method according to any one of claims 12 to 19 and a polymerizable compound represented by the following formula (XII): 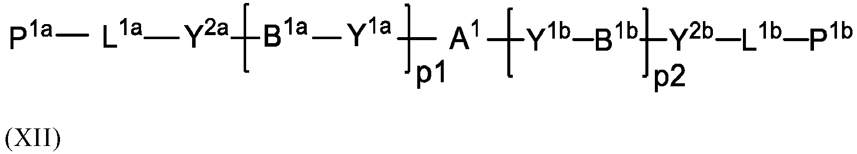 where in the formula (XII),
A¹, B^{1a} and B^{1b} each independently represent a cyclic aliphatic group which may have a substituent, or an aromatic group which may have a substituent,
Y^{1a}, Y^{1b}, Y^{2a} and Y^{2b} each independently represent a single bond, -O-, -C(=O)-, -C(=O)-O-, -O-C(=O)-, -NR²¹-C(=O)-, -C(=O)-NR²¹-, -O-C(=O)-O-, -NR²¹-C(=O)-O-, -O-C(=O)-NR²¹-, -NR²¹-C(=O)-NR²²-, -O-CH₂-, -O-CH₂-CH₂-, -CH₂-O-, -CH₂-CH₂-O-, -CH₂-O-CH₂-, -CH₂-CH₂-O-CH₂-, -CH₂-O-CH₂-CH₂-, -CH₂-CH₂-O-CH₂-CH₂-, -C(=O)-CH₂-, -C(=O)-CH₂-CH₂-, -CH₂-C(=O)-, -CH₂-CH₂-C(=O)-, -O-C(=O)-CH₂-, -CH₂-C(=O)-O-, -O-C(=O)-CH₂-CH₂-, -CH₂-CH₂-C(=O)-O-, -NR²¹-C(=O)-CH₂-, -NR²¹-C(=O)-CH₂-CH₂-, -CH₂-C(=O)-NR²¹-, -CH₂-CH₂-C(=O)-NR²¹-, -O-C(=O)-O-CH₂-, -O-C(=O)-O-CH₂-CH₂-, -CH₂-O-C(=O)-O-, -CH₂-CH₂-O-C(=O)-O-, -CH₂-O-C(=O)-O-CH₂-, -CH₂-CH₂-O-C(=O)-O-CH₂-, -CH₂-O-C(=O)-O-CH₂-CH₂-, -CH₂-CH₂-O-C(=O)-O-CH₂-CH₂-, -NR²¹-C(=O)-NR²²-CH₂-, -NR²¹-C(=O)-NR²²-O-CH₂-CH₂-, -CH₂-CH₂-O-NR²¹-C(=O)-NR²²-, -CH₂-NR²¹-C(=O)-NR²²-, -CH₂-CH₂-NR²¹-C(=O)-NR²²-, -NR²¹-C(=O)-NR²²-CH₂-CH₂-, -CH₂-NR²¹-C(=O)-NR²²-CH₂-, -CH₂-CH₂-NR²¹-C(=O)-NR²²-CH₂-, -CH₂-NR²¹-C(=O)-NR²²-CH₂-CH₂-, -CH₂-NR²¹-C(=O)-NR²²-O-CH₂-CH₂-, -CH₂-CH₂-O-NR²¹-C(=O)-NR²²-CH₂-, -CH₂-CH₂-NR²¹-C(=O)-NR²²-O-CH₂-CH₂-, or -CH₂-CH₂-O-NR²¹-C(=O)-NR²²-CH₂-CH₂-, where R²¹ and R²² each independently represent a hydrogen atom or an alkyl group having 1 to 6 carbon atoms,
L^{1a} and L^{1b} each independently represent an organic group which is either an alkylene group having 1 to 20 carbon atoms, or an alkylene group having 3 to 20 carbon atoms in which at least one methylene group (-CH₂-) contained in the alkylene group is substituted by -O- or -C(=O)-, and the hydrogen atom included in the organic group of L^{1a} and L^{1b} may be substituted by an alkyl group having 1 to 5 carbon atoms, an alkoxy group having 1 to 5 carbon atoms, or a halogen atom, with the proviso that the methylene groups (-CH₂-) on both ends of L^{1a} and L^{1b} are not substituted with -O- or -C(=O)-,
P^{1a} and P^{1b} each independently represent a polymerizable group, and
p1 and p2 each independently represent an integer from 0 to 3.
